# EUROPEAN PATENT APPLICATION

(11) **EP 2 497 778 A2**
(43) Date of publication of application: **12.09.2012**
(21) Application number: 11163721.1
(22) Date of filing: 23.05.2007
(51) Int. Cl.: C07K 14/00, C12N 15/82

(54) **Manipulation of the nitrogen metabolism**

(30) Priority: 31.05.2006 EP 06114744
(62) Divisional of application: 07786718.2
(71) Applicant: Metanomics GmbH, 10589 Berlin (DE)
(72) Inventor: Plesch, Gunnar, 14482, Potsdam (DE); Puzio, Piotr, 9030, Mariakerke (Gent) (BE); Blau, Astrid, 14532, Stahnsdorf (DE); Wendel, Birgit, 13437, Berlin (DE); Looser, Ralf, 13158, Berlin (DE); Kamlage, Beate, 12161, Berlin (DE); Schmitz, Oliver, 14624, Dallgow-Döberitz (DE)
(74) Representative: Fitzner, Uwe

(57) **Abstract**

The present invention relates to the manipulation of the nitrogen metabolism in photosynthetic active organisms, preferably in plants. In particular, the present invention relates to a process for the enhanced nitrogen assimilation, accumulation and/or utilization and/or for the increased total nitrogen content in a photosynthetic active organism.

## Description

The present invention relates to the manipulation of the nitrogen metabolism in photosynthetic active organisms, preferably in plants. In particular, the present invention relates to a process for the enhanced nitrogen assimilation, accumulation and/or utilization and/or for the increased total nitrogen content in a photosynthetic active organism.

Plant nutrition assimilation is essential to the growth and development of plants and therefore also for quantity and quality of plant products. Because of the strong influence of nutrition utilization efficiency on plant yield and product quality, a huge amount of fertilizer is poured onto fields to optimize plant growth and quality. Productivity of plants ordinarily is limited by three primary nutrients, phosphorous, potassium and nitrogen, which is usually the rate-limiting element in plant growth of these three. Therefore the major nutritional element required for plant growth is nitrogen (N). It is a constituent of numerous important compounds found in living cells, including amino acids, proteins (enzymes), nucleic acids, and chlorophyll. 1.5% to 2% of plant dry matter is nitrogen and approximately 16% of total plant protein. Thus, nitrogen availability is a major limiting factor for crop plant growth and production (Frink et al., 1999), and has as well a major impact on protein accumulation and amino acid composition.

Plant can utilize a wide range of nitrogen species including volatile ammonia (NH3), nitrogen oxides (NOx), mineral nitrogen, like nitrate(NO3-) and ammonium salts (NH4+), urea and urea derivates, and organic nitrogen (amino acids, peptides, etc.). Some plants are able to utilize the atmospheric nitrogen by symbiotic bacteria or certain fungi. However, in most agricultural soils, nitrate (NO3-) is the most important source of nitrogen (Crawford and Glass, 1998; Hirsch and Sussman, 1999). Nevertheless also ammonium NH4+ plays an important probably underestimated role, because most plants preferentially take up NH4+ when both forms are present- even if NH4+ is present at lower concentrations than NO3- (Von Wiren et al., 2000).

Because of the high nitrogen requirements for crop plants, nitrogen fertilization is a major worldwide agricultural investment, with 80 million metric tons of nitrogen fertilizers (as nitrate and/or ammonium) applied annually (Frink et al., 1999). There are also negative environmental consequences for the extensive use of nitrogen containing fertilizers in crop production because agricultural crops only retain about two-thirds of the applied nitrogen. Therefore high inputs of fertilizer are followed by large outputs by leaching, gaseous losses and crop removal. The unabsorbed nitrogen can subsequently leach into the soil and contaminate water supplies (Frink et al., 1999). Because of the high leaching losses of nitrogen from agricultural ecosystems to groundwater and surface water, nitrogen is now recognized as an important pollutant. Nitrogen leaching, namely as nitrate from agricultural lands, affects drinking water quality and causes eutrophication of lakes and coastal areas. Abundant use of nitrogen containing fertilizers can further lead to final deterioration of soil quality, to environmental pollution and health hazards.

Because of the high costs of nitrogen fertilizer to agricultural production, and additionally its deleterious effect on the environment, it is desirable to develop strategies to reduce nitrogen input and/or to optimize nitrogen assimilation, accumulation and/or utilization by a given nitrogen availability while simultaneously maintaining optimal productivity and quality of photosynthetic active organisms, preferably cultivated plants, e.g. crops. Preferably the cultivated plants used as food and/or feed should have an improved quality, especially in terms of protein accumulation and composition.

For efficient nitrogen uptake assimilation, accumulation and utilization, complex processes associated with absorption, translocation, assimilation, and redistribution of nitrogen are required to operate effectively. Differences in nitrogen absorption and utilization between genotypes have been demonstrated for several species by different researchers (Chang & Robison, 2001). Considerable evidence of genotypic differences in nitrogen uptake eg. accumulation has also been reported for maize and canola (Weisler et al., 2001; Gallais & Hirel, 2004).

Nitrate uptake in plants is highly regulated and coordinated with other transport and metabolic pathways (Crawford, 1995), and a number of nitrate uptake and assimilation-related genes have been identified and characterized (Forde, 2002). Plants absorb nitrate via transporters localized to the root epidermal and cortical cell plasma membrane over a wide nitrate concentration range using several different transport mechanisms, including constitutive and nitrate-inducible high-affinity transport systems, as well as nitrate-inducible low-affinity transporters (Stitt, 1999). Once in the root cell cytoplasm, nitrate may be stored in the vacuole for later use, transported into the xylem and translocated to the shoot for assimilation and/or storage, released back into the rhizosphere, or reduced to nitrite and then ammonia via nitrate reductase (NR) and nitrite reductases (NiR). The reduction of nitrate to nitrite and then ammonia generates nitrogen in a form that can be assimilated into amino acids via the GOGAT pathway (Stitt, 1999). In order to be incorporated into amino acids, nucleic acids, and other compounds, NO⁻₃ must be reduced to NH⁺₄. NR (nitrate reductase) is the first enzyme in the process of NO⁻₃ reduction to amino form. It is a substrate-inducible enzyme and is thought to be the most limiting step in nitrogen assimilation.

The *in-situ* rate of NO⁻₃ reduction is controlled primarily by the rate of NO⁻₃ uptake, rather than by alterations in nitrate reductase activity (NRA) or limitations in reducing power. Thus, NO⁻₃ uptake appears to be of primary importance in nitrogen assimilation in NO⁻₃-fed plants. Genetic variation in NRA is well documented in several species. NRA is affected by factors such as environmental conditions and plant developmental stages, as well as plant part, such as roots and tops. Furthermore, in vivo and in vitro assays usually give different results. Variable results were found by several researchers in their efforts to relate NRA to grain yield and N-related traits such as total reduced plant N, grain nitrogen content, grain nitrogen concentration, and nitrogen harvest index.

In order to describe the efficiency of the complete pathway of nitrogen, starting with the uptake from soil, assimilating, accumulating and finally utilizing the nitrogen for growth till maturity and for ripeness of fruits and seeds, different approaches are known. In light of the importance of optimal nitrogen acquisition and utilization, different strategies have been followed for plant optimizations.

US-patent 6,727,411 discloses a method of producing transgenic tomatoes having an increased free amino acid content in tomato fruits by transforming a tomato with a genetic construct containing the antisense sequence of a gene encoding glutamate decarboxylase.

In some cases enzymes of the nitrogen assimilation pathway were overexpressed. Although initially unsuccessful like the overexpression of a cytosolic glutamine synthetase gene in Lotus (Vincent et al., Planta. 201 (4):424-33, 1997), recent documents show at least some success. WO95/09911 describes the overexpression of glutamine-synthetase, asparagine-synthetase and asparaginase in transgenic plant for application in enhanced nitrogen-fixation and improved yield. Chichkova et al., J. Exp. Bot.; (2001) reported that transgenic tobacco plants that overexpress alfalfa NADH-glutamate-synthase have higher carbon and nitrogen content, but not a specific enrichment in nitrogen in comparison to carbon. In other case, for example as described in Long et al., Plant-Physiol.; (1996) 111, 2, Suppl., 48, the overexpression of a nitrogen assimilation gene, in this case the Escherichia coli glutamate-dehydrogenase, did not lead to a relative increase in nitrogen content, but rather to an significant increase in fresh weight and dry weight. In another case, overexpression of the ASN1 gene enhances the nitrogen status in seeds of Arabidopsis (Lam et al., Plant Physiology, 2003, 321, 926-935. In seeds of those overexpressing lines the authors observed the elevation of soluble seed protein contents, elevation of total protein, contents from acid-hydrolyzed seeds and a higher tolerance of young seedlings when grown under nitrogen-limiting conditions, demonstrating that those traits are tightly interlinked.

The US-Patent 6,969,782 disclose plants containing free amino acids accumulated in a large amount by excessive expression of glutamate dehydrogenase (GDH).
United States Patent Application 20030115638 provides a transformed plant having free-amino acid content increased by introducing phosphoenolpyruvate carboxylase (PEPC) genes.
Plants with elevated levels of nitrogen utilization proteins in the root of those plants are disclosed in US 20050044585 by expression of an alanine aminotransferase gene.

A different interesting approach was followed by Yanagisawa et al., PNAS (2004) 101, 20, 7833-7838. The authors identified and overexpressed a regulatory factor, which induced the up-regulation of genes encoding enzymes for carbon skeleton production, a marked increase of amino acid contents, and a reduction of the glucose level in transgenic Arabidopsis. Elementary analysis revealed that the nitrogen content increased in transgenic plants (approximate to 30%), indicating promotion of net nitrogen assimilation. Most significantly, the Dof1 transgenic plants exhibit improved growth under low-nitrogen conditions, an agronomically important trait. Although looking promising, this approach likely has the drawback, that it relies on a plant transcription factor and the complex corresponding signalling cascade which both might be the subject of different internal regulatory and feedback mechanism modifying or even diminishing the desired effect at least under certain conditions. In addition the function of a plant transcription factor relies on its interaction with its target sequences in different promoters, making the transfer of results between different plant species complex and unpredictable.

Nevertheless, there is a need for photosynthetic active organisms that are capable to assimilate and accumulate nitrogen more efficiently. In addition, the photosynthetic active organisms have to be capable of a more efficient utilization of nitrogen so that less nitrogen is required for the same yield or higher yields may be obtained with current levels of nitrogen use.

There is furthermore a need for photosynthetic active organisms that show an increased biomass yield, preferably with a faster growth rate, which may lead in a greater fruit or seed yield.
The new photosynthetic active organisms shall present a greater but defined (relating to the proportion of the different amino acids) amino acid content in the fruit or seed or in the whole organism.
The new photosynthetic active organisms shall present a greater but defined (relating to the proportion of the different amino acids) protein content in the fruit or seed or in the whole organism.

The new photosynthetic active organisms shall show at least one of these traits also under conditions of reduced nitrogen content in the surrounding medium, soil or environment.

In one embodiment of the resent invention, this traits are attained by a process for the enhanced nitrogen assimilation, accumulation and/or utilization in a photosynthetic active organism leading to a increased total nitrogen content in the fruit or seed or in the whole organism.

In one embodiment of the resent invention, this achieved by an increased nitrogen use efficiency (NUE).
In one embodiment of the present invention, the NUE is defined as the grain yield per unit of nitrogen available from the soil, including nitrogen fertilizer.
In an other embodiment of the present invention, the NUE is defined according to Reynolds, M.P., J.J. Ortiz-Monasterio, and A. McNab (eds.), 2001. Application of Physiology in Whaet Breeding, Mexico, D.F.:CIMMYT, which is incorporated by reference.
In an other embodiment of the present invention, the NUE is defined as the biomass yield per unit of nitrogen available from the soil, including nitrogen fertilizer.
In an other embodiment of the present invention, the NUE is defined as the total nitrogen content of the photosynthetic active organism per unit of nitrogen available from the soil, including nitrogen fertilizer.

Plants can take up nitrogen also in the form of ammonium. Although the average NH4+ concentrations in soil are often 10 to 1000 times lower than those of NO3- (Marschner HL, Mineral Nutrition in Higher Plants. London: Academic Press; 1995), the difference in soil concentrations does not necessarily reflect the uptake ration of each nitrogen source. Plants take up NH4+ preferentially when both forms of nitrogen are available, eventually because its assimilation requires less energy because NO3- has to be reduced prior to assimilation (Bloom et al., Plant Phys. 1992, 1294-1301).

Ammonium uptake systems have been characterized in different organisms, including yeast and plants. The yeast saccharomyces cerevisiae contains three MEP genes for ammonium transporters, which are all controlled by nitrogen, being repressed in the presence of an nitrogen source that is readily metabolised, such as NH4+ (Marini et al., Mol Cell Biol 1997, 17:4282-4293) Plant genes encoding ammonium transports systems have been cloned by complementation of a yeast mutant, homology searches in databases and heterologous hybridisations (Reviewed in van Wieren et al., Current Opinion in Plant Biology, 200, 3:254-261. Experimental evidence of the physiological function of NH4+ transporters mainly rely on correlations between ammonium transporter expression and influx of labeled ammonium. The situation is complicated by the fact, that in Arabidopsis but also other plants ammonium transporters are present in gene families, the members of which have different expression patterns and physiological characteristics. Although DE 4337597 claims sequences for plant ammonium transporters and their use for manipulation of the nitrogen metabolism and plant growth under certain conditions, any evidence for positive effects on nitrogen assimilation or plant growth under certain conditions through ectopic expression of the plant ammonium transporters were missing. Therefore literature evidence for the engineering of nitrogen assimilation in plants is still limited to a few cases, not including transporters.

It is an object of the present invention to develop an inexpensive process for an enhanced nitrogen assimilation, accumulation and/or utilization and/or for the increased total nitrogen content in a photosynthetic active organism leading to a increased total nitrogen content in the fruit or seed or in the whole organism and an increased nitrogen use efficiency (NUE).

It was now found that this object is achieved by providing the process according to the invention described herein and the embodiments characterized in the claims.

Accordingly, in a first embodiment, the invention relates to a process for the enhanced nitrogen assimilation, accumulation and/or utilization in a photosynthetic active organism.
Accordingly, in an other embodiment, the invention relates to a process for increasing the total nitrogen content in a photosynthetic active organism.

Accordingly, in one embodiment this is achieved by (increased) production of nitrogen or nitrogen containing compounds, whereby nitrogen or nitrogen containing compounds is a compound containing nitrogen (N). In one embodiment the term "nitrogen or nitrogen containing compounds" as used herein relates to "amino acid", preferably phenylalanine, proline, aspartic acid, 5-oxoproline, and/or alanine, "heme-complex", "purine" and/or "pyrimidine"-containing compounds and/or derivates. Further, in another embodiment the term "nitrogen or nitrogen containing compounds s" as used herein also relates to compositions of fine chemicals comprising N-containing compounds..

Accordingly, the present invention relates to a process comprising
**(a)** increasing or generating the activity of one or more of the protein as shown table II, column 3 encoded by the nucleic acid sequences as shown in table I, column 5, in a non-human organism or in one or more parts or compartments thereof and
**(b)** growing the organism under conditions which permit the production of nitrogen or nitrogen containing compounds , thus, N-containing compound and/or enhanced nitrogen assimilation, accumulation and/or utilization and/or increasing total nitrogen content, in said organism.

Accordingly, the present invention relates to a process for the production of a fine chemical comprising
**(a)** increasing or generating the activity of one or more proteins having the activity of a protein selected from the group as indicated in Table II, column 3, application no. 1 and/or 2 and/or 3, lines 1 and/or 2 and/or 3 and/or
   4 and/or 5 respectively
   or having the sequence of a polypeptide encoded by a nucleic acid molecule indicated in Table I, column 5 or 7, application no. 1 and/or 2 and/or 3, in a non-human organism in one or more parts or compartments thereof and
**(b)** growing the organism under conditions which permit the production of nitrogen or nitrogen containing compounds , in particular N-containing compound.

Comprises/comprising and grammatical variations thereof when used in this specification are to be taken to specify the presence of stated features, integers, steps or components or groups thereof, but not to preclude the presence or addition of one or more other features, integers, steps, components or groups thereof. The term "Table I" used in this specification is to be taken to specify the content of Table I A and Table I B. The term "Table II" used in this specification is to be taken to specify the content of Table II A and Table II B. The term "Table I A" used in this specification is to be taken to specify the content of Table I A. The term "Table I B" used in this specification is to be taken to specify the content of Table I B. The term "Table II A" used in this specification is to be taken to specify the content of Table II A. The term "Table II B" used in this specification is to be taken to specify the content of Table II B. In one preferred embodiment, the term "Table I" means Table I B. In one preferred embodiment, the term "Table II" means Table II B.
The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of nitrogen or nitrogen containing compounds in comparison to the reference as defined below, e.g. that means in comparison to an organism without the aforementioned modification of the activity of a protein having the activity of a protein selected from the group as indicated in Table II, column 3, application no. 1 and/or application no. 2 and/or application no. 3 or encoded by nucleic acid molecule indicated in Table I, columns 5 or 7, application no. 1 and/or application no. 2 and/or application 3. The term compartment relates to all different subcellular compartments of a cell, including but not limited to mitochondria, vacuole, the nucleus, all types of plastids, such as amyloplasts, chloroplasts, chromoplasts, the extracellular space, the mitochondria, the endoplasmic reticulum, elaioplasts, peroxisomes, glycosomes, and other compartments.

Surprisingly it was found, that the transgenic expression of at least one of the Saccharomyces cerevisiae protein(s) indicated in Table II, Column 3, application no. 1 or application no. 2, lines 1 or 3 respectively and/or application no. 3, lines 4 and/or 5, and/or at least one of the Escherichia coli K12 protein(s) indicated in Table II, Column 3, application no. 2, line 2 in Arabidopsis thaliana conferred an increase in the N-containing compound content and/or conferred an enhanced nitrogen assimilation, accumulation and/or utilization and/or a increased total nitrogen content of the transformed organism.

Surprisingly it was found, that the transgenic expression of at least one of the Saccharomyces cerevisiae protein(s) indicated in Table II, Column 3, application no. 1,line 1 in Arabidopsis thaliana conferred an increase in the N-containing compound content and/or conferred an enhanced nitrogen assimilation, accumulation and/or utilization and/or a increased total nitrogen content of the transformed organism, when expressed in the host cells, preferably in the cytosol of the plant cells

Surprisingly it was found, that the transgenic expression of at least one of the Saccharomyces cerevisiae protein(s) indicated in Table II, Column 3, application no. 2, line 3 and/or application no. 3, line 4 and/or 5 and/or at least one of the Escherichia coli K12 protein(s) indicated in Table II, Column 3, application no. 2, line 2 in Arabidopsis thaliana conferred an increase in the N-containing compound content and/or conferred an enhanced nitrogen assimilation, accumulation and/or utilization and/or a increased total nitrogen content of the transformed organism, when expressed in the host cells, preferably when expressed in the plastids.

In accordance with the invention, the term "organism" as understood herein relates always to a non-human organism, in particular to a photosynthetic active organism, preferably plant organism or to a microorganism.

The sequence of YPR138C from Saccharomyces cerevisiae has been published in Goffeau et al., Science 274 (5287), 546-547, 1996 and Bussey et al., Nature 387 (6632 Suppl), 103-105 (1997) and its activity is being defined as a NH⁴⁺ transporter. Accordingly, in one embodiment, the process of the present invention comprises the use of a gene product with an activity of ammonium transport protein; ammonium transporter nrgA superfamily, preferably a protein with a NH⁴⁺ transporter activity, from Saccharomyces cerevisiae or its homolog, e.g. as shown herein, for the production of nitrogen or nitrogen containing compounds , meaning of N-containing compound, and/or for conferring an enhanced nitrogen assimilation, accumulation and/or utilization and/or a increased total nitrogen content, in particular for increasing the amount of a N-containing compound in an organism or a part thereof, as mentioned.
Accordingly, in one embodiment, the process of the present invention comprises the use of a gene product with an activity of ammonium transport protein; ammonium transporter nrgA superfamily, preferably a protein with a NH⁴⁺ transporter activity, from Saccharomyces cerevisiae or its homolog, e.g. as shown herein, for the improved uptake and/or assimilation of nitrogen.
Accordingly, in one embodiment, the process of the present invention comprises the use of a gene product with an activity of ammonium transport protein; ammonium transporter nrgA superfamily, preferably a protein with a NH⁴⁺ transporter activity, from Saccharomyces cerevisiae or its homolog, e.g. as shown herein, for the increased uptake and/or utilization and/or assimilation of nitrogen under nitrogen limited conditions.
The sequence of YNL241C (Accession number NP_014158) from Saccharomyces cerevisiae has been published in Goffeau et al., Science 274 (5287), 546-547, 1996 and Philippsen et al., Nature 387 (6632 Suppl), 93-98 (1997), and its activity is being defined as "glucose-6-phosphate dehydrogenase (Zwf1p)". Accordingly, in one embodiment, the process of the present invention comprises the use of said "glucose-6-phosphate dehydrogenase" or its homolog, preferably in plastids , e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for increasing the amount of a N-containing compound in an organism or a part thereof, as mentioned.
Accordingly, in one embodiment, the process of the present invention comprises the use of said "glucose-6-phosphate dehydrogenase" or its homolog, preferably in plastids, e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for the improved uptake and/or assimilation of nitrogen. Accordingly, in one embodiment, the process of the present invention comprises the use of said "glucose-6-phosphate dehydrogenase" or its homolog, preferably in plastids , e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for the increased uptake and/or utilization and/or assimilation of nitrogen under nitrogen limited conditions.
The sequence of b1852 (Accession number NP_416366) from Escherichia coli has been published in Blattner et al., Science 277 (5331), 1453-1474 (1997), and its activity is being defined as "glucose-6-phosphate dehydrogenase". Accordingly, in one embodiment, the process of the present invention comprises the use of a "glucose-6-phosphate dehydrogenase" or its homolog, preferably in plastids, e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for increasing the amount of a N-containing compound in an organism or a part thereof, as mentioned.
Accordingly, in one embodiment, the process of the present invention comprises the use of a "glucose-6-phosphate dehydrogenase" or its homolog, preferably in plastids, e.g. as shown herein, for the production of nitrogen or nitrogen containing compounds and/or for conferring an enhanced nitrogen assimilation, accumulation and/or utilization and/or a increased total nitrogen content, meaning of a N-containing compound, in for the improved uptake and/or assimilation of nitrogen..
Accordingly, in one embodiment, the process of the present invention comprises the use of a "glucose-6-phosphate dehydrogenase" or its homolog, preferably in plastids,, e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for the increased uptake and/or utilization and/or assimilation of nitrogen under nitrogen limited conditions.
The sequence of YJL167W (Accession number NP_012368.1) from Saccharomyces cerevisiae has been published in Goffeau et al., Science 274 (5287), 546-547, 1996 and Anderson et al., J. Biol. Chem 264, 19176-19184 (1989), and its activity is being defined as "farnesyl pyrophosphate synthetase (FPP synthase)". Accordingly, in one embodiment, the process of the present invention comprises the use of said "farnesyl pyrophosphate synthetase (FPP synthase)" or its homolog, preferably in plastids, e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for increasing the amount of a N-containing compound in an organism or a part thereof, as mentioned.
Accordingly, in one embodiment, the process of the present invention comprises the use of said "farnesyl pyrophosphate synthetase (FPP synthase)" or its homolog, preferably in plastids, e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for the improved uptake and/or assimilation of nitrogen.
Accordingly, in one embodiment, the process of the present invention comprises the use of said "farnesyl pyrophosphate synthetase (FPP synthase)" or its homolog, preferably in plastids , e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for the increased uptake and/or utilization and/or assimilation of nitrogen under nitrogen limited conditions.
The sequence of YML045C (Accession number NP_013658.1) from Saccharomyces cerevisiae has been published in Goffeau et al., Science 274 (5287), 546-547, 1996 and Guiard et al., EMBO J. 4, 3265-3272 (1985), and its activity is being defined as "L-lactate cytochrome c oxidoreductase/cytochrome b2". Accordingly, in one embodiment, the process of the present invention comprises the use of said "L-lactate cytochrome c oxidoreductase/cytochrome b2" or its homolog, preferably in plastids, e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for increasing the amount of a N-containing compound in an organism or a part thereof, as mentioned.
Accordingly, in one embodiment, the process of the present invention comprises the use of said "L-lactate cytochrome c oxidoreductase/cytochrome b2" or its homolog, preferably in plastids, e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for the improved uptake and/or assimilation of nitrogen.
Accordingly, in one embodiment, the process of the present invention comprises the use of said "L-lactate cytochrome c oxidoreductase/cytochrome b2" or its homolog, preferably in plastids , e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for the increased uptake and/or utilization and/or assimilation of nitrogen under nitrogen limited conditions.

Homologues (= homologs) of the present gene products can be derived from any organisms as long as the homologue confers the herein mentioned activity, in particular, confers an increase in nitrogen or nitrogen containing compounds amount or content. Further, in the present invention, the term "homologue" relates to the sequence of an organism having the highest sequence homology to the herein mentioned or listed sequences of all expressed sequences of said organism. However, the person skilled in the art knows, that, preferably, the homologue has said nitrogen content-increasing activity and, if known, the same biological function or activity in the organism as at least one of the protein(s) selected from the group as indicated in Table I, Column 3, application no. 1 and/or application no. 2 and/or application no. 3, e.g. having the sequence of a polypeptide encoded by a nucleic acid molecule comprising the sequence indicated in indicated in Table I, Column 5 or 7, application no. 1 and/or application no. 2 and/or application no. 3.
In one embodiment, the homolog of any one of the polypeptides indicated in Table II, lines 1 or 3 or 4 or 5 is a homolog having the same or a similar activity, in particular an increase of activity confers an increase in the content of nitrogen or nitrogen containing compounds nitrogen or nitrogen containing compounds in the organisms and being derived from an Eukaryot. In one embodiment, the homolog of a polypeptide indicated in Table II, column 3, line 2 is a homolog having the same or a similar activity, in particular an increase of activity confers an increase in the nitrogen content nitrogen or nitrogen containing compounds in the organisms or part thereof, and being derived from bacteria. In one embodiment, the homolog of a polypeptide indicated in Table II, column 3, lines 1 or 3 or 4 or 5 is a homolog having the same or a similar activity, in particular an increase of activity confers an increase in the content of nitrogen or nitrogen containing compounds in an organisms or part thereof, and being derived from Fungi.
In one embodiment, the homolog of a polypeptide indicated in Table II, column 3, line 2 is a homolog having the same or a similar activity, in particular an increase of activity confers an increase in the content of nitrogen or nitrogen containing compounds in the organisms or part thereof and being derived from Proteobacteria.
In one embodiment, the homolog of a polypeptide indicated in Table II, column 3, lines 1 or 3 or 4 or 5 is a homolog having the same or a similar activity, in particular an increase of activity confers an increase in the content of nitrogen or nitrogen containing compoundsnitrogen or nitrogen containing compounds in the organisms or a part thereof and being derived from Ascomycota.
In one embodiment, the homolog of a polypeptide indicated in Table II, column 3, line 2 is a homolog having the same or a similar activity, in particular an increase of activity confers an increase in the content nitrogen or nitrogen containing compounds nitrogen or nitrogen containing compounds in the organisms or part thereof, and being derived from Gammaproteobacteria.
In one embodiment, the homolog of a polypeptide indicated in Table II, column 3, lines 1 or 3 or 4 or 5 is a homolog having the same or a similar activity, in particular an increase of activity confers an increase in the content of nitrogen or nitrogen containing compounds nitrogen or nitrogen containing compounds in the organisms or part thereof, and being derived from Saccharomycotina.
In one embodiment, the homolog of a polypeptide indicated in Table II, column 3, line 2 is a homolog having the same or a similar activity, in particular an increase of activity confers an increase in the content of nitrogen or nitrogen containing compounds nitrogen or nitrogen containing compounds in the organisms or part thereof, and being derived from Enterobacteriales.
In one embodiment, the homolog of a polypeptide indicated in Table II, column 3, lines 1 or 3 or 4 or 5 is a homolog having the same or a similar activity, in particular an increase of activity confers an increase in the content of nitrogen or nitrogen containing compounds nitrogen or nitrogen containing compounds in the organisms or a part thereof, and being derived from Saccharomycetes.
In one embodiment, the homolog of the a polypeptide indicated in Table II, column 3, line 2 is a homolog having the same or a similar activity, in particular an increase of activity confers an increase in the content of nitrogen or nitrogen containing compounds nitrogen or nitrogen containing compounds in the organisms or part thereof, and being derived from Enterobacteriaceae.
In one embodiment, the homolog of a polypeptide indicated in Table II, column 3, lines 1 or 3 or 4 or 5 is a homolog having the same or a similar activity, in particular an increase of activity confers an increase in the content of nitrogen or nitrogen containing compounds nitrogen or nitrogen containing compounds in the organisms, and being derived from Saccharomycetales.
In one embodiment, the homolog of a polypeptide indicated in Table II, column 3, line 2 is a homolog having the same or a similar activity, in particular an increase of activity confers an increase in the content of nitrogen or nitrogen containing compounds nitrogen or nitrogen containing compounds in the organisms or a part thereof, and being derived from Escherichia.
In one embodiment, the homolog of a polypeptide indicated in Table II, column 3, lines 1 or 3 or 4 or 5 is a homolog having the same or a similar activity, in particular an increase of activity confers an increase in the content of nitrogen or nitrogen containing compounds nitrogen or nitrogen containing compounds in the organisms or a part thereof, and being derived from Saccharomycetaceae.
In one embodiment, the homolog of a polypeptide indicated in Table II, column 3, line 1 or 3 or 4 or 5 is a homolog having the same or a similar activity, in particular an increase of activity confers an increase in the content of nitrogen or nitrogen containing compounds nitrogen or nitrogen containing compounds in the organisms or a part thereof, and being derived from Saccharomycetes.

Homologs of the polypeptides indicated in Table II, column 3, application no. 1 and/or application no. 2 and/or application no. 3, may be the polypeptides encoded by the nucleic acid molecules polypeptide indicated in Table I, column 7, application no. 1 and/or application no. 2 and/or application no. 3 or may be the polypeptides indicated in Table II, column 7, application no. 1 and/or application no. 2 and/or application no. 3.

Further homologs of are described herein below.

In accordance with the invention, a protein or polypeptide has the "activity of an protein of the invention", or of a protein as used in the invention, e.g. a protein having the activity of a protein indicated in Table II, column 3, application no. 1 if its *de novo* activity, or its increased expression directly or indirectly leads to an increased total nitrogen content, preferably of N-containing compounds, preferably amino acids, more preferably phenylalanine, proline, aspartic acid, 5-oxoproline, and/or alanine level in the organism or a part thereof, preferably in a cell of said organism. In one embodiment of the present invention the expression of a protein having the activity of a protein indicated in Table II, column 3, application no. 1 has the activity of an protein of the invention if its *de novo* activity, or its increased expression directly or indirectly leads to an increased total nitrogen content, preferably of N-containing compounds, preferably amino acids, more preferably phenylalanine, proline level in leaves of a plant and of aspartic acid, 5-oxoproline, and/or alanine level preferably in the seeds of a plant.

In accordance with the invention, a protein or polypeptide has the "activity of an protein of the invention", or of a protein as used in the invention, e.g. a protein having the activity of a protein indicated in Table II, column 3, application no. 2, line 2 if its *de novo* activity, or its increased activity directly or indirectly leads to an increased total nitrogen content, preferably of N-containing compounds, preferably amino acids, more preferably proline level in the organism or a part thereof, preferably in a cell of said organism.
In one embodiment of the present invention the expression of a protein having the activity of a protein indicated in Table II, column 3, application no. 2, line 2 has the activity of an protein of the invention if its *de novo* activity, or its increased directly or indirectly leads to an increased total nitrogen content, preferably of N-containing compounds, preferably amino acids, more preferably proline level in leaves a plant.

In accordance with the invention, a protein or polypeptide has the "activity of an protein of the invention", or of a protein as used in the invention, e.g. a protein having the activity of a protein indicated in Table II, column 3, application no. 2, line 3 if its *de novo* activity, or its increased expression directly or indirectly leads to an increased total nitrogen content, preferably of N-containing compounds, preferably amino acids, more preferably tyrosine, tryptophane, isoleucine, arginine, threonine, valine and/or alanine level in the organism or a part thereof, preferably in a cell of said organism.
In one embodiment of the present invention the expression of a protein having the activity of a protein indicated in Table II, column 3, application no. 2, line 3 has the activity of an protein of the invention if its *de novo* activity, or its increased activity directly or indirectly leads to an increased total nitrogen content, perferably of N-containing compounds, preferably amino acids, more preferably tyrosine, tryptophane, isoleucine, arginine, threonine, valine and/or alanine level in leaves a plant and/or alanine in the seeds of a plant.

In a preferred embodiment, the protein or polypeptide has the above-mentioned additional activities of a protein selected from the group as indicated in Table II, column 3, application no. 1 and/or application no. 2 and/or application no. 3, preferably application no. 1. During the specification the activity or preferably the biological activity of such a protein or polypeptide or an nucleic acid molecule or sequence encoding such protein or polypeptide is identical or similar if it still has the biological or enzymatic activity of any one of the proteins selected from the group as indicated in Table II, column 3, application no. 1 and/or application no. 2 and/or application no. 3, preferably application no. 1, i.e. if it has at least 10% of the original enzymatic activity, preferably 20%, particularly preferably 30%, most particularly preferably 40% in comparison to an any one of the proteins indicated in Table II, column 3, application no. 1 and/or application no. 2 and/or application no. 3, preferably application no. 1.

In one embodiment, the polypeptide of the invention or the polypeptide used in the method of the invention confers said activity, e.g. the increase of nitrogen or nitrogen containing compounds and/or the enhanced nitrogen assimilation, accumulation and/or utilization and/or for the increased total nitrogen content in an organism or a part thereof, if it is derived from an organism, which is evolutionary distant to the organism in which it is expressed. For example origin and expressing organism are derived from different families, orders, classes or phylums.

In one embodiment, the polypeptide of the invention or the polypeptide used in the method of the invention confers said activity, e.g. the increase of nitrogen or nitrogen containing compounds, and/or the enhanced nitrogen assimilation, accumulation and/or utilization and/or for the increased total nitrogen content in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

The terms "increased", "rose", "extended", "enhanced", "improved" or "amplified" relate to a corresponding change of a property in an organism, a part of an organism such as a tissue, seed, root, leave, flower etc. or in a cell and are interchangeable. Preferably, the overall activity in the volume is increased or enhanced in cases if the increase or enhancement is related to the increase or enhancement of an activity of a gene product, independent whether the amount of gene product or the specific activity of the gene product or both is increased or enhanced or whether the amount, stability or translation efficacy of the nucleic acid sequence or gene encoding for the gene product is increased or enhanced. The terms "reduction", "decrease" or "deletion" relate to a corresponding change of a property in an organism, a part of an organism such as a tissue, seed, root, leave, flower etc. or in a cell. Preferably, the overall activity in the volume is reduced, decreased or deleted in cases if the reduction, decrease or deletion is related to the reduction, decrease or deletion of an activity of a gene product, independent whether the amount of gene product or the specific activity of the gene product or both is reduced, decreased or deleted or whether the amount, stability or translation efficacy of the nucleic acid sequence or gene encoding for the gene product is reduced, decreased or deleted.

The terms "increase" or "decrease" relate to a corresponding change of a property an organism or in a part of an organism, such as a tissue, seed, root, leave, flower etc. or in a cell.. Preferably, the overall activity in the volume is increased in cases the increase relates to the increase of an activity of a gene product, independent whether the amount of gene product or the specific activity of the gene product or both is increased or generated or whether the amount, stability or translation efficacy of the nucleic acid sequence or gene encoding for the gene product is increased.

Under "change of a property" it is understood that the activity, expression level or amount of a gene product or the metabolite content or the element content is changed in a specific volume relative to a corresponding volume of a control, reference or wild type, including the de novo creation of the activity or expression.

The terms "increase" or "decrease" include the change or the modulation of said property in only parts of the subject of the present invention, for example, the modification can be found in compartment of a cell, like a organelle, or in a part of a plant, like tissue, seed, root, leave, flower etc. but is not detectable if the overall subject, i.e. complete cell or plant, is tested. Preferably, the increase or decrease is found cellular, thus the term "increase of an activity" or "increase of a metabolite or element content" relates to the cellular increase compared to the wild type cell.
However, the terms increase or decrease as used herein also includes the change or modulation of a property in the whole organism as mentioned.

Accordingly, the term "increase" or "decrease" means that the specific activity of an enzyme, preferably the amount of a compound or metabolite, e.g. of a polypeptide, a nucleic acid molecule or of nitrogen or nitrogen containing compounds of the invention or an encoding mRNA or DNA, can be increased or decreased in a volume.

The terms "wild type", "control" or "reference" are exchangeable and can be a cell or a part of organisms such as an organelle or a tissue, or an organism, in particular a microorganism or a plant, which was not modified or treated according to the herein described process according to the invention. Accordingly, the cell or a part of organisms such as an organelle or a tissue, or an organism, in particular a microorganism or a plant used as wild type, control or reference corresponds to the cell, organism or part thereof as much as possible and is in any other property but in the result of the process of the invention as identical to the subject matter of the invention as possible. Thus, the wild type, control, or reference is treated identically or as identical as possible, saying that only conditions or properties might be different which do not influence the quality of the tested property.

Preferably, any comparison is carried out under analogous conditions. The term "analogous conditions" means that all conditions such as, for example, culture or growing conditions, assay conditions (such as buffer composition, temperature, substrates, pathogen strain, concentrations and the like) are kept identical between the experiments to be compared.

The "reference", "control", or "wild type" is preferably a subject, e.g. an organelle, a cell, a tissue, an organism, in particular a plant or a microorganism, which was not modified or treated according to the herein described process of the invention and is in any other property as similar to the subject matter of the invention as possible. The reference, control, or wild type is in its genome, transcriptome, proteome or meta-bolome as similar as possible to the subject of the present invention. Preferably, the term "reference-" "control-" or "wild type-"-organelle, -cell, -tissue or-organism, in particular plant or microorganism, relates to an organelle, cell, tissue or organism, in particular plant or microorganism, which is nearly genetically identical to the organelle, cell, tissue or organism, in particular microorganism or plant, of the present invention or a part thereof preferably 95%, more preferred are 98%, even more preferred are 99,00%, in particular 99,10%, 99,30%, 99,50%, 99,70%, 99,90%, 99,99%, 99, 999% or more.. Most preferable the "reference", "control", or "wild type" is a subject, e.g. an organelle, a cell, a tissue, an organism, which is genetically identical to the organism, cell or organelle used according to the process of the invention except that the responsible or activity conferring nucleic acid molecules or the gene product encoded by them are amended, manipulated, exchanged or introduced according to the inventive process.
Preferably, the reference, control or wild type differs form the subject of the present invention only in the cellular activity of the polypeptide of the invention or the polypeptide used in the method of the invention, e.g. as result of an increase in the level of the nucleic acid molecule of the present invention or an increase of the specific activity of the polypeptide of the invention or the polypeptide used in the method of the invention. E.g., it differs by or in the expression level or activity of an protein having the activity of a protein selected from the group as as indicated in Table II, column 3, application no. 1 and/or application no. 2 and/or application no. 3, preferably application no. 1, or being encoded by a nucleic acid molecule indicated in Table I, column 5, application no. 1 and/or application no. 2, preferably application no. 1, or its homologs, e.g. as indicated in Table I, column 7, application no. 1 and/or application no. 2 and/or application no. 3, preferably application no. 1, its biochemical or genetical causes and therefore shows the increased amount of nitrogen or nitrogen containing compounds, the enhanced nitrogen assimilation, accumulation and/or utilization and/or the increased total nitrogen content.

In case, a control, reference or wild type differing from the subject of the present invention only by not being subject of the process of the invention can not be provided, a control, reference or wild type can be an organism in which the cause for the modulation of an activity conferring the increase of nitrogen or nitrogen containing compounds nitrogen or nitrogen containing compounds or expression of the nucleic acid molecule as described herein has been switched back or off, e.g. by knocking out the expression of responsible gene product, e.g. by antisense inhibition, by inactivation of an activator or agonist, by activation of an inhibitor or antagonist, by inhibition through adding inhibitory antibodies, by adding active compounds as e.g. hormones, by introducing negative dominant mutants, etc. A gene production can for example be knocked out by introducing inactivating point mutations, which lead to an enzymatic or biological activity inhibition or a destabilization or an inhibition of the ability to bind to cofactors etc.

Accordingly, preferred reference subject is the starting subject of the present process of the invention. Preferably, the reference and the subject matter of the invention are compared after standardization and normalization, e.g. to the amount of total RNA, DNA, or Protein or activity or expression of reference genes, like housekeeping genes, such as ubiquitin, actin or ribosomal proteins.

A series of mechanisms exists via which a modification of a protein, e.g. the polypeptide of the invention or the polypeptide used in the method of the invention can directly or indirectly affect the uptake or assimilation of nitrogen or the yield, production and/or production efficiency of of nitrogen containing compounds.

For example, the molecule number or the specific activity of the polypeptide or the nucleic acid molecule may be increased. Larger amounts of nitrogen can be assimilated or taken up or in case of nitrogen containing compounds produced if the polypeptide or the nucleic acid of the invention is expressed *de novo* in an organism lacking the activity of said protein. However, it is also possible to increase the expression of the gene which is naturally present in the organisms, for example by amplifying the number of gene(s), by modifying the regulation of the gene, or by increasing the stability of the corresponding mRNA or of the corresponding gene product encoded by the nucleic acid molecule of the invention or the nucleic acid molecule used in the method of the invention, or by introducing homologous genes from other organisms which are differently regulated, e.g. not feedback sensitive.
The increase, decrease or modulation according to this invention can be constitutive, e.g. due to a stable permanent transgenic expression or to a stable mutation in the corresponding endogenous gene encoding the nucleic acid molecule of the invention or the nucleic acid molecule used in the method of the invention or to a modulation of the expression or of the behaviour of a gene conferring the expression of the polypeptide of the invention or the polypeptide used in the method of the invention, or transient, e.g. due to an transient transformation or temporary addition of a modulator such as a agonist or antagonist or inducible, e.g. after transformation with an inducible construct carrying the nucleic acid molecule of the invention or the nucleic acid molecule used in the method of the invention under control of a inducible promoter and adding the inducer, e.g. tetracycline or as described herein below.

The increase in activity of the polypeptide amounts in a cell, a tissue, a organelle, an organ or an organism or a part thereof preferably to at least 5%, preferably to at least 20% or at to least 50%, especially preferably to at least 70%, 80%, 90% or more, very especially preferably are to at least 200%, most preferably are to at least 500% or more in comparison to the control, reference or wild type.

The specific activity of a polypeptide encoded by a nucleic acid molecule of the present invention or of the polypeptide of the present invention can be tested as described in the examples. In particular, the expression of a protein in question in a cell, e.g. a plant cell or a microorganism and the detection of an increase in nitrogen or nitrogen containing compounds level in comparison to a control is an easy test and can be performed as described in the state of the art.

The term "increase" includes, that a compound or an activity is introduced into a cell *de novo* or that the compound or the activity has not been detectable before, in other words it is "generated".

Accordingly, in the following, the term "increasing" also comprises the term "generating" or "stimulating". The increased activity manifests itself in an increased amount of nitrogen or nitrogen containing compounds.

In one embodiment, in case the activity of the Saccharomyces cerevisiae protein YPR138C or its homologs, e.g. as indicated in Table II, columns 5 or 7, application no. 1, line 1, is increased; preferably, an increase of nitrogen or nitrogen containing compounds between 17% and 24% or more is conferred, preferably an increase of protein or amino acid content in a plant between 17% and 24% or more is conferred. Preferably this increase in conferred in plant seeds or fruits.

In one embodiment, in case the activity of the Saccharomyces cerevisiae protein YPR138C or its homologs is increased, preferably, an increase of nitrogen or nitrogen containing compounds and of coenzyme Q10, fumaric acid, malic acid and/or lignoceric acid in leaves and/or glycerol-3-phosphate, benzoic acid, hydroxyl-benzoic acid and/or dodecanol in seeds of a plant is conferred.

In one embodiment, in case the activity of the Saccharomyces cerevisiae protein YNL241C or its homologs, e.g. as indicated in Table II, columns 5 or 7, application no. 2, line 3, is increased, preferably in a cellular compartment, preferably in the plastids, preferably, an increase of nitrogen or nitrogen containing compounds between 10% and 15 % or more, preferably of 12% or more is conferred, preferably an increase of amino acid content in a plant between 10% and 15 % or more, preferably of 12 % or more is conferred. Preferably this increase in conferred in plant seeds or fruits.

In one embodiment, in case the activity of the Echerichia coli protein b1852 or its homologs, e.g. as indicated in Table II, columns 5 or 7, application no. 2, line 2, is increased, preferably in a cellular compartment, preferably in the plastids, preferably, an increase of nitrogen or nitrogen containing compounds between 10% and 15 % or more, preferably of 13% or more is conferred, preferably an increase of amino acid content in a plant between 10% and 15 % or more, preferably of 13% or more is conferred preferably in the seeds.

In one embodiment, in case the activity of the Saccharomyces cerevisiae protein YJL167W or its homologs, e.g. as indicated in Table II, columns 5 or 7, application no. 3, line 4, is increased, preferably in a cellular compartment, preferably in the plastids, preferably, an increase of nitrogen or nitrogen containing compounds between 5% and 30 % or more, preferably between 8% and 26% or more is conferred preferably in the seeds.

In one embodiment, in case the activity of the Saccharomyces cerevisiae protein YML054C or its homologs, e.g. as indicated in Table II, columns 5 or 7, application no. 3, line 5, is increased, preferably in a cellular compartment, preferably in the plastids, preferably, an increase of nitrogen or nitrogen containing compounds between 5% and 20% or more, preferably of between 6% and 15% or more is conferred preferably in the seeds.

A protein having an activity conferring an increase in the amount or level of nitrogen or nitrogen containing compounds and/or enhanced nitrogen assimilation, accumulation and/or utilization and/or the increased total nitrogen content preferably has the structure of the polypeptide described herein, in particular of a polypeptides comprising a consensus sequence selected from the group as indicated in Table IV, columns 7, application no. 1 and/or application no. 2 and/or application no. 3, preferably application no. 1 or of a polypeptide selected from the group as indicated in Table II, columns 5 or 7, application no. 1 and/or application no. 2 and/or application no. 3, preferably application no. 1 or the functional homologues thereof as described herein, or of a polypeptide which is encoded by the nucleic acid molecule characterized herein or the nucleic acid molecule according to the invention, for example by a nucleic acid molecule as indicated in Table I, columns 5 or 7, application no. 1 and/or application no. 2 and/or application no. 3, preferably application no. 1 or its herein described functional homologues and has the herein mentioned activity.

Owing to the biological activity of the proteins which are used in the process according to the invention and which are encoded by nucleic acid molecules according to the invention, it is possible to produce compositions comprising nitrogen or nitrogen containing compounds. Depending on the choice of the organism used for the process according to the present invention, for example a microorganism or a plant, compositions or mixtures of various nitrogen containing compounds, e.g. comprising further distinct amino acids, fatty acids, vitamins, hormones, sugars, lipids, etc. can be produced.

The term "expression" refers to the transcription and/or translation of a codogenic gene segment or gene. As a rule, the resulting product is an mRNA or a protein. However, expression products can also include functional RNAs such as, for example, antisense, nucleic acids, tRNAs, snRNAs, rRNAs, RNAi, siRNA, ribozymes etc. Expression may be systemic, local or temporal, for example limited to certain cell types, tissues organs or time periods.

In one embodiment, the process of the present invention comprises one or more of the following steps:
**a)** stabilizing a protein conferring the increased expression of a protein encoded by the nucleic acid molecule of the invention or of the polypeptid of the invention or the nucleic acid molecule or the polypeptide used in the method of the invention, e.g. of a polypeptide having an activity of a protein selected from the group as indicated in Table II, column 3, or its homologs activity, e.g. as indicated in Table II, columns 5 or 7, having herein-mentioned -increasing activity;
**b)** stabilizing a mRNA conferring the increased expression of a protein encoded by the nucleic acid molecule of the invention or the nucleic acid molecule used in the method of the invention, e.g. of a polypeptide having an activity of a protein selected from the group as indicated in Table II, column 3 or its homologs activity, e.g. as indicated in Table II, columns 5 or 7, , or of a mRNA encoding the polypeptide of the present invention having herein-mentioned -increasing activity;
**c)** increasing the specific activity of a protein conferring the increased expression of a protein encoded by the nucleic acid molecule of the invention or of the polypeptide of the present invention or the nucleic acid molecule or polypeptide used in the method of the invention, having herein-mentioned -increasing activity, e.g. of a polypeptide having an activity of a protein selected from the group as indicated in Table II, column 3, , or its homologs activity, e.g. as indicated in Table II, columns 5 or 7, or decreasing the inhibitory regulation of the polypeptide of the invention or the polypeptide used in the method of the invention;
**d)** generating or increasing the expression of an endogenous or artificial transcription factor mediating the expression of a protein conferring the increased expression of a protein encoded by the nucleic acid molecule of the invention or the nucleic acid molecule used in the method of the invention or of the polypeptide of the invention or the polypeptide used in the method of the invention having herein-mentioned -increasing activity, e.g. of a polypeptide having an activity of a protein selected from the group as indicated in Table II, column 3, , or its homologs activity, e.g. as indicated in Table II, columns 5 or 7,;
**e)** stimulating activity of a protein conferring the increased expression of a protein encoded by the nucleic acid molecule of the present invention or a polypeptide of the present invention having herein-mentioned -increasing activity, e.g. of a polypeptide having an activity of a protein selected from the group as indicated in Table II, column 3, or its homologs activity, e.g. as indicated in Table II, columns 5 or 7, by adding one or more exogenous inducing factors to the organism or parts thereof;
**f)** expressing a transgenic gene encoding a protein conferring the increased expression of a polypeptide encoded by the nucleic acid molecule of the present invention or a polypeptide of the present invention, having herein-mentioned-increasing activity, e.g. of a polypeptide having an activity of a protein selected from the group as indicated in Table II, column 3, or its homologs activity, e.g. as indicated in Table II, columns 5 or 7,;
**g)** increasing the copy number of a gene conferring the increased expression of a nucleic acid molecule encoding a polypeptide encoded by the nucleic acid molecule of the invention or the nucleic acid molecule used in the method of the invention or the polypeptide of the invention or the polypeptide used in the method of the invention having herein-mentioned - increasing activity, e.g. of a polypeptide having an activity of a protein selected from the group as indicated in Table II, column 3, or its homologs activity, e.g. as indicated in Table II, columns 5 or 7,;
**h)** Increasing the expression of the endogenous gene encoding the polypeptide of the invention or the polypeptide used in the method of the invention, e.g. a polypeptide having an activity of a protein selected from the group as indicated in Table II, column 3, or its homologs activity, e.g. selected from the group as indicated in Table II, columns 5 or 7, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements. Positive elements can be randomly introduced in plants by T-DNA or transposon mutagenesis and lines can be identified in which the positive elements have be integrated near to a gene of the invention, the expression of which is thereby enhanced;
**i)** Modulating growth conditions of an organism in such a manner, that the expression or activity of the gene encoding the protein of the invention or the protein itself is enhanced for example microorganisms or plants can be grown under a higher temperature regime leading to an enhanced expression of heat shock proteins, e.g. the heat shock protein of the invention, which can lead an enhanced the fine chemical production; and/or
**j)** selecting of organisms with especially high activity of the proteins of the invention from natural or from mutagenized resources and breeding them into the target organisms, e.g. the elite crops.

Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of N-containing compound after increasing the expression or activity of the encoded polypeptide or having the activity of a polypeptide having an activity of a protein selected from the group as indicated in Table II, column 3, or its homologs activity, e.g. as indicated in Table II, columns 5 or 7,.

In general, the amount of mRNA or polypeptide in a cell or a compartment of a organism correlates with the amount of encoded protein and thus with the overall activity of the encoded protein in said volume. Said correlation is not always linear, the activity in the volume is dependent on the stability of the molecules or the presence of activating or inhibiting co-factors. Further, product and educt inhibitions of enzymes are well known and described in Textbooks, e.g. Stryer, Biochemistry.

In general, the amount of mRNA, polynucleotide or nucleic acid molecule in a cell or a compartment of an organism correlates with the amount of encoded protein and thus with the overall activity of the encoded protein in said volume. Said correlation is not always linear, the activity in the volume is dependent on the stability of the molecules, the degradation of the molecules or the presence of activating or inhibiting co-factors. Further, product and educt inhibitions of enzymes are well known, e.g. Zinser et al. "Enzyminhibitoren"/Enzyme inhibitors".

The activity of the abovementioned proteins and/or polypeptide encoded by the nucleic acid molecule of the present invention can be increased in various ways. For example, the activity in an organism or in a part thereof, like a cell or a organelle, is increased via increasing the gene product number, e.g. by increasing the expression rate, like introducing a stronger promoter, or by increasing the stability of the mRNA expressed, thus increasing the translation rate, and/or increasing the stability of the gene product, thus reducing the proteins decayed. Further, the activity or turnover of enzymes can be influenced in such a way that a reduction or increase of the reaction rate or a modification (reduction or increase) of the affinity to the substrate results, is reached. A mutation in the catalytic centre of an polypeptide of the invention or the polypeptide used in the method of the invention, e.g. as enzyme, can modulate the turn over rate of the enzyme, e.g. a knock out of an essential amino acid can lead to a reduced or completely knock out activity of the enzyme, or the deletion or mutation of regulator binding sites can reduce a negative regulation like a feedback inhibition (or a substrate inhibition, if the substrate level is also increased). The specific activity of an enzyme of the present invention can be increased such that the turn over rate is increased or the binding of a co-factor is improved. Improving the stability of the encoding mRNA or the protein can also increase the activity of a gene product. The stimulation of the activity is also under the scope of the term "increased activity"..

Moreover, the regulation of the abovementioned nucleic acid sequences may be modified so that gene expression is increased. This can be achieved advantageously by means of heterologous regulatory sequences or by modifying, for example mutating, the natural regulatory sequences which are present. The advantageous methods may also be combined with each other.

In general, an activity of a gene product in an organism or part thereof, in particular in a plant cell, a plant, or a plant tissue, a part thereof or a organelle or in a microorganism can be increased by increasing the amount of the specific encoding mRNA or the corresponding protein in said organism or part thereof. "Amount of protein or mRNA" is understood as meaning the molecule number of polypeptides or mRNA molecules in an organism, a tissue, a cell, or a cell compartment. "Increase" in the amount of a protein means the quantitative increase of the molecule number of said protein in an organism, a tissue, a cell or a cell compartment or part thereof - for example by one of the methods described herein below - in comparison to a wild type, control or reference.

The increase in molecule number amounts preferably to at least 1%, preferably to more than 10%, more preferably to 30% or more, especially preferably to 50%, 70% or more, very especially preferably to 100%, most preferably to 500% or more. However, a de novo expression is also regarded as subject of the present invention.

A modification, i.e. an increase or decrease, can be caused by endogenous or exogenous factors. For example, an increase in activity in an organism or a part thereof can be caused by adding a gene product or a precursor or an activator or an agonist to the media or nutrition or can be caused by introducing said subjects into a organism, transient or stable.

In one embodiment the increase in the amount of nitrogen or nitrogen containing compounds in the organism or a part thereof, e.g. in a cell, a tissue, a organ, an organelle etc., is achieved by increasing the endogenous level of the polypeptide of the invention or the polypeptide used in the method of the invention in the cytosol or in a compartment like the plastids. Accordingly, in an embodiment of the present invention, the present invention relates to a process wherein the gene copy number of a gene encoding the polynucleotide or nucleic acid molecule of the invention or the nucleic acid molecule used in the method of the invention as herein described is increased. Further, the endogenous level of the polypeptide of the invention or the polypeptide used in the method of the invention as described can for example be increased by modifying the transcriptional or translational regulation of the polypeptide.

In one embodiment the amount of nitrogen or nitrogen containing compounds in the organism or part thereof can be increase by targeted or random mutagenesis of the endogenous genes of the invention. For example homologous recombination can be used to either introduce positive regulatory elements like for plants the 35S enhancer into the promoter or to remove repressor elements form regulatory regions. In addition gene conversion like methods described by Kochevenko and Willmitzer (Plant Physiol. 2003 May; 132(1): 174-84) and citations therein can be used to disrupt repressor elements or to enhance to activity of positive regulatory elements.
Furthermore positive elements can be randomly introduced in (plant) genomes by T-DNA or transposon mutagenesis and lines can be screened for, in which the positive elements has be integrated near to a gene of the invention, the expression of which is thereby enhanced. The activation of plant genes by random integrations of enhancer elements has been described by Hayashi et al., 1992 (Science 258:1350-1353) or Weigel et al., 2000 (Plant Physiol. 122, 1003-1013) and others citied therein. Reverse genetic strategies to identify insertions (which eventually carrying the activation elements) near in genes of interest have been described for various cases e.g. Krysan et al., 1999 (Plant Cell 1999, 11, 2283-2290); Sessions et al., 2002 (Plant Cell 2002, 14, 2985-2994); Young et al., 2001, (Plant Physiol. 2001, 125, 513-518); Koprek et al., 2000 (Plant J. 2000, 24, 253-263) ; Jeon et al., 2000 (Plant J. 2000, 22, 561-570) ; Tissier et al., 1999 (Plant Cell 1999, 11, 1841-1852); Speulmann et al., 1999 (Plant Cell 1999,11 , 1853-1866). Briefly material from all plants of a large T-DNA or transposon mutagenized plant population is harvested and genomic DNA prepared. Then the genomic DNA is pooled following specific architectures as described for example in Krysan et al., 1999 (Plant Cell 1999, 11, 2283-2290). Pools of genomics DNAs are then screened by specific multiplex PCR reactions detecting the combination of the insertional mutagen (e.g. T-DNA or Transposon) and the gene of interest. Therefore PCR reactions are run on the DNA pools with specific combinations of T-DNA or transposon border primers and gene specific primers. General rules for primer design can again be taken from Krysan et al., 1999 (Plant Cell 1999, 11, 2283-2290) Rescreening of lower levels DNA pools lead to the identification of individual plants in which the gene of interest is disrupted by the insertional mutagen.
The enhancement of positive regulatory elements or the disruption or weaking of negative regulatory elements can also be achieved through common mutagenesis techniques: The production of chemically or radiation mutated populations is a common technique and known to the skilled worker. Methods for plants are described by Koorneef et al. 1982 and the citations therein and by Lightner and Caspar in "Methods in Molecular Biology" Vol 82. These techniques usually induce pointmutations that can be identified in any known gene using methods such as tilling (Colbert et al. 2001).
Accordingly, the expression level can be increased if the endogenous genes encoding a polypeptide conferring an increased expression of the polypeptide of the present invention, in particular genes comprising the nucleic acid molecule of the present invention, are modified via homologous recombination, tilling approaches or gene conversion.

Regulatory sequences can be operatively linked to the coding region of an endogenous protein and control its transcription and translation or the stability or decay of the encoding mRNA or the expressed protein. In order to modify and control the expression, promoter, UTRs, splicing sites, processing signals, polyadenylation sites, terminators, enhancers, repressors, post transcriptional or posttranslational modification sites can be changed, added or amended for example, the activation of plant genes by random integrations of enhancer elements has been described by Hayashi et al., 1992 (Science 258:1350-1353) or Weigel et al., 2000 (Plant Physiol. 122, 1003-1013) and others citied therein. For example, the expression level of the endogenous protein can be modulated by replacing the endogenous promoter with a stronger transgenic promoter or by replacing the endogenous 3'UTR with a 3'UTR, which provides more stability without amending the coding region. Further, the transcriptional regulation can be modulated by introduction of an artificial transcription factor as described in the examples. Alternative promoters, terminators and UTR are described below.

The activation of an endogenous polypeptide having above-mentioned activity, of the polypeptide of the invention or the polypeptide used in the method of the invention, e.g. conferring the increase of nitrogen or nitrogen containing compounds after increase of expression or activity in the cytsol and/or in an organelle like a plastid, can also be increased by introducing a synthetic transcription factor, which binds close to the coding region of an endogenous polypeptide of the invention or the polypeptide used in the method of the invention- or used in the process of the invention or its endogenous homolog -encoding gene whereby the synthetic transcription factor activates its transcription. A chimeric zinc finger protein can be construed, which comprises a specific DNA-binding domain and an activation domain as e.g. the VP16 domain of Herpes Simplex virus. The specific binding domain can bind to the regulatory region of the endogenous protein coding region. The expression of the chimeric transcription factor in a organism, in particular in a plant, leads to a specific expression of an endogenous polypeptid of the invention or used in the process of the invention, in particular a plant homolog thereof, see e.g. in WO01/52620, Oriz, Proc. Natl. Acad. Sci. USA, 2002, Vol. 99, 13290 or Guan, Proc. Natl. Acad. Sci. USA, 2002, Vol. 99, 13296.

In one further embodiment of the process according to the invention, organisms are used in which one of the abovementioned genes, or one of the abovementioned nucleic acids, is mutated in a way that the activity of the encoded gene products is less influenced by cellular factors, or not at all, in comparison with the unmutated proteins. For example, well known regulation mechanism of enzymic activity are substrate inhibition or feed back regulation mechanisms. Ways and techniques for the introduction of substitutions, deletions and additions of one or more bases, nucleotides or amino acids of a corresponding sequence are described herein below in the corresponding paragraphs and the references listed there, e.g. in Sambrook et al., Molecular Cloning, Cold Spring Habour, NY, 1989. The person skilled in the art will be able to identify regulation domains and binding sites of regulators by comparing the sequence of the nucleic acid molecule of the present invention or the expression product thereof with the state of the art by computer software means which comprise algorithms for the identifying of binding sites and regulation domains or by introducing into a nucleic acid molecule or in a protein systematically mutations and assaying for those mutations which will lead to an increased specific activity or an increased activity per volume, in particular per cell.

It is therefore advantageously to express in an organism a nucleic acid molecule of the invention or the nucleic acid molecule used in the method of the invention or a polypeptide of the invention or the polypeptide used in the method of the invention derived from a evolutionary distantly related organism, as e.g. using a prokaryotic gene in an eukaryotic host, as in these cases the regulation mechanism of the host cell may not weaken the activity (cellular or specific) of the gene or its expression product .

%

Less influence on the regulation of a gene or its gene product is understood as meaning a reduced regulation of the enzymatic or biological activity leading to an increased specific or cellular activity of the gene or its product. An increase of the enzymatic or biological activity is understood as meaning an enzymatic or biological activity, which is increased by at least 10%, advantageously at least 20, 30 or 40%, especially advantageously by at least 50, 60 or 70% in comparison with the starting organism. This leads to an increased productivity of the desired nitrogen or nitrogen containing compounds.

Owing to the introduction of a gene or a plurality of genes conferring the expression of the nucleic acid molecule of the invention or the nucleic acid molecule used in the method of the invention or the polypeptide of the invention or the polypeptide used in the method of the invention as described below, for example the nucleic acid construct mentioned below, into an organism alone or in combination with other genes, it is possible not only to increase the biosynthetic flux towards the end product, e.g. meaning nitrogen containing compounds, but also to increase, modify or create *de novo* an advantageous, preferably novel metabolites composition in the organism, e.g. an advantageous amino acid composition comprising a higher content of (from a viewpoint of nutrional physiology limited) respective fine chemicals, in particular amino acids, likewise nitrogen or nitrogen containing compounds .

Preferably the composition further comprises higher amounts of metabolites positively affecting or lower amounts of metabolites negatively affecting the nutrition or health of animals or humans provided with said compositions or organisms of the invention or parts thereof. Likewise, the number or activity of further genes which are required for the import or export of nutrients or metabolites, including amino acids or its precursors, required for the cell's biosynthesis of amino acids may be increased so that the concentration of necessary or relevant precursors, cofactors or intermediates within the cell(s) or within the corresponding storage compartments is increased. Owing to the increased or novel generated activity of the polypeptide of the invention or the polypeptide used in the method of the invention or owing to the increased number of nucleic acid sequences of the invention and/or to the modulation of further genes which are involved in the biosynthesis of the amino acids, e.g. by increasing the activity of enzymes synthesizing precursors or by destroying the activity of one or more genes which are involved in the breakdown of the amino acids, it is possible to increase the yield, production and/or production efficiency of amino acids in the host organism, such as the plants or the microorganisms.

Accordingly, in one embodiment, the process according to the invention relates to a process which comprises:
a) providing a photosynthetic active organism, preferably a microorganism, a plant or a plant tissue or a plant;
b) increasing an activity of a polypeptide of the invention or the polypeptide used in the method of the invention or a homolog thereof, e.g. as indicated in Table II, columns 5 or 7, or of a polypeptide being encoded by the nucleic acid molecule of the present invention and described below, i.e. conferring an increase of nitrogen or nitrogen containing compounds in the organism, preferably in a photosynthetic active organism, preferably a microorganism, a plant or a plant tissue or a plant,
c) growing the organism, preferably a photosynthetic active organism, preferably a microorganism, a plant or a plant tissue or a plant, under conditions which permit the accumulation and/or production of nitrogen or nitrogen containing compounds respectively in the organism, preferably a photosynthetic active organism, preferably a microorganism, a plant or a plant tissue or a plant.
d) After the above-described increasing (which as defined above also encompasses the generating of an activity in an organism, i.e. a *de novo* activity), for example after the introduction and the expression of the nucleic acid molecules of the invention or described in the methods or processes according to the invention, the organism according to the invention, advantageously, a photosynthetic active organism, preferably a microorganism, a plant or a plant tissue or a plant, is grown and subsequently harvested.

Suitable organisms or host organisms (transgenic organism) for the nucleic acid molecule used according to the invention and for the inventive process, the nucleic acid construct or the vector (both as described below) are, in principle, all organisms which are capable of synthesizing nitrogen or nitrogen containing compounds, and which are suitable for the activation, introduction or stimulation of genes. Examples which may be mentioned are plants, microorganisms such as fungi, bacteria, yeasts, alga or diatom, transgenic or obtained by site directed mutagenesis or random mutagenesis combined with specific selection procedures. Preferred organisms are those which are naturally capable of accumlating and/or synthesizing nitrogen or nitrogen containing compounds in substantial amounts, like fungi, yeasts, bacteria or plants. In principle, transgenic animals, for example Caenorhabditis elegans, are also suitable as host organisms.

In the event that the transgenic organism is a microorganism, such as a eukaryotic organism, for example a fungus, an alga, diatom or a yeast in particular a fungus, alga, diatom or yeast selected from the families Chaetomiaceae, Choanephoraceae, Cryptococcaceae, Cunninghamellaceae, Demetiaceae, Moniliaceae, Mortierellaceae, Mucoraceae, Pythiaceae, Sacharomycetaceae, Saprolegniaceae, Schizosacharomycetaceae, Sodariaceae, Sporobolomycetaceae Tuberculariaceae, Adelotheciaceae, Dinophyceae, Ditrichaceae or Prasinophyceae, or a prokaryotic organism, for example a bacterium or blue alga, in particular a bacterium from the families Actinomycetaceae, Bacillaceae, Brevibacteriaceae, Corynebacteriaceae, Enterobacteriacae, Gordoniaceae, Nocardiaceae, Micrococcaceae, Mycobacteriaceae, Pseudomonaceae, Rhizobiaceae or Streptomycetaceae, this microorganism is grown on a solid or in a liquid medium which is known to the skilled worker and suits the organism. After the growing phase, the organisms can be harvested.

The microorganisms or the recovered, and if desired isolated, respective nitrogen or nitrogen containing compounds like amino acids can then be processed further directly into foodstuffs or animal feeds or for other applications, for example according to the disclosures made in EP-B-0 533 039 or EP-A-0 615 693, which are expressly incorporated herein by reference. The fermentation broth or fermentation products can be purified in the customary manner by extraction and precipitation or via ion exchangers and other methods known to the person skilled in the art and described herein below. Products of these different work-up procedures are amino acids or amino acid compositions which still comprise fermentation broth and cell components in different amounts, advantageously in the range of from 0 to 99% by weight, preferably below 80% by weight, especially preferably between below 50% by weight.

%

Preferred strains are strains selected from the group consisting of Bacillaceae, Brevibacteriaceae, Corynebacteriaceae, Nocardiaceae, Mycobacteriaceae, Streptomycetaceae, Enterobacteriaceae such as *Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp., Nocardia rhodochrous (Rhodococcus rhodochrous) , Mycobacterium rhodochrous, Streptomyces lividans* and *Escherichia coli* especially *Escherichia coli K12.*

In addition particular preferred strains are strains selected from the group consisting of Cryptococcaceae, Saccharomycetaceae, Schizosaccharomycetacease such as the genera Candida, Hansenula, Pichia, Saccharomyces and Schizosaccharomyces preferred are strains selected from the group consisting of the species *Rhodotorula rubra, Rhodotorula glutinis, Rhodotorula graminis, Yarrowia lipolytica, Sporobolomyces salmonicolor, Sporobolomyces shibatanus, Saccharomyces cerevisiae, Candida boidinii, Candida bombicola, Candida cylindracea, Candida parapsilosis, Candida rugosa, Candida tropicalis, Pichia methanolica and Pichia pastoris.*

Anacardiaceae such as the genera Pistacia, Mangifera, Anacardium e.g. the species *Pistacia vera* [pistachios, Pistazie], *Mangifer indica* [Mango] or *Anacardium occidentale* [Cashew]; Asteraceae such as the genera Calendula, Carthamus, Centaurea, Cichorium, Cynara, Helianthus, Lactuca, Locusta, Tagetes, Valeriana e.g. the species *Calendula officinalis* [Marigold], *Carthamus tinctorius* [safflower], *Centaurea cyanus* [cornflower], *Cichorium intybus* [blue daisy], *Cynara scolymus* [Artichoke], *Helianthus annus* [sunflower], Lactuca sativa, *Lactuca crispa, Lactuca esculenta, Lactuca scariola L. ssp. sativa, Lactuca scariola L. var. integrata*, *Lactuca scariola L. var. integrifolia, Lactuca sativa subsp. romana, Locusta communis, Valeriana locusta* [lettuce], *Tagetes lucida, Tagetes erecta or Tagetes tenuifolia* [Marigold]; Apiaceae such as the genera Daucus e.g. the species *Daucus carota* [carrot]; Betulaceae such as the genera Corylus e.g. the species *Corylus avellana* or *Corylus colurna* [hazelnut]; Boraginaceae such as the genera Borago e.g. the species *Borago officinalis* [borage]; Brassicaceae such as the genera Brassica, Melanosinapis, Sinapis, Arabadopsis e.g. the species *Brassica napus, Brassica rapa* ssp. [canola, oilseed rape, turnip rape], *Sinapis arvensis Brassica juncea, Brassica juncea var. juncea, Brassica juncea var. crispifolia, Brassica juncea var. foliosa, Brassica nigra, Brassica sinapioides, Melanosinapis communis* [mustard], *Brassica oleracea* [fodder beet] or Arabidopsis thaliana; Bromeliaceae such as the genera Anana, Bromelia e.g. the species *Anana comosus, Ananas ananas* or *Bromelia comosa* [pineapple]; Caricaceae such as the genera Carica e.g. the species *Carica papaya* [papaya]; Cannabaceae such as the genera Cannabis e.g. the species *Cannabis sative* [hemp], Convolvulaceae such as the genera Ipomea, Convolvulus e.g. the species *Ipomoea batatus, Ipomoea pandurata, Convolvulus batatas, Convolvulus tiliaceus, Ipomoea fastigiata, Ipomoea tiliacea, Ipomoea triloba* or *Convolvulus panduratus* [sweet potato, Man of the Earth, wild potato], Chenopodiaceae such as the genera Beta, i.e. the species *Beta vulgaris, Beta vulgaris var. altissima, Beta vulgaris var. Vulgaris, Beta maritima, Beta vulgaris var. perennis, Beta vulgaris var. conditiva* or *Beta vulgaris var. esculenta* [sugar beet]; Cucurbitaceae such as the genera Cucubita e.g. the species *Cucurbita maxima, Cucurbita mixta, Cucurbita* pepo or *Cucurbita moschata* [pumpkin, squash]; Elaeagnaceae such as the genera Elaeagnus e.g. the species Olea *europaea* [olive]; Ericaceae such as the genera Kalmia e.g. the species *Kalmia latifolia, Kalmia angustifolia, Kalmia microphylla, Kalmia polifolia, Kalmia occidentalis, Cistus chamaerhodendros* or *Kalmia lucida* [American laurel, broad-leafed laurel, calico bush, spoon wood, sheep laurel, alpine laurel, bog laurel, western bog-laurel, swamp-laurel]; Euphorbiaceae such as the genera Manihot, Janipha, Jatropha, Ricinus e.g. the species *Manihot utilissima, Janipha manihot,, Jatropha manihot., Manihot aipil, Manihot dulcis, Manihot manihot, Manihot melanobasis, Manihot esculenta* [manihot, arrowroot, tapioca, cassava] or *Ricinus communis* [castor bean, Castor Oil Bush, Castor Oil Plant, Palma Christi, Wonder Tree]; Fabaceae such as the genera Pisum, Albizia, Cathormion, Feuillea, Inga, Pithecolobium, Acacia, Mimosa, Medicajo, Glycine, Dolichos, Phaseolus, Soja e.g. the species *Pisum sativum, Pisum arvense, Pisum humile* [pea], *Albizia berteriana, Albizia julibrissin, Albizia lebbeck*, *Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa* [bastard logwood, silk tree, East Indian Walnut], *Medicago sativa, Medicago falcata, Medicago varia* [alfalfa] *Glycine max Dolichos soja, Glycine gracilis, Glycine hispida, Phaseolus max, Soja hispida* or *Soja max* [soybean]; Geraniaceae such as the genera Pelargonium, Cocos, Oleum e.g. the species Cocos *nucifera, Pelargonium grossularioides* or *Oleum cocois* [coconut]; Gramineae such as the genera Saccharum e.g. the species *Saccharum officinarum;* Juglandaceae such as the genera Juglans, Wallia e.g. the species *Juglans regia*, *Juglans ailanthifolia, Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra* or *Wallia nigra* [walnut, black walnut, common walnut, persian walnut, white walnut, butternut, black walnut]; Lauraceae such as the genera Persea, Laurus e.g. the species laurel *Laurus nobilis* [bay, laurel, bay laurel, sweet bay], *Persea americana Persea americana, Persea gratissima* or *Persea persea* [avocado]; Leguminosae such as the genera Arachis e.g. the species *Arachis hypogaea* [peanut]; Linaceae such as the genera Linum, Adenolinum e.g. the species *Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense* or *Linum trigynum* [flax, linseed]; Lythrarieae such as the genera Punica e.g. the species *Punica granatum* [pomegranate]; Malvaceae such as the genera Gossypium e.g. the species *Gossypium hirsutum, Gossypium arboreum, Gossypium barbadense, Gossypium herbaceum* or *Gossypium thurberi* [cotton]; Musaceae such as the genera Musa e.g. the species *Musa nana, Musa acuminata, Musa paradisiaca, Musa* spp. [banana]; Onagraceae such as the genera Camissonia, Oenothera e.g. the species *Oenothera biennis* or *Camissonia brevipes* [primrose, evening primrose]; Palmae such as the genera Elacis e.g. the species *Elaeis guineensis* [oil plam]; Papaveraceae such as the genera Papaver e.g. the species *Papaver orientale, Papaver rhoeas, Papaver dubium* [poppy, oriental poppy, corn poppy, field poppy, shirley poppies, field poppy, long-headed poppy, long-pod poppy]; Pedaliaceae such as the genera Sesamum e.g. the species *Sesamum indicum* [sesame]; Piperaceae such as the genera Piper, Artanthe, Peperomia, Steffensia e.g. the species *Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata.* [Cayenne pepper, wild pepper]; Poaceae such as the genera Hordeum, Secale, Avena, Sorghum, Andropogon, Holcus, Panicum, Oryza, Zea, Triticum e.g. the species *Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum* [barley, pearl barley, foxtail barley, wall barley, meadow barley], *Secale cereale* [rye], Avena sativa, *Avena fatua, Avena byzantina, Avena fatua var. sativa, Avena hybrida* [oat], *Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum* millet, *Panicum militaceum [Sorghum, millet], Oryza sativa, Oryza latifolia* [rice], *Zea mays* [corn, maize] *Triticum aestivum, Triticum durum, Triticum turgidum, Triticum hybernum, Triticum macha, Triticum sativum* or *Triticum vulgare* [wheat, bread wheat, common wheat], Proteaceae such as the genera Macadamia e.g. the species *Macadamia intergrifolia* [macadamia]; Rubiaceae such as the genera Coffea e.g. the species Cofea spp., *Coffea arabica, Coffea canephora* or *Coffea liberica* [coffee]; Scrophulariaceae such as the genera Verbascum e.g. the species *Verbascum blattaria, Verbascum chaixii, Verbascum densiflorum, Verbascum lagurus, Verbascum longifolium, Verbascum lychnitis, Verbascum nigrum, Verbascum olympicum, Verbascum phlomoides, Verbascum phoenicum, Verbascum pulverulentum* or *Verbascum thapsus* [mullein, white moth mullein, nettle-leaved mullein, dense-flowered mullein, silver mullein, long-leaved mullein, white mullein, dark mullein, greek mullein, orange mullein, purple mullein, hoary mullein, great mullein]; Solanaceae such as the genera Capsicum, Nicotiana, Solanum, Lycopersicon e.g. the species *Capsicum annuum, Capsicum annuum var. glabriusculum, Capsicum frutescens* [pepper], *Capsicum annuum* [paprika], *Nicotiana tabacum, Nicotiana alata, Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris* [tobacco], *Solanum tuberosum* [potato], *Solanum melongena* [egg-plant] (*Lycopersicon esculentum, Lycopersicon lycopersicum., Lycopersicon pyriforme, Solanum integrifolium* or *Solanum lycopersicum* [*tomato*]; Sterculiaceae such as the genera Theobroma e.g. the species *Theobroma* cacao [cacao]; Theaceae such as the genera Camellia e.g. the species *Camellia sinensis*) [tea] can either be donor organisms for the nucleic acids or polypeptides of the invention or used in the present invention or represents preferred host organims.

Particular preferred host plants are plants selected from the group consisting of Asteraceae such as the genera Helianthus, Tagetes e.g. the species *Helianthus annus* [sunflower], *Tagetes lucida, Tagetes erecta or Tagetes tenuifolia* [Marigold], Brassicaceae such as the genera Brassica, Arabadopsis e.g. the species *Brassica napus, Brassica rapa* ssp. [canola, oilseed rape, turnip rape] or Arabidopsis thaliana. Fabaceae such as the genera Glycine e.g. the species *Glycine max, Soja hispida* or *Soja max* [soybean]. Linaceae such as the genera Linum e.g. the species *Linum usitatissimum,* [flax, linseed]; Poaceae such as the genera Hordeum, Secale, Avena, Sorghum, Oryza, Zea, Triticum e.g. the species *Hordeum vulgare* [barley]; *Secale* cereale [rye], Avena sativa, *Avena fatua, Avena byzantina, Avena fatua var. sativa, Avena hybrida* [oat], *Sorghum bicolor* [Sorghum, millet], *Oryza sativa, Oryza latifolia* [rice], *Zea mays* [corn, maize] *Triticum aestivum, Triticum durum, Triticum turgidum, Triticum hybernum, Triticum macha, Triticum sativum* or *Triticum vulgare* [wheat, bread wheat, common wheat]; Solanaceae such as the genera Solanum, Lycopersicon e.g. the species *Solanum tuberosum* [potato], *Lycopersicon esculentum, Lycopersicon lycopersicum., Lycopersicon pyriforme, Solanum integrifolium* or *Solanum lycopersicum* [*tomato*]. A further preferred host organism is cotton for example *Gossypium hirsutum, Gossypium arboreum, Gossypium barbadense, Gossypium herbaceum or Gossypium thurberi.*

All abovementioned organisms can in princible also function as donor organisms.

With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
**a)** a nucleic acid sequence selected from the group as indicated in Table I, columns 5 or 7, lines 1, 2, 3, 4 and/or 5,or a derivative thereof, or
**b)** a genetic regulatory element, for example a promoter, which is functionally linked to the nucleic acid sequence as indicated in Table I, columns 5 or 7, lines 1, 2, 3 4 and/or 5, or a derivative thereof, or
**c)** (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

The use of the nucleic acid sequence according to the invention or of the nucleic acid construct according to the invention for the generation of transgenic plants is therefore also subject matter of the invention.

In an advantageous embodiment of the invention, the organism takes the form of a plant whose nitrogen or nitrogen containing compound content is modified advantageously owing to the nucleic acid molecule of the present invention expressed. This is important for plant breeders for several reasons:
a) The fast majority of nitrogen is present in cells in form of protein bound amino acids. Therefore an increased nitrogen or nitrogen containing compound content reflects an increased protein content and therefore additional nutritional value for the feed industry.
b) A method for increased nitrogen uptake and/or accumulation of nitrogen or nitrogen containing compounds might allow to reduce the application of nitrogen-fertilzers, which in turn lead to reduced costs and environmental benefits.
c) A method for increased nitrogen uptake and/or accumulation might support plant growth, health and productivity, preferably under nitrogen limited conditions.

In one embodiment, after an activity of a polypeptide of the present invention or used in the process of the present invention has been increased or generated, or after the expression of a nucleic acid molecule or polypeptide according to the invention has been generated or increased, the transgenic plant generated can be grown on or in a nutrient medium or else in the soil and subsequently harvested. In one embodiment the transgenic plant generated can be grown under nitrogen limiting conditions.

The plants or parts thereof, e.g. the leaves, roots, flowers, and/or stems and/or other harvestable material as described below, can then be used directly as foodstuffs or animal feeds or else be further processed. Again, the amino acids can be purified further in the customary manner via extraction and precipitation or via ion exchangers and other methods known to the person skilled in the art and described herein below. Products which are suitable for various applications and which result from these different processing procedures are amino acids or amino acid compositions which can still comprise further plant components in different amounts, advantageously in the range of from 0 to 99% by weight, preferably from below 90% by weight, especially preferably below 80% by weight. The plants can also advantageously be used directly without further processing, e.g. as feed or for extraction.

The chemically pure nitrogen containing compounds or chemically pure compositions comprising nitrogen or nitrogen containing compounds may also be produced by the process described above. To this end, nitrogen or nitrogen containing compounds or the compositions are isolated in the known manner from an organism according to the invention, such as the microorganisms, non-human animal or the plants, and/or their culture medium in which or on which the organisms had been grown,. These chemically pure nitrogen containing compounds or said compositions are advantageous for applications in the field of the feed orfood industry.

In a preferred embodiment, the present invention relates to a process for for the enhanced nitrogen assimilation, accumulation and/or utilization in photosynthetic active organisms, which comprises, increasing or generating in an organism or a part or a compartment thereof the expression of at least one nucleic acid molecule comprising a nucleic acid molecule selected from the group consisting of:
a) nucleic acid molecule encoding a polypeptide selected from the group as shown in table II, columns 5 and 7 or a fragment thereof, which confers enhanced nitrogen assimilation, accumulation and/or utilization,
b) nucleic acid molecule comprising of a nucleic acid molecule selected from the group as shown in table I, columns 5 and 7 which confers enhanced nitrogen assimilation, accumulation and/or utilization
c) nucleic acid molecule whose sequence can be deduced from a polypeptide sequence encoded by a nucleic acid molecule of (a) or (b) as a result of the degeneracy of the genetic code and which confers enhanced nitrogen assimilation, accumulation and/or utilization
d) nucleic acid molecule which encodes a polypeptide which has at least 50% identity with the amino acid sequence of the polypeptide encoded by the nucleic acid molecule of (a) to (c) and which confers enhanced nitrogen assimilation, accumulation and/or utilization
e) nucleic acid molecule which hybidizes with a nucleic acid molecule of (a) to (c) under stringent hybridisation and which confers enhanced nitrogen assimilation, accumulation and/or utilization
f) nucleic acid molecule which encompasses a nucleic acid molecule which is obtained by amplifying nucleic acid molecules from a cDNA library or a genomic library using the primers or primer pairs as indicated in table III, column 7 and which confers enhanced nitrogen assimilation, accumulation and/or utilization
g) nucleic acid molecule encoding a polypeptide which is isolated with the aid of monoclonal antibodies against a polypeptide encoded by one of the nucleic acid molecules of (a) to (f) and which confers enhanced nitrogen assimilation, accumulation and/or utilization
h) nucleic acid molecule encoding a polypeptide comprising a consensus as shown in table IV, columns 7 and which confers enhanced nitrogen assimilation, accumulation and/or utilization
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and which confers enhanced nitrogen assimilation, accumulation and/or utilization
or comprising a sequence which is complementary thereto.

In one embodiment, the nucleic acid molecule used in the process of the invention distinguishes over the sequence indicated in Table IA, columns 5 or 7, by one or more nucleotides. In one embodiment, the nucleic acid molecule used in the process of the invention does not consist of the sequence shown in Table I A, columns 5 or 7,: In one embodiment, the nucleic acid molecule used in the process of the invention is less than 100%, 99,999%, 99,99%, 99,9% or 99% identical to a sequence indicated in Table I A, columns 5 or 7,. In another embodiment, the nucleic acid molecule does not encode a polypeptide of a sequence indicated in Table II A, columns 5 or 7,.

In one embodiment, the nucleic acid molecule used in the process of the invention distinguishes over the sequence indicated in Table I B, columns 5 or 7, by one or more nucleotides. In one embodiment, the nucleic acid molecule used in the process of the invention does not consist of the sequence shown in indicated in Table I B, columns 5 or 7.

In one embodiment, the nucleic acid molecule used in the process of the invention is less than 100%, 99,999%, 99,99%, 99,9% or 99% identical to a sequence indicated in Table I B, columns 5 or 7,. In another embodiment, the nucleic acid molecule does not encode a polypeptide of a sequence indicated in Table II B, columns 5 or 7.

In one embodiment, the nucleic acid molecule of the invention or used in the process of the invention distinguishes over the sequence indicated in Table I, columns 5 or 7, by one or more nucleotides. In one embodiment, the nucleic acid molecule of the invention or the nucleic acid used in the process of the invention does not consist of the sequence shown in indicated in Table I, columns 5 or 7,. In one embodiment, the nucleic acid molecule of the present invention is less than 100%, 99,999%, 99,99%, 99,9% or 99% identical to a sequence indicated in Table I, columns 5 or 7,. In another embodiment, the nucleic acid molecule does not encode a polypeptide of a sequence indicated in Table II, columns 5 or 7.

Unless otherwise specified, the terms "polynucleotides", "nucleic acid" and "nucleic acid molecule" are interchangeably in the present context. Unless otherwise specified, the terms "peptide", "polypeptide" and "protein" are interchangeably in the present context. The term "sequence" may relate to polynucleotides, nucleic acids, nucleic acid molecules, peptides, polypeptides and proteins, depending on the context in which the term "sequence" is used. The terms "gene(s)", "polynucleotide", "nucleic acid sequence", "nucleotide sequence", or "nucleic acid molecule(s)" as used herein refers to a polymeric form of nucleotides of any length, either ribonucleotides or deoxyribonucleotides. The terms refer only to the primary structure of the molecule.

Thus, The terms "gene(s)", "polynucleotide", "nucleic acid sequence", "nucleotide sequence", or "nucleic acid molecule(s)" as used herein include double-and single-stranded DNA and RNA. They also include known types of modifications, for example, methylation, "caps", substitutions of one or more of the naturally occurring nucleotides with an analog. Preferably, the DNA or RNA sequence of the invention comprises a coding sequence encoding the herein defined polypeptide.

A "coding sequence" is a nucleotide sequence, which is transcribed into mRNA and/or translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleotide sequences or genomic DNA, while introns may be present as well under certain circumstances.

Nucleic acid molecules with the sequence as indicated in Table I, columns 5 or 7, nucleic acid molecules which are derived from a amino acid sequences as indicated in Table II, columns 5 or 7, or from polypeptides comprising the consensus sequence as indicated in Table IV, columns 7, or their derivatives or homologues encoding polypeptides with the enzymatic or biological activity of a polypeptide as indicated in Table II, column 3, 5 or 7, or e.g. conferring a increase of nitrogen or nitrogen containing compounds after increasing its expression or activity in the cytosol or in the plastids are advantageously increased in the process according to the invention.

In one embodiment, said sequences are cloned into nucleic acid constructs, either individually or in combination. These nucleic acid constructs enable an optimal accumulation and/or synthesis of nitrogen or nitrogen containing compounds respectively produced in the process according to the invention.

Nucleic acid molecules, which are advantageous for the process according to the invention and which encode polypeptides with an activity of a polypeptide of the invention or the polypeptide used in the method of the invention or used in the process of the invention, e.g. of a protein as indicated in Table II, column 5, or being encoded by a nucleic acid molecule indicated in Table I, column 5, or of its homologs, e.g. as indicated in Table II, column 7, can be determined from generally accessible databases.

Those, which must be mentioned, in particular in this context are general gene databases such as the EMBL database (Stoesser G. et al., Nucleic Acids Res 2001, Vol. 29,17-21), the GenBank database (Benson D.A. et al., Nucleic Acids Res 2000, Vol. 28,15-18), or the PIR database (Barker W. C. et al., Nucleic Acids Res. 1999, Vol. 27, 39-43). It is furthermore possible to use organism-specific gene databases for determining advantageous sequences, in the case of yeast for example advantageously the SGD database (Cherry J. M. et al., Nucleic Acids Res. 1998, Vol. 26, 73-80) or the MIPS database (Mewes H.W. et al., Nucleic Acids Res. 1999, Vol. 27, 44-48), in the case of E. coli the GenProtEC database (http://web.bham.ac.uk/bcm4ght6/res.html), and in the case of Arabidopsis the TAIR-database (Huala, E. et al., Nucleic Acids Res. 2001 Vol. 29(1), 102-5) or the MIPS database.

The nucleic acid molecules used in the process according to the invention take the form of isolated nucleic acid sequences, which encode polypeptides with an activity of a polypeptide selected from the group as indicated in Table I, column 3, lines 1, 2,3 4 and/or 5 or having the sequence of a polypeptide as indicated in Table II, columns 5 and 7, lines 1, 2, 3, 4 and/or 5 and conferring an increase of nitrogen or nitrogen containing compounds.

The nucleic acid sequence(s) used in the process for the production of nitrogen or nitrogen containing compounds in transgenic organisms originate advantageously from an eukaryote but may also originate from a prokaryote or an archebacterium, thus it can derived from e.g. a microorganism, an animal or a plant.

For the purposes of the invention, as a rule the plural is intended to encompass the singular and vice versa.

In order to improve the introduction of the nucleic acid sequences and the expression of the sequences in the transgenic organisms, which are used in the process, the nucleic acid sequences are incorporated into a nucleic acid construct and/or a vector. In addition to the herein described sequences which are used in the process according to the invention, further nucleic acid sequences, advantageously encoding nitrogen assimilation or amino acid biosynthesis genesor nutritional valuable storage proteins, may additionally be present in the nucleic acid construct or in the vector and may be introduced into the organism together. However, these additional sequences may also be introduced into the organisms via other, separate nucleic acid constructs or vectors.

Using the herein mentioned cloning vectors and transformation methods such as those which are published and cited in: Plant Molecular Biology and Biotechnology (CRC Press, Boca Raton, Florida), chapter 6/7, pp. 71-119 (1993); F.F. White, Vectors for Gene Transfer in Higher Plants; in: Transgenic Plants, vol. 1, Engineering and Utilization, Ed.: Kung and R. Wu, Academic Press, 1993, 15-38; B. Jenes et al., Techniques for Gene Transfer, in: Transgenic Plants, vol. 1, Engineering and Utilization, Ed.: Kung and R. Wu, Academic Press (1993), 128-143; Potrykus, Annu. Rev. Plant Physiol. Plant Molec. Biol. 42 (1991), 205-225)) and further cited below, the nucleic acids may be used for the recombinant modification of a wide range of organisms, in particular prokaryotic or eukaryotic microorganisms or plants, so that they become a better and more efficient accumulater and/or producer of nitrogen or nitrogen containing compounds respectively according to the invention. This improved accmulation of nitrogen or production of nitrogen containing compounds or products derived there from, such as proteins, can be brought about by a direct effect of the manipulation or by an indirect effect of this manipulation.

In one embodiment, the nucleic acid molecule according to the invention originates from a plant, such as a plant selected from the families Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae or Poaceae and preferably from a plant selected from the group of the families Apiaceae, Asteraceae, Brassicaceae, Cucurbitaceae, Fabaceae, Papaveraceae, Rosaceae, Solanaceae, Liliaceae or Poaceae. Preferred are crop plants and in particular plants mentioned herein above as host plants such as the families and genera mentioned above for example preferred the species *Anacardium occidentale, Calendule officinalis, Carthamus tinctorius, Cichorium intybus, Cynara scolymus*, *Helianthus annus, Tagetes lucida, Tagetes erecta, Tagetes tenuifolia*; *Daucus carota; Corylus avellana, Corylus colurna*, *Borago officinalis; Brassica napus, Brassica rapa* ssp., *Sinapis arvensis Brassica juncea, Brassica juncea var. juncea, Brassica juncea var. crispifolia, Brassica juncea var. foliosa, Brassica nigra, Brassica sinapioides, Melanosinapis communis, Brassica oleracea,* Arabidopsis thaliana, *Anana comosus, Ananas ananas, Bromelia comosa, Carica papaya, Cannabis sative, Ipomoea batatus, Ipomoea pandurata, Convolvulus batatas, Convolvulus tiliaceus, Ipomoea fastigiata, Ipomoea tiliacea, Ipomoea triloba, Convolvulus panduratus, Beta vulgaris, Beta vulgaris var. altissima*, *Beta vulgaris var. vulgaris*, *Beta maritima, Beta vulgaris var. perennis*, *Beta vulgaris var. conditiva*, *Beta vulgaris var. esculenta*, *Cucurbita maxima, Cucurbita mixta, Cucurbita pepo, Cucurbita moschata, Olea europaea, Manihot utilissima, Janipha manihot*,, *Jatropha manihot., Manihot aipil, Manihot dulcis, Manihot manihot*, *Manihot melanobasis, Manihot esculenta, Ricinus communis, Pisum sativum, Pisum arvense, Pisum humile, Medicago sativa, Medicago falcata, Medicago varia, Glycine max Dolichos soja, Glycine gracilis, Glycine hispida, Phaseolus max, Soja hispida, Soja max, Cocos nucifera, Pelargonium grossularioides, Oleum cocoas, Laurus nobilis, Persea americana, Arachis hypogaea, Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense, Linum trigynum, Punica granatum, Gossypium hirsutum, Gossypium arboreum, Gossypium barbadense, Gossypium herbaceum, Gossypium thurberi, Musa nana, Musa acuminata, Musa paradisiaca, Musa* spp., *Elaeis guineensis, Papaver orientale, Papaver rhoeas, Papaver dubium, Sesamum indicum, Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata, , Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum,* Avena sativa, *Avena fatua, Avena byzantina, Avena fatua var. sativa, Avena hybrida, Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cemuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum* millet, *Panicum militaceum, Zea mays, Triticum aestivum, Triticum durum, Triticum turgidum, Triticum hybernum, Triticum macha, Triticum sativum* or *Triticum vulgare, Cofea spp., Coffea arabica, Coffea canephora, Coffea liberica, Capsicum annuum, Capsicum annuum var. glabriusculum, Capsicum frutescens, Capsicum annuum, Nicotiana tabacum, Solanum tuberosum, Solanum melongena, Lycopersicon esculentum, Lycopersicon lycopersicum., Lycopersicon pyriforme, Solanum integrifolium, Solanum lycopersicum Theobroma cacao* or *Camellia sinensis.*

In one embodiment, the nucleic acid molecule sequence for the accumulation and/or production of nitrogen or nitrogen containing compounds originates advantageously from a microorganism as mentioned above under host organism such as a fungus for example the genera Aspergillus, Penicillium or Claviceps or from yeasts such as the genera Pichia, Torulopsis, Hansenula, Schizosaccharomyces, Candida, Rhodotorula or Saccharomyces, very especially advantageously from the yeast of the family Saccharomycetaceae, such as the advantageous genus Saccharomyces and the very advantageous genus and species Saccharomyces cerevisiae.

The skilled worker knows other suitable sources for nucleic acids that can be used for the accumulation and/or production of nitrogen or nitrogen containing compounds repectively. They include in general all prokaryotic or eukaryotic cells, preferably unicellular microorganisms, such as fungi like the genus Claviceps or Aspergillus or gram-positive bacteria such as the genera Bacillus, Corynebacterium, Micrococcus, Brevibacterium, Rhodococcus, Nocardia, Caseobacter or Arthrobacter or gram-negative bacteria such as the genera Escherichia, Flavobacterium or Salmonella, or yeasts such as the genera Rhodotorula, Hansenula or Candida.

%

However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, columns 5 or 7, or the functional homologues thereof as described herein, preferably conferring above-mentioned activity, i.e. conferring a increase of nitrogen or nitrogen containing compounds after increasing its activity. In peferred embodiments of the present invention the polypeptide sequences indicated in Table II, columns 5 or 7, or the functional homologues thereof as described herein, preferably conferring above-mentioned activity, i.e. conferring a increase of nitrogen or nitrogen containing compounds after increasing its activity, are expressed from nucleic acid sequences which have been optimized according the codon usage in the selected host organism or organelle. The person skilled in the art is familiar with sources for tables showing the preferred codon usage for the different amino acids in the selected organims.

In the process according to the invention nucleic acid sequences can be used, which, if appropriate, contain synthetic, non-natural or modified nucleotide bases, which can be incorporated into DNA or RNA. Said synthetic, non-natural or modified bases can for example increase the stability of the nucleic acid molecule outside or inside a cell. The nucleic acid molecules of the invention can contain the same modifications as aforementioned.

As used in the present context the term "nucleic acid molecule" may also encompass the untranslated sequence located at the 3' and at the 5' end of the coding gene region, for example at least 500, preferably 200, especially preferably 100, nucleotides of the sequence upstream of the 5' end of the coding region and at least 100, preferably 50, especially preferably 20, nucleotides of the sequence downstream of the 3' end of the coding gene region. It is often advantageous only to choose the coding region for cloning and expression purposes.

Preferably, the nucleic acid molecule used in the process according to the invention or the nucleic acid molecule of the invention is an isolated nucleic acid molecule.

An "isolated" polynucleotide or nucleic acid molecule is separated from other polynucleotides or nucleic acid molecules, which are present in the natural source of the nucleic acid molecule. An isolated nucleic acid molecule may be a chromosomal fragment of several kb, or preferably, a molecule only comprising the coding region of the gene. Accordingly, an isolated nucleic acid molecule of the invention may comprise chromosomal regions, which are adjacent 5' and 3' or further adjacent chromosomal regions, but preferably comprises no such sequences which naturally flank the nucleic acid molecule sequence in the genomic or chromosomal context in the organism from which the nucleic acid molecule originates (for example sequences which are adjacent to the regions encoding the 5'- and 3'-UTRs of the nucleic acid molecule). In various embodiments, the isolated nucleic acid molecule used in the process according to the invention may, for example comprise less than approximately 5 kb, 4 kb, 3 kb, 2 kb, 1 kb, 0.5 kb or 0.1 kb nucleotide sequences which naturally flank the nucleic acid molecule in the genomic DNA of the cell from which the nucleic acid molecule originates.

The nucleic acid molecules used in the process, for example the polynucleotides of the invention or of a part thereof can be isolated using molecular-biological standard techniques and the sequence information provided herein. Also, for example a homologous sequence or homologous, conserved sequence regions at the DNA or amino acid level can be identified with the aid of comparison algorithms. The former can be used as hybridization probes under standard hybridization techniques (for example those described in Sambrook et al., Molecular Cloning: A Laboratory Manual. 2nd Ed., Cold Spring Harbor Laboratory, Cold Spring Harbor Laboratory Press, Cold Spring Harbor, NY, 1989) for isolating further nucleic acid sequences useful in this process.

A nucleic acid molecule encompassing a complete sequence of the nucleic acid molecules used in the process, for example the polynucleotide of the invention, or a part thereof may additionally be isolated by polymerase chain reaction, oligonucleotide primers based on this sequence or on parts thereof being used. For example, a nucleic acid molecule comprising the complete sequence or part thereof can be isolated by polymerase chain reaction using oligonucleotide primers which have been generated on the basis of this sequence for example, mRNA can be isolated from cells (for example by means of the guanidinium thiocyanate extraction method of Chirgwin et al. (1979) Biochemistry 18:5294-5299) and cDNA can be generated by means of reverse transcriptase (for example Moloney MLV reverse transcriptase, available from Gibco/BRL, Bethesda, MD, or AMV reverse transcriptase, obtainable from Seikagaku America, Inc., St.Petersburg, FL).

Synthetic oligonucleotide primers for the amplification, e.g. as the pairs indicated in Table III, columns 7, lines 1, 2, 3 4 and/or 5, by means of polymerase chain reaction can be generated on the basis of a sequence shown herein, for example the sequence as indicated in Table I, columns 5 or 7, lines 1, 2, 3, 4 and/or 5 or the nucleic acid sequences derived from polypeptide sequences as indicated in Table II, columns 5 or 7, lines 1, 2, 3, 4 and/or 5.

Moreover, it is possible to identify conserved regions from various organisms by carrying out protein sequence alignments with the polypeptide encoded by the nucleic acid molecules of the present invention, in particular with the sequences selected from the group as shown in column 5 or 7 of Table II, from which conserved regions, and in turn, degenerate primers can be derived.

Conserved regions are those, which show a very little variation in the amino acid in one particular position of several homologs from different origin. The consenus sequence and polypeptide motifs shown in column 7 of Table IV are derived from said aligments. Moreover, it is possible to identify conserved regions from various organisms by carrying out protein sequence alignments with the polypeptide encoded by the nucleic acid of the present invention, in particular with the polypeptide molecules shown in column 5 or 7 of Table II, from which conserved regions, and in turn, degenerate primers can be derived.
In one advantageous embodiment, in the method of the present invention the activity of a polypeptide is increased comprising or consisting of a consensus sequence or a polypeptide motif shown in table IV column 7 and in one another embodiment, the present invention relates to a polypeptide comprising or consisting of a consensus sequence or a polypeptide motif shown in table IV, column 7 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid. In one embodiment not more than 15%, preferably 10%, even more preferred 5%, 4%, 3%, or 2%, most preferred 1 % or 0% of the amino acid position indicated by a letter are/is replaced another amino acid. In one embodiment 20 or less, preferably 15 or 10, preferably 9, 8, 7, or 6, more preferred 5 or 4, even more preferred 3, even more preferred 2, even more preferred 1, most preferred 0 amino acids are inserted into a consensus sequence or protein motif.
The consensus sequence was derived from a multiple alignment of the sequences as listed in column 5 and 7 of table II. The letters represent the one letter amino acid code and indicate that the amino acids are conserved in all aligned proteins. The letter X stands for amino acids, which are not conserved in all sequences. In one example, in the cases where only a small selected subset of amino acids are possible at a certain position these amino acids are given in brackets. The number of given X indicates the distances between conserved amino acid residues, e.g. Y-x(21,23)-F means that conserved tyrosine and phenylalanine residues are separated from each other by minimum 21 and maximum 23 amino acid residues in all investigated sequences.
Conserved domains were identified from all sequences and are described using a subset of the standard Prosite notation, e.g the pattern Y-x(21,23)-[FW] means that a conserved tyrosine is separated by minimum 21 and maximum 23 amino acid residues from either a phenylalanine or tryptophane.
Conserved patterns were identified with the software tool MEME version 3.5.1 or manually. MEME was developed by Timothy L. Bailey and Charles Elkan, Dept. of Computer Science and Engeneering, University of California, San Diego, USA and is described by Timothy L. Bailey and Charles Elkan [Fitting a mixture model by expectation maximization to discover motifs in biopolymers, Proceedings of the Second International Conference on Intelligent Systems for Molecular Biology, pp. 28-36, AAAI Press, Menlo Park, California, 1994]. The source code for the stand-alone program is public available from the San Diego Supercomputer center (http://meme.sdsc.edu).
For identifying common motifs in all sequences with the software tool MEME, the following settings were used: -maxsize 500000, -nmotifs 15, -evt 0.001, -maxw 60, - distance 1e-3, -minsites number of sequences used for the analysis. Input sequences for MEME were non-aligned sequences in Fasta format. Other parameters were used in the default settings in this software version.
Prosite patterns for conserved domains were generated with the software tool Pratt version 2.1 or manually. Pratt was developed by Inge Jonassen, Dept. of Informatics, University of Bergen, Norway and is described by Jonassen et al. [I.Jonassen, J.F.Collins and D.G.Higgins, Finding flexible patterns in unaligned protein sequences, Protein Science 4 (1995), pp. 1587-1595; I.Jonassen, Efficient discovery of conserved patterns using a pattern graph, Submitted to CABIOS Febr. 1997]. The source code (ANSI C) for the stand-alone program is public available, e.g. at establisched Bioinformatic centers like EBI (European Bioinformatics Institute).
For generating patterns with the software tool Pratt, following settings were used: PL (max Pattern Length): 100, PN (max Nr of Pattern Symbols): 100, PX (max Nr of consecutive x's): 30, FN (max Nr of flexible spacers): 5, FL (max Flexibility): 30, FP (max Flex.Product): 10, ON (max number patterns): 50. Input sequences for Pratt were distinct regions of the protein sequences exhibiting high similarity as identified from software tool MEME. The minimum number of sequences, which have to match the generated patterns (CM, min Nr of Seqs to Match) was set to at least 80% of the provided sequences. Parameters not mentioned here were used in their default settings.
The Prosite patterns of the conserved domains can be used to search for protein sequences matching this pattern. Various establisched Bioinformatic centers provide public internet portals for using those patterns in database searches (e.g. PIR [Protein Information Resource, located at Georgetown University Medical Center] or ExPASy [Expert Protein Analysis System]). Alternatively, stand-alone software is available, like the program Fuzzpro, which is part of the EMBOSS software package. For example, the program Fuzzpro not only allows to search for an exact pattern-protein match but also allows to set various ambiguities in the performed search.

The alignment was performed with the software ClustalW (version 1.83) and is described by Thompson et al. [Thompson, J.D., Higgins, D.G. and Gibson, T.J. (1994) CLUSTAL W: improving the sensitivity of progressive multiple sequence alignment through sequence weighting, positions-specific gap penalties and weight matrix choice. Nucleic Acids Research, 22:4673-4680]. The source code for the stand-alone program is public available from the European Molecular Biology Laboratory; Heidelberg, Germany. The analysis was performed using the default parameters of ClustalW v1.83 (gap open penalty: 10.0; gap extension penalty: 0.2; protein matrix: Gonnet; pprotein/DNA endgap: -1; protein/DNA gapdist: 4).

Degenerated primers can then be utilized by PCR for the amplification of fragments of novel proteins having above-mentioned activity, e.g. conferring the increase of nitrogen or nitrogen containing compounds after increasing its expression or activity or further functional homologs of the polypeptide of the invention or the polypeptide used in the method of the invention from other organisms.

These fragments can then be utilized as hybridization probe for isolating the complete gene sequence. As an alternative, the missing 5' and 3' sequences can be isolated by means of RACE-PCR (rapid amplification of cDNA ends). A nucleic acid molecule according to the invention can be amplified using cDNA or, as an alternative, genomic DNA as template and suitable oligonucleotide primers, following standard PCR amplification techniques. The nucleic acid molecule amplified thus can be cloned into a suitable vector and characterized by means of DNA sequence analysis. Oligonucleotides, which correspond to one of the nucleic acid molecules used in the process, can be generated by standard synthesis methods, for example using an automatic DNA synthesizer.

Nucleic acid molecules which are advantageously for the process according to the invention can be isolated based on their homology to the nucleic acid molecules disclosed herein using the sequences or part thereof as hybridization probe and following standard hybridization techniques under stringent hybridization conditions. In this context, it is possible to use, for example, isolated nucleic acid molecules of at least 15, 20, 25, 30, 35, 40, 50, 60 or more nucleotides, preferably of at least 15, 20 or 25 nucleotides in length which hybridize under stringent conditions with the above-described nucleic acid molecules, in particular with those which encompass a nucleotide sequence of the nucleic acid molecule used in the process of the invention or encoding a protein used in the invention or of the nucleic acid molecule of the invention. Nucleic acid molecules with 30, 50, 100, 250 or more nucleotides may also be used.

The term "homology" means that the respective nucleic acid molecules or encoded proteins are functionally and/or structurally equivalent. The nucleic acid molecules that are homologous to the nucleic acid molecules described above and that are derivatives of said nucleic acid molecules are, for example, variations of said nucleic acid molecules which represent modifications having the same biological function, in particular encoding proteins with the same or substantially the same biological function. They may be naturally occurring variations, such as sequences from other plant varieties or species, or mutations. These mutations may occur naturally or may be obtained by mutagenesis techniques. The allelic variations may be naturally occurring allelic variants as well as synthetically produced or genetically engineered variants. Structurally equivalents can, for example, be identified by testing the binding of said polypeptide to antibodies or computer based predictions. Structurally equivalents have the similar immunological characteristic, e.g. comprise similar epitopes.

By "hybridizing" it is meant that such nucleic acid molecules hybridize under conventional hybridization conditions, preferably under stringent conditions such as described by, e.g., Sambrook (Molecular Cloning; A Laboratory Manual, 2nd Edition, Cold Spring Harbor Laboratory Press, Cold Spring Harbor, NY (1989)) or in Current Protocols in Molecular Biology, John Wiley & Sons, N. Y. (1989), 6.3.1-6.3.6.

According to the invention, DNA as well as RNA molecules of the nucleic acid of the invention can be used as probes. Further, as template for the identification of functional homologues Northern blot assays as well as Southern blot assays can be performed. The Northern blot assay advantageously provides further information about the expressed gene product: e.g. expression pattern, occurrence of processing steps, like splicing and capping, etc. The Southern blot assay provides additional information about the chromosomal localization and organization of the gene encoding the nucleic acid molecule of the invention.

A preferred, nonlimiting example of stringent hydridization conditions are hybridizations in 6 x sodium chloride/sodium citrate (= SSC) at approximately 45°C, followed by one or more wash steps in 0.2 x SSC, 0.1 % SDS at 50 to 65°C, for example at 50°C, 55°C or 60°C. The skilled worker knows that these hybridization conditions differ as a function of the type of the nucleic acid and, for example when organic solvents are present, with regard to the temperature and concentration of the buffer. The temperature under "standard hybridization conditions" differs for example as a function of the type of the nucleic acid between 42°C and 58°C, preferably between 45°C and 50°C in an aqueous buffer with a concentration of 0.1 x 0.5 x, 1 x, 2x, 3x, 4x or 5 x SSC (pH 7.2). If organic solvent(s) is/are present in the abovementioned buffer, for example 50% formamide, the temperature under standard conditions is approximately 40°C, 42°C or 45°C. The hybridization conditions for DNA: DNA hybrids are preferably for example 0.1 x SSC and 20°C, 25°C, 30°C, 35°C, 40°C or 45°C, preferably between 30°C and 45°C. The hybridization conditions for DNA:RNA hybrids are preferably for example 0.1 x SSC and 30°C, 35°C, 40°C, 45°C, 50°C or 55°C, preferably between 45°C and 55°C. The abovementioned hybridization temperatures are determined for example for a nucleic acid approximately 100 bp (= base pairs) in length and a G + C content of 50% in the absence of formamide. The skilled worker knows to determine the hybridization conditions required with the aid of textbooks, for example the ones mentioned above, or from the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford.

A further example of one such stringent hybridization condition is hybridization at 4XSSC at 65°C, followed by a washing in 0.1XSSC at 65°C for one hour. Alternatively, an exemplary stringent hybridization condition is in 50 % formamide, 4XSSC at 42°C. Further, the conditions during the wash step can be selected from the range of conditions delimited by low-stringency conditions (approximately 2X SSC at 50°C) and high-stringency conditions (approximately 0.2X SSC at 50°C, preferably at 65°C) (20X SSC: 0.3M sodium citrate, 3M NaCl, pH 7.0). In addition, the temperature during the wash step can be raised from low-stringency conditions at room temperature, approximately 22°C, to higher-stringency conditions at approximately 65°C. Both of the parameters salt concentration and temperature can be varied simultaneously, or else one of the two parameters can be kept constant while only the other is varied. Denaturants, for example formamide or SDS, may also be employed during the hybridization. In the presence of 50% formamide, hybridization is preferably effected at 42°C. Relevant factors like i) length of treatment, ii) salt conditions, iii) detergent conditions, iv) competitor DNAs, v) temperature and vi) probe selection can be combined case by case so that not all possibilities can be mentioned herein.

Thus, in a preferred embodiment, Northern blots are prehybridized with Rothi-Hybri-Quick buffer (Roth, Karlsruhe) at 68°C for 2h. Hybridization with radioactive labelled probe is done overnight at 68°C. Subsequent washing steps are performed at 68°C with 1xSSC.
For Southern blot assays the membrane is prehybridized with Rothi-Hybri-Quick buffer (Roth, Karlsruhe) at 68°C for 2h. The hybridization with radioactive labelled probe is conducted over night at 68°C. Subsequently the hybridization buffer is discarded and the filter shortly washed using 2xSSC; 0, 1 % SDS. After discarding the washing buffer new 2xSSC; 0,1% SDS buffer is added and incubated at 68°C for 15 minutes. This washing step is performed twice followed by an additional washing step using 1xSSC; 0,1 % SDS at 68°C for 10 min.

Some further examples of conditions for DNA hybridization (Southern blot assays) and wash step are shown herein below:
1. Hybridization conditions can be selected, for example, from the following conditions:
   a) 4X SSC at 65°C,
   b) 6X SSC at 45°C,
   c) 6X SSC, 100 mg/ml denatured fragmented fish sperm DNA at 68°C,
   d) 6X SSC, 0.5% SDS, 100 mg/ml denatured salmon sperm DNA at 68°C,
   e) 6X SSC, 0.5% SDS, 100 mg/ml denatured fragmented salmon sperm DNA, 50% formamide at 42°C,
   f) 50% formamide, 4X SSC at 42°C,
   g) 50% (vol/vol) formamide, 0.1% bovine serum albumin, 0.1% Ficoll, 0.1% polyvinylpyrrolidone, 50 mM sodium phosphate buffer pH 6.5, 750 mM NaCl, 75 mM sodium citrate at 42°C,
   h) 2X or 4X SSC at 50°C (low-stringency condition), or
   i) 30 to 40% formamide, 2X or 4X SSC at 42°C (low-stringency condition).
2. Wash steps can be selected, for example, from the following conditions:
   a) 0.015 M NaCl/0.0015 M sodium citrate/0.1% SDS at 50°C.
   b) 0.1X SSC at 65°C.
   c) 0.1 X SSC, 0.5 % SDS at 68°C.
   d) 0.1X SSC, 0.5% SDS, 50% formamide at 42°C.
   e) 0.2XSSC, 0.1% SDS at 42°C.
   f) 2X SSC at 65°C (low-stringency condition).

Polypeptides having above-mentioned activity, i.e. conferring the increase of nitrogen or nitrogen containing compound, derived from other organisms, can be encoded by other DNA sequences which hybridize to a sequences indicated in Table I, columns 5 or 7, preferably of Table I B, columns 5 or 7, under relaxed hybridization conditions and which code on expression for peptides having the nitrogen or nitrogen containing compounds increasing activity.

Further, some applications have to be performed at low stringency hybridisation conditions, without any consequences for the specificity of the hybridisation. For example, a Southern blot analysis of total DNA could be probed with a nucleic acid molecule of the present invention and washed at low stringency (55°C in 2xSSPE0,1% SDS). The hybridisation analysis could reveal a simple pattern of only genes encoding polypeptides of the present invention or used in the process of the invention, e.g. having herein-mentioned activity of increasing nitrogen or nitrogen containing compounds. A further example of such low-stringent hybridization conditions is 4XSSC at 50°C or hybridization with 30 to 40% formamide at 42°C. Such molecules comprise those which are fragments, analogues or derivatives of the polypeptide of the invention or used in the process of the invention and differ, for example, by way of amino acid and/or nucleotide deletion(s), insertion(s), substitution (s), addition(s) and/or recombination (s) or any other modification(s) known in the art either alone or in combination from the above-described amino acid sequences or their underlying nucleotide sequence(s). However, it is preferred to use high stringency hybridisation conditions.

Hybridization should advantageously be carried out with fragments of at least 5, 10, 15, 20, 25, 30, 35 or 40 bp, advantageously at least 50, 60, 70 or 80 bp, preferably at least 90, 100 or 110 bp. Most preferably are fragments of at least 15, 20, 25 or 30 bp. Preferably are also hybridizations with at least 100 bp or 200, very especially preferably at least 400 bp in length. In an especially preferred embodiment, the hybridization should be carried out with the entire nucleic acid sequence with conditions described above.

The terms "fragment", "fragment of a sequence" or "part of a sequence" mean a truncated sequence of the original sequence referred to. The truncated sequence (nucleic acid or protein sequence) can vary widely in length; the minimum size being a sequence of sufficient size to provide a sequence with at least a comparable function and/or activity of the original sequence referred to or hybridising with the nucleic acid molecule of the invention or the nucleic acid molecule used in the method of the invention or used in the process of the invention under stringent conditions, while the maximum size is not critical. In some applications, the maximum size usually is not substantially greater than that required to provide the desired activity and/or function(s) of the original sequence.
Typically, the truncated amino acid sequence will range from about 5 to about 310 amino acids in length. More typically, however, the sequence will be a maximum of about 250 amino acids in length, preferably a maximum of about 200 or 100 amino acids. It is usually desirable to select sequences of at least about 10, 12 or 15 amino acids, up to a maximum of about 20 or 25 amino acids.

The term "epitope" relates to specific immunoreactive sites within an antigen, also known as antigenic determinates. These epitopes can be a linear array of monomers in a polymeric composition - such as amino acids in a protein - or consist of or comprise a more complex secondary or tertiary structure. Those of skill will recognize that immunogens (i.e., substances capable of eliciting an immune response) are antigens; however, some antigen, such as haptens, are not immunogens but may be made immunogenic by coupling to a carrier molecule. The term "antigen" includes references to a substance to which an antibody can be generated and/or to which the antibody is specifically immunoreactive.

In one embodiment the present invention relates to a epitope of the polypeptide of the present invention or used in the process of the present invention and conferring above mentioned activity, preferably conferring an increase in nitrogen or nitrogen containing compounds.

The term "one or several amino acids" relates to at least one amino acid but not more than that number of amino acids, which would result in a homology of below 50% identity. Preferably, the identity is more than 70% or 80%, more preferred are 85%, 90%, 91%, 92%, 93%, 94% or 95%, even more preferred are 96%, 97%, 98%, or 99% identity.

Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, preferably of Table I B, columns 5 or 7, is one which is sufficiently complementary to one of said nucleotide sequences such that it can hybridize to one of said nucleotide sequences thereby forming a stable duplex. Preferably, the hybridisation is performed under stringent hybridization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, , preferably of Table I B, columns 5 or 7,or a functional portion thereof and preferably has above mentioned activity, in particular has the-nitrogen or nitrogen containing-compound-increasing activity after increasing its activity or an activity of a product of a gene encoding said sequence or its homologs.

The nucleic acid molecule of the invention or the nucleic acid molecule used in the method of the invention comprises a nucleotide sequence which hybridises, preferably hybridises under stringent conditions as defined herein, to one of the nucleotide sequences indicated in Table I, columns 5 or 7, preferably of Table I B, columns 5 or 7, or a portion thereof and encodes a protein having above-mentioned activity and as indicated in indicated in Table II, columns 5 or 7, preferably of Table II B, columns 5 or 7, e.g. conferring an increase of nitrogen or nitrogen containing compounds .

Optionally, the nucleotide sequence, which hybridises to one of the nucleotide sequences indicated in Table I, columns 5 or 7, preferably of Table I B, columns 5 or 7, has further one or more of the activities annotated or known for the a protein as indicated in Table II, column 3,

Moreover, the nucleic acid molecule of the invention or used in the process of the invention can comprise only a portion of the coding region of one of the sequences indicated in Table I, columns 5 or 7, preferably of Table I B, columns 5 or 7, for example a fragment which can be used as a probe or primer or a fragment encoding a biologically active portion of the polypeptide of the present invention or of a polypeptide used in the process of the present invention, i.e. having above-mentioned activity, e.g. conferring an increased content of nitrogen or nitrogen contanining compounds if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences indicated in Table I, columns 5 or 7, an anti-sense sequence of one of the sequences indicated in Table I, columns 5 or 7, or naturally occurring mutants thereof. Primers based on a nucleotide sequence of the invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, will result in a fragment of a polynucleotide sequence as indicated in Table I, columns 5 or 7,. Preferred is Table I B, column 7.

Primer sets are interchangeable. The person skilled in the art knows to combine said primers to result in the desired product, e.g. in a full-length clone or a partial sequence. Probes based on the sequences of the nucleic acid molecule of the invention or used in the process of the present invention can be used to detect transcripts or genomic sequences encoding the same or homologous proteins. The probe can further comprise a label group attached thereto, e.g. the label group can be a radioisotope, a fluorescent compound, an enzyme, or an enzyme co-factor. Such probes can be used as a part of a genomic marker test kit for identifying cells which express an polypeptide of the invention or used in the process of the present invention, such as by measuring a level of an encoding nucleic acid molecule in a sample of cells, e.g., detecting mRNA levels or determining, whether a genomic gene comprising the sequence of the polynucleotide of the invention or used in the processes of the present invention has been mutated or deleted.

The nucleic acid molecule of the invention or the nucleic acid molecule used in the method of the invention encodes a polypeptide or portion thereof which includes an amino acid sequence which is sufficiently homologous to an amino acid sequence as indicated in Table II, columns 5 or 7, lines 1, 2, 3, 4 and/or 5 such that the protein or portion thereof maintains the ability to participate in the accumlation and/or production of nitrogen or nitrogen containing compounds respectively, in particular a protein content increasing activity as mentioned above or as described in the examples in plants or microorganisms is comprised.

As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, columns 5 or 7, such that the protein or portion thereof is able to participate in the the accumulation and/or production of nitrogen or nitrogen containing compounds respectively. In one embodiment, a protein or portion thereof as indicated in Table II, columns 5 or 7, has for example an activity of a polypeptide indicated in Table II, column 3.

In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, columns 5 or 7, and has above-mentioned activity, e.g. conferring preferably the increase of nitrogen or nitrogen containing compounds.

Portions of proteins encoded by the nucleic acid molecule of the invention or the nucleic acid molecule used in the method of the invention are preferably biologically active, preferably having above-mentioned annotated activity, e.g. conferring a increase of nitrogen or nitrogen containing compounds after increase of activity.

As mentioned herein, the term "biologically active portion" is intended to include a portion, e.g., a domain/motif, that confers increase of nitrogen or nitrogen containing compounds or has an immunological activity such that it is binds to an antibody binding specifically to the polypeptide of the present invention or a polypeptide used in the process of the present invention for producing nitrogen or nitrogen containing compounds;

The invention further relates to nucleic acid molecules that differ from one of the nucleotide sequences indicated in Table I, columns 5 or 7, (and portions thereof) due to degeneracy of the genetic code and thus encode a polypeptide of the present invention, in particular a polypeptide having above mentioned activity, e.g. conferring an increase in nitrogen or nitrogen containing compounds in a organism, e.g. as that polypeptides comprising the consensus sequences as indicated in Table IV, column 7, or of the polypeptide as indicated in Table II, columns 5 or 7, or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, column 7, or of the polypeptide as indicated in Table II, columns 5 or 7, or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, column 7, or of a polypeptide as indicated in Table II, columns 5 or 7, or the functional homologues thereof. However, in a preferred embodiment, the nucleic acid molecule of the present invention does not consist of a sequence as indicated in Table I, columns 5 or 7, preferably as indicated in Table I A, columns 5 or 7. Preferably the nucleic acid molecule of the invention is a functional homologue or identical to a nucleic acid molecule indicated in Table I B, columns 5 or 7.

In addition, it will be appreciated by those skilled in the art that DNA sequence polymorphisms that lead to changes in the amino acid sequences may exist within a population. Such genetic polymorphism in the gene encoding the polypeptide of the invention or the polypeptide used in the method of the invention or comprising the nucleic acid molecule of the invention or the nucleic acid molecule used in the method of the invention may exist among individuals within a population due to natural variation.

As used herein, the terms "gene" and "recombinant gene" refer to nucleic acid molecules comprising an open reading frame encoding the polypeptide of the invention or the polypeptide used in the method of the invention or comprising the nucleic acid molecule of the invention or the nucleic acid molecule used in the method of the invention or encoding the polypeptide used in the process of the present invention, preferably from a crop plant or from a microorganism useful for the accumulation and/or production of nitrogen or nitrogen containing compounds respectively, in particular for the production of proteins. Such natural variations can typically result in 1-5% variance in the nucleotide sequence of the gene. Any and all such nucleotide variations and resulting amino acid polymorphisms in genes encoding a polypeptide of the invention or the polypeptide used in the method of the invention or comprising a the nucleic acid molecule of the invention or the nucleic acid molecule used in the method of the invention that are the result of natural variation and that do not alter the functional activity as described are intended to be within the scope of the invention.

Nucleic acid molecules corresponding to natural variants homologues of a nucleic acid molecule of the invention or the nucleic acid molecule used in the method of the invention, which can also be a cDNA, can be isolated based on their homology to the nucleic acid molecules disclosed herein using the nucleic acid molecule of the invention or the nucleic acid molecule used in the method of the invention, or a portion thereof, as a hybridization probe according to standard hybridization techniques under stringent hybridization conditions.

Accordingly, in another embodiment, a nucleic acid molecule of the invention or the nucleic acid molecule used in the method of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, columns 5 or 7,. The nucleic acid molecule is preferably at least 20, 30, 50, 100, 250 or more nucleotides in length.

The term "hybridizes under stringent conditions" is defined above. In one embodiment, the term "hybridizes under stringent conditions" is intended to describe conditions for hybridization and washing under which nucleotide sequences at least 30 %, 40 %, 50 % or 65% identical to each other typically remain hybridized to each other. Preferably, the conditions are such that sequences at least about 70%, more preferably at least about 75% or 80%, and even more preferably at least about 85%, 90% or 95% or more identical to each other typically remain hybridized to each other.

Preferably, the nucleic acid molecule of the invention or the nucleic acid molecule used in the method of the invention that hybridizes under stringent conditions to a sequence as indicated in Table I, columns 5 or 7, lines 1, 2, 3, 4 and/or 5 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring nitrogen or nitrogen containing compounds increase after increasing the expression or activity thereof or the activity of a protein of the invention or used in the process of the invention.

In addition to naturally-occurring variants of the sequences of the polypeptide or nucleic acid molecule of the invention as well as of the polypeptide or nucleic acid molecule used in the process of the invention that may exist in the population, the skilled artisan will further appreciate that changes can be introduced by mutation into a nucleotide sequence of the nucleic acid molecule encoding the polypeptide of the invention or used in the process of the present invention, thereby leading to changes in the amino acid sequence of the encoded said polypeptide, without altering the functional ability of the polypeptide, preferably not decreasing said activity.

For example, nucleotide substitutions leading to amino acid substitutions at "non-essential" amino acid residues can be made in a sequence of the nucleic acid molecule of the invention or used in the process of the invention, e.g. as indicated in Table I, columns 5 or 7, lines 1, 2, 3 4 and/or 5.

A "non-essential" amino acid residue is a residue that can be altered from the wild-type sequence of one without altering the activity of said polypeptide, whereas an "essential" amino acid residue is required for an activity as mentioned above, e.g. leading to an increase in nitrogen or nitrogen containing compounds in an organism after an increase of activity of the polypeptide. Other amino acid residues, however, (e.g., those that are not conserved or only semi-conserved in the domain having said activity) may not be essential for activity and thus are likely to be amenable to alteration without altering said activity.

Further, a person skilled in the art knows that the codon usage between organism can differ. Therefore, he may adapt the codon usage in the nucleic acid molecule of the present invention to the usage of the organism in which the polynucleotide or polypeptide is expressed.

Accordingly, the invention relates to nucleic acid molecules encoding a polypeptide having above-mentioned activity, e.g. conferring an increase in nitrogen or nitrogen containing compounds in an organisms or parts thereof that contain changes in amino acid residues that are not essential for said activity. Such polypeptides differ in amino acid sequence from a sequence contained in a sequence as indicated in Table II, columns 5 or 7, preferably of Table II B, column 7, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, preferably of Table II B, column 7, and is capable of participation in the increased accumulation and/or production of nitrogen or nitrogen containing compounds respectively after increasing its activity, e.g. its expression. Preferably, the protein encoded by the nucleic acid molecule is at least about 60% identical to a sequence as indicated in Table II, columns 5 or 7, lines 1, 2, 3 4 and/or 5, preferably of Table II B, column 7, more preferably at least about 70% identical to one of the sequences as indicated in Table II, columns 5 or 7, preferably of Table II B, column 7, even more preferably at least about 80%, 90%, or 95% homologous to a sequence as indicated in Table II, columns 5 or 7, preferably of Table II B, column 7, and most preferably at least about 96%, 97%, 98%, or 99% identical to the sequence as indicated in Table II, columns 5 or 7, preferably of Table II B, column 7,.

To determine the percentage homology (= identity) of two amino acid sequences or of two nucleic acid molecules, the sequences are written one underneath the other for an optimal comparison (for example gaps may be inserted into the sequence of a protein or of a nucleic acid in order to generate an optimal alignment with the other protein or the other nucleic acid).

The amino acid residues or nucleic acid molecules at the corresponding amino acid positions or nucleotide positions are then compared. If a position in one sequence is occupied by the same amino acid residue or the same nucleic acid molecule as the corresponding position in the other sequence, the molecules are homologous at this position (i.e. amino acid or nucleic acid "homology" as used in the present context corresponds to amino acid or nucleic acid "identity". The percentage homology between the two sequences is a function of the number of identical positions shared by the sequences (i.e. % homology = number of identical positions/total number of positions x 100). The terms "homology" and "identity" are thus to be considered as synonyms.

For the determination of the percentage homology (=identity) of two or more amino acids or of two or more nucleotide sequences several computer software programs have been developed. The homology of two or more sequences can be calculated with for example the software fasta, which presently has been used in the version fasta 3 (W. R. Pearson and D. J. Lipman (1988), Improved Tools for Biological Sequence Comparison. PNAS 85:2444- 2448; W. R. Pearson (1990) Rapid and Sensitive Sequence Comparison with FASTP and FASTA, Methods in Enzymology 183:63 - 98. Another useful program for the calculation of homologies of different sequences is the standard blast program, which is included in the Biomax pedant software (Biomax, Munich, Federal Republic of Germany). This leads unfortunately sometimes to suboptimal results since blast does not always include complete sequences of the subject and the query. Nevertheless as this program is very efficient it can be used for the comparison of a huge number of sequences. The following settings are typically used for such a comparisons of sequences:
-p Program Name [String]; -d Database [String]; default = nr; -i Query File [File In]; default = stdin; -e Expectation value (E) [Real]; default = 10.0; -m alignment view options: 0 = pairwise; 1 = query-anchored showing identities; 2 = query-anchored no identities; 3 = flat query-anchored, show identities; 4 = flat query-anchored, no identities; 5 = query-anchored no identities and blunt ends; 6 = flat query-anchored, no identities and blunt ends; 7 = XML Blast output; 8 = tabular; 9 tabular with comment lines [Integer]; default = 0; -o BLAST report Output File [File Out] Optional; default = stdout; -F Filter query sequence (DUST with blastn, SEG with others) [String]; default = T; -G Cost to open a gap (zero invokes default behavior) [Integer]; default = 0; -E Cost to extend a gap (zero invokes default behavior) [Integer]; default = 0; -X X dropoff value for gapped alignment (in bits) (zero invokes default behavior); blastn 30, megablast 20, tblastx 0, all others 15 [Integer]; default = 0; -I Show GI's in deflines [T/F]; default = F; -q Penalty for a nucleotide mismatch (blastn only) [Integer]; default = -3; -r Reward for a nucleotide match (blastn only) [Integer]; default = 1; -v Number of database sequences to show one-line descriptions for (V) [Integer]; default = 500; -b Number of database sequence to show alignments for (B) [Integer]; default = 250; -f Threshold for extending hits, default if zero; blastp 11, blastn 0, blastx 12, tblastn 13; tblastx 13, megablast 0 [Integer]; default = 0; -g Perfom gapped alignment (not available with tblastx) [T/F]; default = T; -Q Query Genetic code to use [Integer]; default = 1; -D DB Genetic code (for tblast[nx] only) [Integer]; default = 1; -a Number of processors to use [Integer]; default = 1; -O SeqAlign file [File Out] Optional; -J Believe the query defline [T/F]; default = F; -M Matrix [String]; default = BLOSUM62; - W Word size, default if zero (blastn 11, megablast 28, all others 3) [Integer]; default = 0; -z Effective length of the database (use zero for the real size) [Real]; default = 0; -K Number of best hits from a region to keep (off by default, if used a value of 100 is recommended) [Integer]; default = 0; -P 0 for multiple hit, 1 for single hit [Integer]; default = 0; -Y Effective length of the search space (use zero for the real size) [Real]; default = 0; -S Query strands to search against database (for blast[nx], and tblastx); 3 is both, 1 is top, 2 is bottom [Integer]; default = 3; -T Produce HTML output [T/F]; default = F; -I Restrict search of database to list of GI's [String] Optional; -U Use lower case filtering of FASTA sequence [T/F] Optional; default = F; -y X dropoff value for ungapped extensions in bits (0.0 invokes default behavior); blastn 20, megablast 10, all others 7 [Real]; default = 0.0; -Z X dropoff value for final gapped alignment in bits (0.0 invokes default behavior); blastn/megablast 50, tblastx 0, all others 25 [Integer]; default = 0; -R PSI-TBLASTN checkpoint file [File In] Optional; -n MegaBlast search [T/F]; default = F; -L Location on query sequence [String] Optional; -A Multiple Hits window size, default if zero (blastn/megablast 0, all others 40 [Integer]; default = 0; -w Frame shift penalty (OOF algorithm for blastx) [Integer]; default = 0; -t Length of the largest intron allowed in tblastn for linking HSPs (0 disables linking) [Integer]; default = 0.

Results of high quality are reached by using the algorithm of Needleman and Wunsch or Smith and Waterman. Therefore programs based on said algorithms are preferred. Advantageously the comparisons of sequences can be done with the program PileUp (J. Mol. Evolution., 25, 351-360, 1987, Higgins et al., CABIOS, 5 1989: 151-153) or preferably with the programs Gap and BestFit, which are respectively based on the algorithms of Needleman and Wunsch [J. Mol. Biol. 48; 443-453 (1970)] and Smith and Waterman [Adv. Appl. Math. 2; 482-489 (1981)]. Both programs are part of the GCG software-package [Genetics Computer Group, 575 Science Drive, Madison, Wisconsin, USA 53711 (1991); Altschul et al. (1997) Nucleic Acids Res. 25:3389 et seq.]. Therefore preferably the calculations to determine the percentages of sequence homology are done with the program Gap over the whole range of the sequences. The following standard adjustments for the comparison of nucleic acid sequences were used: gap weight: 50, length weight: 3, average match: 10.000, average mismatch: 0.000.

For example a sequence which has a 80% homology with sequence SEQ ID NO: 1 at the nucleic acid level is understood as meaning a sequence which, upon comparison with the sequence SEQ ID NO: 1 by the above Gap program algorithm with the above parameter set, has a 80% homology.

In the state of the art, homology between two polypeptides is also understood as meaning the identity of the amino acid sequence over in each case the entire sequence length which is calculated by comparison with the aid of the program algorithm GAP (Wisconsin Package Version 10.0, University of Wisconsin, Genetics Computer Group (GCG), Madison, USA), setting the following parameters:

| | | | |
|---|---|---|---|
| Gap weight: | 8 | Length weight: | 2 |
| Average match: | 2,912 | Average mismatch: | -2,003 |

For example a sequence which has a 80% homology with sequence SEQ ID NO: 2 at the protein level is understood as meaning a sequence which, upon comparison with the sequence SEQ ID NO: 2 by the above program algorithm with the above parameter set, has a 80% homology.

Functional equivalents derived from one of the polypeptides as indicated in Table II, columns 5 or 7, according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of the polypeptides as indicated in Table II, columns 5 or 7, according to the invention and are distinguished by essentially the same properties as a polypeptide as indicated in Table II, columns 5 or 7,.

Functional equivalents derived from a nucleic acid sequence as indicated in Table I, columns 5 or 7, preferably of Table I B, column 7, lines 1, 2, 3,4 and/or 5 according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of a polypeptide as indicated in Table II, columns 5 or 7, according to the invention and encode polypeptides having essentially the same properties as a polypeptide as indicated in Table II, columns 5 or 7.

"Essentially the same properties" of a functional equivalent is above all understood as meaning that the functional equivalent has above mentioned activity, e.g. conferring an increase in nitrogen or nitrogen containing compounds amount while increasing the amount of protein, activity or function of said functional equivalent in an organism, e.g. a microorganism, a plant or plant or animal tissue, plant or animal cells or a part of the same, for example the plastids

A nucleic acid molecule encoding a homologous to a protein sequence of as indicated in Table II, columns 5 or 7 preferably of Table II B, column 7, can be created by introducing one or more nucleotide substitutions, additions or deletions into a nucleotide sequence of the nucleic acid molecule of the present invention, in particular as indicated in Table I, columns 5 or 7, such that one or more amino acid substitutions, additions or deletions are introduced into the encoded protein. Mutations can be introduced into the encoding sequences for example into sequences as indicated in Table I, columns 5 or 7, by standard techniques, such as site-directed mutagenesis and PCR-mediated mutagenesis.

Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Thus, a predicted nonessential amino acid residue in a polypeptide of the invention or a polypeptide used in the process of the invention is preferably replaced with another amino acid residue from the same family. Alternatively, in another embodiment, mutations can be introduced randomly along all or part of a coding sequence of a nucleic acid molecule of the invention or used in the process of the invention, such as by saturation mutagenesis, and the resultant mutants can be screened for activity described herein to identify mutants that retain or even have increased above mentioned activity, e.g. conferring an increase in content of nitrogen or nitrogen containing compounds.

Following mutagenesis of one of the sequences shown herein, the encoded protein can be expressed recombinantly and the activity of the protein can be determined using, for example, assays described herein (see Examples).

The highest homology of the nucleic acid molecule used in the process according to the invention can be found for generally accessible databases entries by Gap search.
Those databases, which must be mentioned, in particular in this context are general gene databases such as the EMBL database (Stoesser G. et al., Nucleic Acids Res 2001, Vol. 29, 17-21), the GenBank database (Benson D.A. et al., Nucleic Acids Res 2000, Vol. 28,15-18), or the PIR database (Barker W. C. et al., Nucleic Acids Res. 1999, Vol. 27, 39-43). It is furthermore possible to use organism-specific gene databases for determining advantageous sequences, in the case of yeast for example advantageously the SGD database (Cherry J. M. et al., Nucleic Acids Res. 1998, Vol. 26, 73-80) or the MIPS database (Mewes H.W. et al., Nucleic Acids Res. 1999, Vol. 27, 44-48), in the case of E. coli the GenProtEC database (http://web.bham.ac.uk/bcm4ght6/res.html), and in the case of Arabidopsis the TAIR-database (Huala, E. et al., Nucleic Acids Res. 2001 Vol. 29(1), 102-5) or the MIPS database.

Homologues of the nucleic acid sequences used, with a sequence as indicated in Table I, columns 5 or 7, preferably of Table I B, column 7, or of the nucleic acid sequences derived from a sequences as indicated in Table II, columns 5 or 7, lines 1, 2, 3, 4 and/or 5, preferably of Table II B, column 7, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, or from the derived nucleic acid sequences, the intention being, however, that the enzyme activity or the biological activity of the resulting proteins synthesized is advantageously retained or increased.

In one embodiment of the present invention, the nucleic acid molecule of the invention or used in the process of the invention comprises one or more sequences as indicated in Table I, columns 5 or 7preferably of Table I B, column 7. In one embodiment, it is preferred that the nucleic acid molecule comprises as little as possible other nucleotide sequences not shown in any one of sequences as indicated in Table I, columns 5 or 7, preferably of Table I B, column 7. In one embodiment, the nucleic acid molecule comprises less than 500, 400, 300, 200,100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, columns 5 or 7, preferably of Table I B, column 7.

Also preferred is that one or more nucleic acid molecule(s) used in the process of the invention encode a polypeptide comprising a sequence selected from the group as indicated in Table II, columns 5 or 7, preferably of Table II B, column 7, lines 1, 2, 3, 4 and/or 5. In one embodiment, the nucleic acid molecule encodes less than 150,130,100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, preferably of Table II B, column 7.

In one embodiment, the nucleic acid molecule of the invention or used in the process encodes a polypeptide comprising a sequence as indicated in Table II, columns 5 or 7, preferably of Table II B, column 7, comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, columns 5 or 7, preferably of Table II B, column 7.

Polypeptides (= proteins), which still have the essential enzymatic activity of the polypeptide of the present invention conferring an increase of nitrogen or nitrogen containing compounds i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, columns 5 or 7, preferably compared to a sequence as indicated in Table II, column 3 and 5, and expressed under identical conditions.
In one embodiment, the polypeptide of the invention is a homolog consisting of or comprising the sequence as indicated in Table II B, columns 7,.

Homologues of a sequence as indicated in Table I, columns 5 or 7, or of a derived sequence as indicated in Table II, columns 5 or 7, also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

In a further embodiment, the process according to the present invention comprises the following steps:
(a) selecting an organism or a part thereof expressing the polypeptide of this invention in the cytsol and/or in an organelle such as a plastid or mitochondria,;
(b) mutagenizing the selected organism or the part thereof;
(c) comparing the activity or the expression level of said polypeptide in the mutagenized organism or the part thereof with the activity or the expression of said polypeptide in the selected organisms or the part thereof;
(d) selecting the mutagenized organisms or parts thereof, which comprise an increased activity or expression level of said polypeptide compared to the selected organism (a) or the part thereof;
(e) optionally, growing and cultivating the organisms or the parts thereof:

Advantageously the selected organisms are mutagenized according to the invention. According to the invention mutagenesis is any change of the genetic information in the genome of an organism, that means any structural or compositional change in the nucleic acid preferably DNA of an organism that is not caused by normal segregation or genetic recombination processes. Such mutations may occur spontaneously, or may be induced by mutagens as described below. Such change can be induced either randomly or selectively. In both cases the genetic information of the organism is modified. In general this lead to the situation that the activity of the gene product of the relevant genes inside the cells or inside the organism is increased.

In case of the specific or so called site directed mutagenesis a distinct gene is mutated and thereby its activity and/or the activity or the encoded gene product is repressed, reduced or increased, preferably increased. In the event of a random mutagenesis one or more genes are mutated by chance and their activities and/or the activities of their gene products are repressed, reduced or increased, preferably increased.

For the purpose of a mutagenesis of a huge population of organisms, such population can be transformed with a DNA construct, which is useful for the activation of as much as possible genes of an organism, preferably all genes. For example the construct can contain a strong promoter or one or more enhancers, which are capable of transcriptionally activate genes in the vicinity of their integration side. With this method it is possible to statistically mutagenize, e.g. activate nearly all genes of an organism by the random integration of an activation construct. Afterwards the skilled worker can identify those mutagenized lines in which a gene of the invention has been activated, which in turns leads to the desired increase in nitrogen or nitrogen containing compounds.

The genes of the invention can also be activated by mutagenesis, either of regulatory or coding regions. In the event of a random mutagenesis a huge number of organisms are treated with a mutagenic agent. The amount of said agent and the intensity of the treatment will be chosen in such a manner that statistically nearly every gene is mutated once. The process for the random mutagenesis as well as the respective agens is well known by the skilled person. Such methods are disclosed for example by A.M. van Harten [(1998), "Mutation breeding: theory and practical applications", Cambridge University Press, Cambridge, UK], E Friedberg, G Walker, W Siede [(1995), "DNA Repair and Mutagenesis", Blackwell Publishing], or K. Sankaranarayanan, J. M. Gentile, L. R. Ferguson [(2000) "Protocols in Mutagenesis", Elsevier Health Sciences]. As the skilled worker knows the spontaneous mutation rate in the cells of an organism is very low and that a large number of chemical, physical or biological agents are available for the mutagenesis of organisms. These agents are named as mutagens or mutagenic agents. As mentioned before three different kinds of mutagens (chemical, physical or biological agents) are available.

There are different classes of chemical mutagens, which can be separated by their mode of action. For example base analogues such as 5-bromouracil, 2-amino purin. Other chemical mutagens are interacting with the DNA such as sulphuric acid, nitrous acid, hydroxylamine; or other alkylating agents such as monofunctional agents like ethyl methanesulfonate, dimethylsulfate, methyl methanesulfonate), bifunctional like dichloroethyl sulphide, Mitomycin, Nitrosoguanidine - dialkylnitrosamine, N-Nitrosoguanidin derivatives, N-alkyl-N-nitro-N-nitroso-guanidine-), intercalating dyes like Acridine, ethidium bromide).

Physical mutagens are for example ionizing irradiation (X ray), UV irradiation. Different forms of irradiation are available and they are strong mutagens. Two main classes of irradiation can be distinguished: a) non-ionizing irradiation such as UV light or ionizing irradiation such as X ray. Biological mutagens are for example transposable elements for example IS elements such as IS100, transposons such as Tn5, Tn10, Tn916 or Tn1000 or phages like Mu^{amplac}, P1, T5, λplac etc. Methods for introducing this phage DNA into the appropriate microorganism are well known to the skilled worker (see Microbiology, Third Edition, Eds. Davis, B.D., Dulbecco, R., Eisen, H.N. and Ginsberg, H.S., Harper International Edition, 1980). The common procedure of a transposon mutagenesis is the insertion of a transposable element within a gene or nearby for example in the promotor or terminator region and thereby leading to a loss of the gene function. Procedures to localize the transposon within the genome of the organisms are well known by a person skilled in the art.

Preferably a chemical or biochemical procedure is used for the mutagenesis of the organisms. A preferred chemical method is the mutagenesis with N-methyl-N-nitro-nitrosoguanidine.

Other biological method are disclosed by Spee et al. (Nucleic Acids Research, Vol. 21, No. 3, 1993: 777 - 778). Spee et al. teaches a PCR method using dlTP for the random mutagenesis. This method described by Spee et al. was further improved by Rellos et al. (Protein Expr. Purif., 5, 1994 : 270 - 277). The use of an in vitro recombination technique for molecular mutagenesis is described by Stemmer (Proc. Natl. Acad. Sci. USA, Vol. 91, 1994: 10747 - 10751). Moore et al. (Nature Biotechnology Vol. 14, 1996: 458 - 467) describe the combination of the PCR and recombination methods for increasing the enzymatic activity of an esterase toward a para-nitrobenzyl ester. Another route to the mutagenesis of enzymes is described by Greener et al. in Methods in Molecular Biology (Vol. 57, 1996: 375 - 385). Greener et al. use the specific Escherichia coli strain XL1-Red to generate Escherichia coli mutants which have increased antibiotic resistance.

In one embodiment, the protein according to the invention or the nucleic acid molecule characterized herein originates from a eukaryotic or prokaryotic organism such as a non-human animal, a plant, a microorganism such as a fungi, a yeast, an alga, a diatom or a bacterium. Nucleic acid molecules, which advantageously can be used in the process of the invention originate from yeasts, for example the family Saccharomycetaceae, in particular the genus Saccharomyces, or yeast genera such as Candida, Hansenula, Pichia, Yarrowia, Rhodotorula or Schizosaccharomyces and the especially advantageous from the species Saccharomyces cerevisiae.

In one embodiment, nucleic acid molecules, which advantageously can be used in the process of the invention originate from bacteria, for example from Proteobacteria, in particular from Gammaproteobacteria, more preferred from Enterobacteriales, e.g. from the family Enterobacteriaceae, particularly from genera Escherichia, Salmonella, Klebsiella, advantageously form the species Escherichia coli K12.

If, in the process according to the invention, plants are selected as the donor organism, this plant may, in principle, be in any phylogenetic relation of the recipient plant. Donor and recipient plant may belong to the same family, genus, species, variety or line, resulting in an increasing homology between the nucleic acids to be integrated and corresponding parts of the genome of the recipient plant. This also applies analogously to microorganisms as donor and recipient organism.
It might also be advantageously to use nuclei acids molecules from very distinct species, since these might exhibit reduced sensitivity against endogenous regulatory mechanisms and such sequences might not be recognized by endogenous silencing mechanisms.

Accordingly, one embodiment of the application relates to the use of nucleic acid molecules in the process of the invention from plants, e.g. crop plants, e.g. from: B. napus; Glycine max; sunflower, rice, cotton, linseed or maize or their homologues.

Accordingly, in one embodiment, the invention relates to a nucleic acid molecule which comprises a nucleic acid molecule selected from the group consisting of:
**a)** nucleic acid molecule encoding a polypeptide selected from the group as shown in table II; preferably table II B, columns 5 and 7 or a fragment thereof, and which confers enhanced nitrogen assimilation, accumulation and/or utilization in a photosynthetic organism or a part thereof
**b)** nucleic acid molecule comprising of a nucleic acid molecule selected from the group as shown in table I, preferably table I B columns 5 and 7 and which confers enhanced nitrogen assimilation, accumulation and/or utilization in a photosynthetic organism or a part thereof;
**c)** nucleic acid molecule whose sequence can be deduced from a polypeptide sequence encoded by a nucleic acid molecule of (a) or (b) as a result of the degeneracy of the genetic code and and which confers enhanced nitrogen assimilation, accumulation and/or utilization in a photosynthetic organism or a part thereof;
**d)** nucleic acid molecule which encodes a polypeptide which has at least 50% identity with the amino acid sequence of the polypeptide encoded by the nucleic acid molecule of (a) to (c) and and which confers enhanced nitrogen assimilation, accumulation and/or utilization in a photosynthetic organism or a part thereof;
**e)** nucleic acid molecule which hybidizes with a nucleic acid molecule of (a) to (c) under stringent hybridisation conditions and and which confers enhanced nitrogen assimilation, accumulation and/or utilization in a photosynthetic organism or a part thereof;
**f)** nucleic acid molecule which encompasses a nucleic acid molecule which is obtained by amplifying nucleic acid molecules from a cDNA library or a genomic library using the primers or primer pairs as shown in table III, column 7 and which confers enhanced nitrogen assimilation, accumulation and/or utilization in a photosynthetic organism or a part thereof;
**g)** nucleic acid molecule encoding a polypeptide which is isolated with the aid of monoclonal antibodies against a polypeptide encoded by one of the nucleic acid molecules of (a) to (f) and which confers enhanced nitrogen assimilation, accumulation and/or utilization in a photosynthetic organism or a part thereof;
**h)** nucleic acid molecule encoding a polypeptide comprising a consensus as ishown in table IV, column 7 and which confers enhanced nitrogen assimilation, accumulation and/or utilization in a photosynthetic organism or a part thereof; and
**i)** nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and which confers enhanced nitrogen assimilation, accumulation and/or utilization in a photosynthetic organism or a part thereof,
   or which encompasses a sequence which is complementary thereto; whereby, preferably, the nucleic acid molecule according to (a) to (I) distinguishes over the sequence indicated in Table IA or I B, columns 5 or 7, by one or more nucleotides. In one embodiment, the nucleic acid molecule does not consist of the sequence shown and indicated in Table I A or I B, columns 5 or 7,: In one embodiment, the nucleic acid molecule is less than 100%, 99,999%, 99,99%, 99,9% or 99% identical to a sequence indicated in Table I A or I B, columns 5 or 7,. In another embodiment, the nucleic acid molecule does not encode a polypeptide of a sequence indicated in Table II A or II B, columns 5 or 7. In an other embodiment, the nucleic acid molecule of the present invention is at least 30 %, 40 %, 50%, or 60% identical and less than 100%, 99,999%, 99,99%, 99,9% or 99% identical to a sequence indicated in Table I A or I B, columns 5 or 7. In a further embodiment the nucleic acid molecule does not encode a polypeptide sequence as indicated in Table II A or II B, columns 5 or 7. Accordingly, in one embodiment, the nucleic acid molecule of the invention differs at least in one or more residues from a nucleic acid molecule indicated in Table I A or I B, columns 5 or 7. Accordingly, in one embodiment, the nucleic acid molecule of the present invention encodes a polypeptide, which differs at least in one or more amino acids from a polypeptide indicated in Table II A or I B, columns 5 or 7. Accordingly, in one embodiment, the protein encoded by a sequences of a nucleic acid according to (a) to (I) does not consist of a sequence as indicated in Table II A or II B, columns 5 or 7. In a further embodiment, the protein of the present invention is at least 30 %, 40 %, 50%, or 60% identical to a protein sequence indicated in Table II A or II B, columns 5 or 7 and less than 100%, preferably less than 99,999%, 99,99% or 99,9%, more preferably less than 99%, 98%, 97%, 96% or 95% identical to a sequence as indicated in Table II A or II B, columns 5 or 7.

The nucleic acid sequences used in the process are advantageously introduced in a nucleic acid construct, preferably an expression cassette which makes possible the expression of the nucleic acid molecules in an organism, advantageously a plant or a microorganism. The nucleic acid sequences shown in table I A or IB columns 5 or 7 application no. 2 and/or application no. 3 are preferably introduced in a nucleic acid construct, which additionally contains a nucleic acid sequence encoding a plastidial targeting sequence, in such a way that a fusion protein is encoded, which upon translation directs the polypeptide encoded by a nucleic acid sequences shown in table I A or IB columns 5 or 7 into the plastidial compartment. The person skilled in the art is familiar with the design of such nucleic acids constructs. Accordingly, the invention also relates to an nucleic acid construct, preferably to an expression construct, comprising the nucleic acid molecule of the present invention functionally linked to one or more regulatory elements or signals. Accordingly, the invention also relates to a nucleic acid construct, preferably to an expression construct, comprising the nucleic acid molecule of the present invention, preferably a sequence as shown in table I A or IB columns 5 or 7 application no. 2 and/or application no. 3 functionally linked to a plastidal targeting sequence.

As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes are genes of the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

In principle, the nucleic acid construct can comprise the herein described regulator sequences and further sequences relevant for the expression of the comprised genes. Thus, the nucleic acid construct of the invention can be used as expression cassette and thus can be used directly for introduction into the plant, or else they may be introduced into a vector. Accordingly in one embodiment the nucleic acid construct is an expression cassette comprising a microorganism promoter or a microorganism terminator or both. In another embodiment the expression cassette encompasses a plant promoter or a plant terminator or both. In another embodiment the expression cassette encompasses a plant plastidial targeting sequnce.

Accordingly, in one embodiment, the process according to the invention comprises the following steps:
(a) introducing of a nucleic acid construct comprising the nucleic acid molecule of the invention or used in the process of the invention or encoding the polypeptide of the present invention or used in the process of the invention; or
(b) introducing of a nucleic acid molecule, including regulatory sequences or factors, which expression increases the expression of the nucleic acid molecule of the invention or used in the process of the invention or encoding the polypeptide of the present invention or used in the process of the invention;in a cell, or an organism or a part thereof, preferably in a plant, plant cell or a microorganism, and
(c) expressing of the gene product encoded by the nucleic acid construct or the nucleic acid molecule mentioned under (a) or (b) in the cell, in the cytsol or in an organelle such as a plastid or mitochondria or both, preferably in plastids or preferybly in cytosol or the organism.

After the introduction and expression of the nucleic acid construct the transgenic organism or cell is advantageously cultured and subsequently harvested. The transgenic organism or cell may be a prokaryotic or eukaryotic organism such as a microorganism, a plant cell, a plant tissue, preferably a crop plant, or a part thereof.

To introduce a nucleic acid molecule into a nucleic acid construct, e.g. as part of an expression cassette, the codogenic gene segment is advantageously subjected to an amplification and ligation reaction in the manner known by a skilled person. It is preferred to follow a procedure similar to the protocol for the Pfu DNA polymerase or a Pfu/Taq DNA polymerase mixture. The primers are selected according to the sequence to be amplified. The primers should expediently be chosen in such a way that the amplificate comprise the codogenic sequence from the start to the stop codon. After the amplification, the amplificate is expediently analyzed. For example, the analysis may consider quality and quantity and be carried out following separation by gel electrophoresis. Thereafter, the amplificate can be purified following a standard protocol (for example Qiagen). An aliquot of the purified amplificate is then available for the subsequent cloning step. Suitable cloning vectors are generally known to the skilled worker.

They include, in particular, vectors which are capable of replication in easy to handle cloning systems like as bacterial yeast or insect cell based (e.g. baculovirus expression) systems, that is to say especially vectors which ensure efficient cloning in E. coli, and which make possible the stable transformation of plants. Vectors, which must be mentioned in particular are various binary and cointegrated vector systems which are suitable for the T-DNA-mediated transformation. Such vector systems are generally characterized in that they contain at least the vir genes, which are required for the Agrobacterium-mediated transformation, and the T-DNA border sequences.

In general, vector systems preferably also comprise further cis-regulatory regions such as promoters and terminators and/or selection markers by means of which suitably transformed organisms can be identified. While vir genes and T-DNA sequences are located on the same vector in the case of cointegrated vector systems, binary systems are based on at least two vectors, one of which bears vir genes, but no T-DNA, while a second one bears T-DNA, but no vir gene. Owing to this fact, the last-mentioned vectors are relatively small, easy to manipulate and capable of replication in E. coli and in Agrobacterium. These binary vectors include vectors from the series pBIB-HYG, pPZP, pBecks, pGreen. Those which are preferably used in accordance with the invention are Bin19, pBI101, pBinAR, pGPTV and pCAMBIA. An overview of binary vectors and their use is given by Hellens et al, Trends in Plant Science (2000) 5, 446-451.

For a vector preparation, vectors may first be linearized using restriction endonuclease(s) and then be modified enzymatically in a suitable manner. Thereafter, the vector is purified, and an aliquot is employed in the cloning step. In the cloning step, the enzyme-cleaved and, if required, purified amplificate is cloned together with similarly prepared vector fragments, using ligase. In this context, a specific nucleic acid construct, or vector or plasmid construct, may have one or else more codogenic gene segments. The codogenic gene segments in these constructs are preferably linked operably to regulatory sequences. The regulatory sequences include, in particular, plant sequences like the above-described promoters and terminators and eventually a targeting sequence between the codogenic segment and the promotor.. The constructs can advantageously be propagated stably in microorganisms, in particular Escherichia coli and/or Agrobacterium tumefaciens, under selective conditions and enable the transfer of heterologous DNA into plants or other microorganisms. In accordance with a particular embodiment, the constructs are based on binary vectors (overview of a binary vector: Hellens et al., 2000). As a rule, they contain prokaryotic regulatory sequences, such as replication origin and selection markers, for the multiplication in microorganisms such as Escherichia coli and Agrobacterium tumefaciens. Vectors can further contain agrobacterial T-DNA sequences for the transfer of DNA into plant genomes or other eukaryotic regulatory sequences for transfer into other eukaryotic cells, e.g. Saccharomyces sp. or other prokaryotic regulatory sequences for the transfer into other prokaryotic cells, e.g. Corynebacterium sp. or Bacillus sp. For the transformation of plants, the right border sequence, which comprises approximately 25 base pairs, of the total agrobacterial T-DNA sequence is advantageously included. Usually, the plant transformation vector constructs according to the invention contain T-DNA sequences both from the right and from the left border region, which contain expedient recognition sites for site-specific acting enzymes which, in turn, are encoded by some of the vir genes.

Suitable host organisms are known to the skilled worker. Advantageous organisms are described further above in the present application. They include in particular eukaryotes or eubacteria, e.g. prokaryotes or archae bacteria. Advantageously host organisms are microorganisms selected from the group consisting of Actinomycetaceae, Bacillaceae, Brevibacteriaceae, Corynebacteriaceae, Enterobacteriacae, Gordoniaceae, Micrococcaceae, Mycobacteriaceae, Nocardiaceae, Pseudomonaceae, Rhizobiaceae, Streptomycetaceae, Chaetomiaceae, Choanephoraceae, Cryptococcaceae, Cunninghamellaceae, Demetiaceae, Moniliaceae, Mortierellaceae, Mucoraceae, Pythiaceae, Sacharomycetaceae, Saprolegniaceae, Schizosacharomycetaceae, Sodariaceae, Sporobolomycetaceae, Tuberculariaceae, Adelotheciaceae, Dinophyceae, Ditrichaceae and Prasinophyceae. Preferably are unicellular, microorganisms, e.g. fungi, bacteria or protoza, such as fungi like the genus Claviceps or Aspergillus or gram-positive bacteria such as the genera Bacillus, Corynebacterium, Micrococcus, Brevibacterium, Rhodococcus, Nocardia, Caseobacter or Arthrobacter or gram-negative bacteria such as the genera Escherichia, Flavobacterium or Salmonella, or yeasts such as the genera Rhodotorula, Hansenula, Pichia, Yerrowia, Saccharomyces, Schizosaccharomyces or Candida.

Host organisms which are especially advantageously selected in the process according to the invention are microorganisms selected from the group of the genera and species consisting of Hansenula anomala, Candida utilis, Claviceps purpurea, Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp. or Escherichia coli, specifically Escherichia coli K12 and its described strains.

Advantageously preferred in accordance with the invention are host organisms of the genus Agrobacterium tumefaciens or plants. Preferred plants are selected from among the families Aceraceae, Anacardiaceae, Apiaceae, Asteraceae, Apiaceae, Betulaceae, Boraginaceae, Brassicaceae, Bromeliaceae, Cactaceae, Caricaceae, Caryophyllaceae, Cannabaceae, Convolvulaceae, Chenopodiaceae, Elaeagnaceae, Geraniaceae, Gramineae, Juglandaceae, Lauraceae, Leguminosae, Linaceae, Cucurbitaceae, Cyperaceae, Euphorbiaceae, Fabaceae, Malvaceae, Nymphaeaceae, Papaveraceae, Rosaceae, Salicaceae, Solanaceae, Arecaceae, Iridaceae, Liliaceae, Orchidaceae, Gentianaceae, Labiaceae, Magnoliaceae, Ranunculaceae, Carifolaceae, Rubiaceae, Scrophulariaceae, Ericaceae, Polygonaceae, Violaceae, Juncaceae, Poaceae, perennial grass, fodder crops, vegetables and ornamentals.

Especially preferred are plants selected from the groups of the families Apiaceae, Asteraceae, Brassicaceae, Cucurbitaceae, Fabaceae, Papaveraceae, Rosaceae, Solanaceae, Liliaceae or Poaceae. Especially advantageous are, in particular, crop plants. Accordingly, an advantageous plant preferably belongs to the group of the genus peanut, oilseed rape, canola, sunflower, safflower, olive, sesame, hazelnut, almond, avocado, bay, pumpkin/squash, linseed, soya, pistachio, borage, maize, wheat, rye, oats, sorghum and millet, triticale, rice, cotton, barley, cassava, potato, sugarbeet, fodder beet, egg plant, and perennial grasses and forage plants, oil palm, vegetables (brassicas, root vegetables, tuber vegetables, pod vegetables, fruiting vegetables, onion vegetables, leafy vegetables and stem vegetables), buckwheat, Jerusalem artichoke, broad bean, vetches, lentil, alfalfa, dwarf bean, lupin, clover and lucerne.

In order to introduce, into a plant, the nucleic acid molecule of the invention or used in the process according to the invention, it has proved advantageous first to transfer them into an intermediate host, for example a bacterium or a eukaryotic unicellular cell. The transformation into E. coli, which can be carried out in a manner known per se, for example by means of heat shock or electroporation, has proved itself expedient in this context. Thus, the transformed E. coli colonies can be analysed for their cloning efficiency. This can be carried out with the aid of a PCR. Here, not only the identity, but also the integrity, of the plasmid construct can be verified with the aid of a defined colony number by subjecting an aliquot of the colonies to said PCR. As a rule, universal primers which are derived from vector sequences are used for this purpose, it being possible, for example, for a forward primer to be arranged upstream of the start ATG and a reverse primer to be arranged downstream of the stop codon of the codogenic gene segment. The amplificates are separated by electrophoresis and assessed with regard to quantity and quality.

The nucleic acid constructs, which are optionally verified, are subsequently used for the transformation of the plants or other hosts, e.g. other eukaryotic cells or other prokaryotic cells. To this end, it may first be necessary to obtain the constructs from the intermediate host. For example, the constructs may be obtained as plasmids from bacterial hosts by a method similar to conventional plasmid isolation.

The nucleic acid molecule of the invention or used in the process according to the invention can also be introduced into modified viral vectors like baculovirus vectors for expression in insect cells or plant viral vectors like tobacco mosaic virus or potato virus X-based vectors. Approaches leading to the expression of proteins from the modified viral genome including the the nucleic acid molecule of the invention or used in the process according to the invention involve for example the inoculation of tobacco plants with infectious RNA transcribed in vitro from a cDNA copy of the recombinant viral genome. Another approach utilizes the transfection of whole plants from wounds inoculated with Agrobacterium tumefaciens containing cDNA copies of recombinant plus-sense RNA viruses. Different vectors and virus are known to the skilled worker for expression in different target eg. production plants.

A large number of methods for the transformation of plants are known. Since, in accordance with the invention, a stable integration of heterologous DNA into the genome of plants is advantageous, the T-DNA-mediated transformation has proved expedient in particular. For this purpose, it is first necessary to transform suitable vehicles, in particular agrobacteria, with a codogenic gene segment or the corresponding plasmid construct comprising the nucleic acid molecule of the invention.
This can be carried out in a manner known per se. For example, said nucleic acid construct of the invention, or said expression construct or said plasmid construct, which has been generated in accordance with what has been detailed above, can be transformed into competent agrobacteria by means of electroporation or heat shock. In principle, one must differentiate between the formation of cointegrated vectors on the one hand and the transformation with binary vectors on the other hand. In the case of the first alternative, the constructs, which comprise the codogenic gene segment or the nucleic acid molecule of the invention or the nucleic acid molecule used in the method of the invention have no T-DNA sequences, but the formation of the cointegrated vectors or constructs takes place in the agrobacteria by homologous recombination of the construct with T-DNA. The T-DNA is present in the agrobacteria in the form of Ti or Ri plasmids in which exogenous DNA has expediently replaced the oncogenes. If binary vectors are used, they can be transferred to agrobacteria either by bacterial conjugation or by direct transfer. These agrobacteria expediently already comprise the vector bearing the vir genes (currently referred to as helper Ti(Ri) plasmid).

One or more markers may expediently also be used together with the nucleic acid construct, or the vector of the invention and, if plants or plant cells shall be transformed together with the T-DNA, with the aid of which the isolation or selection of transformed organisms, such as agrobacteria or transformed plant cells, is possible. These marker genes enable the identification of a successful transfer of the nucleic acid molecules according to the invention via a series of different principles, for example via visual identification with the aid of fluorescence, luminescence or in the wavelength range of light which is discernible for the human eye, by a resistance to herbicides or antibiotics, via what are known as nutritive markers (auxotrophism markers) or antinutritive markers, via enzyme assays or via phytohormones. Examples of such markers which may be mentioned are GFP (= green fluorescent protein); the luciferin/luceferase system, the -galactosidase with its colored substrates, for example X-Gal, the herbicide resistances to, for example, imidazolinone, glyphosate, phosphinothricin or sulfonylurea, the antibiotic resistances to, for example, bleomycin, hygromycin, streptomycin, kanamycin, tetracyclin, chloramphenicol, ampicillin, gentamycin, geneticin (G418), spectinomycin or blasticidin, to mention only a few, nutritive markers such as the utilization of mannose or xylose, or antinutritive markers such as the resistance to 2-deoxyglucose or D-aminoacids. This list is a small number of possible markers. The skilled worker is very familiar with such markers. Different markers are preferred, depending on the organism and the selection method.

As a rule, it is desired that the plant nucleic acid constructs are flanked by T-DNA at one or both sides of the codogenic gene segment. This is particularly useful when bacteria of the species Agrobacterium tumefaciens or Agrobacterium rhizogenes are used for the transformation. A method, which is preferred in accordance with the invention, is the transformation with the aid of Agrobacterium tumefaciens. However, biolistic methods may also be used advantageously for introducing the sequences in the process according to the invention, and the introduction by means of PEG is also possible. The transformed agrobacteria can be grown in the manner known per se and are thus available for the expedient transformation of the plants. The plants or plant parts to be transformed are grown or provided in the customary manner. The transformed agrobacteria are subsequently allowed to act on the plants or plant parts until a sufficient transformation rate is reached. Allowing the agrobacteria to act on the plants or plant parts can take different forms. For example, a culture of morphogenic plant cells or tissue may be used. After the T-DNA transfer, the bacteria are, as a rule, eliminated by antibiotics, and the regeneration of plant tissue is induced. This is done in particular using suitable plant hormones in order to initially induce callus formation and then to promote shoot development.

The transfer of foreign genes into the genome of a plant is called transformation. In doing this the methods described for the transformation and regeneration of plants from plant tissues or plant cells are utilized for transient or stable transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least the flower primordia. The plant is subsequently grown on until the seeds of the treated plant are obtained (Clough and Bent, Plant J. (1998) 16, 735-743). To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Further advantageous transformation methods, in particular for plants, are known to the skilled worker and are described hereinbelow.

Further advantageous and suitable methods are protoplast transformation by poly(ethylene glycol)-induced DNA uptake, the "biolistic" method using the gene cannon - referred to as the particle bombardment method, electroporation, the incubation of dry embryos in DNA solution, microinjection and gene transfer mediated by Agrobacterium. Said methods are described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming Agrobacterium tumefaciens, for example pBin19 (Bevan et al., Nucl. Acids Res. 12 (1984) 8711). Agrobacteria transformed by such a vector can then be used in known manner for the transformation of plants, in particular of crop plants such as by way of example tobacco plants, for example by bathing bruised leaves or chopped leaves in an agrobacterial solution and then culturing them in suitable media. The transformation of plants by means of Agrobacterium tumefaciens is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.

In addition to a sequence indicated in Table I, columns 5 or 7 or its derivatives, it is advantageous additionally to express and/or mutate further genes in the organisms. Especially advantageously, additionally at least one further gene of the amino acid biosynthetic pathway such as for L-lysine, L-threonine and/or L-methionine is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine a sequence as indicated in Table I, columns 5 or 7, with genes which generally support or enhances to growth or yield of the target organismen, for example genes which lead to faster growth rate of microorganisms or genes which produces stress-, pathogen, or herbicide resistant plants.

In a further advantageous embodiment of the process of the invention, the organisms used in the process are those in which simultaneously at least one of the aforementioned genes or one of the aforementioned nucleic acids is mutated so that the activity of the corresponding proteins is influenced by metabolites to a smaller extent compared with the unmutated proteins, or not at all, and that in particular the accumlation or production according to the invention of nitrogen or nitrogen containing compounds respectively is not impaired, or so that their specific enzymatic activity is increased. Less influence means in this connection that the regulation of the enzymic activity is less by at least 10%, advantageously at least 20, 30 or 40%, particularly advantageously by at least 50, 60, 70, 80 or 90%, compared with the starting organism, and thus the activity of the enzyme is increased by these figures mentioned compared with the starting organism. An increase in the enzymatic activity means an enzymatic activity which is increased by at least 10%, advantageously at least 20, 30, 40 or 50%, particularly advantageously by at least 60, 70, 80, 90, 100, 200, 300, 500 or 1000%, compared with the starting organism. This leads to an enhanced nitrogen assimilation, accumulation and/or utilization the organism.

In a further advantageous embodiment of the process of the invention, the organisms used in the process are those in which simultaneously a N-containing compound degrading protein is attenuated, in particular by reducing the rate of expression of the corresponding gene.

In another embodiment of the process of the invention, the organisms used in the process are those in which simultaneously at least one of the aforementioned nucleic acids or of the aforementioned genes is mutated in such a way that the enzymatic or biological activity of the corresponding protein is partially reduced or completely blocked. A reduction in the enzymatic or biological activity means an enzymatic activity, which is reduced by at least 10%, advantageously at least 20, 30 or 40%, particularly advantageously by at least 50, 60 or 70%, preferably more, compared with the starting organism.

If it is intended to transform the host cell, in particular the plant cell, with several constructs or vectors, the marker of a preceding transformation must be removed or a further marker employed in a following transformation. The markers can be removed from the host cell, in particular the plant cell, as described hereinbelow via methods with which the skilled worker is familiar. In particular plants without a marker, in particular without resistance to antibiotics, are an especially preferred embodiment of the present invention.

In the process according to the invention, the nucleic acid sequences used in the process according to the invention are advantageously linked operably to one or more regulatory signals in order to increase gene expression. These regulatory sequences are intended to enable the specific expression of the genes and the expression of protein. Depending on the host organism for example plant or microorganism, this may mean, for example, that the gene is expressed and/or overexpressed after induction only, or that it is expressed and/or overexpressed constitutively. These regulatory sequences are, for example, sequences to which the inductors or repressors bind and which thus regulate the expression of the nucleic acid. In addition to these novel regulatory sequences, or instead of these sequences, the natural regulation of these sequences may still be present before the actual structural genes and, if appropriate, may have been genetically modified so that the natural regulation has been switched off and gene expression has been increased. However, the nucleic acid construct of the invention suitable as expression cassette (= expression construct = gene construct) can also be simpler in construction, that is to say no additional regulatory signals have been inserted before the nucleic acid sequence or its derivatives, and the natural promoter together with its regulation has not been removed. Instead, the natural regulatory sequence has been mutated in such a way that regulation no longer takes place and/or gene expression is increased. These modified promoters can also be introduced on their own before the natural gene in the form of part sequences (= promoter with parts of the nucleic acid sequences according to the invention) in order to increase the activity. Moreover, the gene construct can advantageously also comprise one or more of what are known as enhancer sequences in operable linkage with the promoter, and these enable an increased expression of the nucleic acid sequence. Also, it is possible to insert additional advantageous sequences at the 3' end of the DNA sequences, such as, for example, further regulatory elements or terminators.

The nucleic acid molecules, which encode proteins according to the invention and nucleic acid molecules, which encode other polypeptides may be present in one nucleic acid construct or vector or in several ones. Advantageously, only one copy of the nucleic acid molecule of the invention or the nucleic acid molecule used in the method of the invention or its encoding genes is present in the nucleic acid construct or vector. Several vectors or nucleic acid construct or vector can be expressed together in the host organism. The nucleic acid molecule or the nucleic acid construct or vectoraccording to the invention can be inserted in a vector and be present in the cell in a free form. If a stable transformation is preferred, a vector is used, which is stably duplicated over several generations or which is else be inserted into the genome. In the case of plants, integration into the plastid genome or, in particular, into the nuclear genome may have taken place. For the insertion of more than one gene in the host genome the genes to be expressed are present together in one gene construct, for example in above-described vectors bearing a plurality of genes.

As a rule, regulatory sequences for the expression rate of a gene are located upstream (5'), within, and/or downstream (3') relative to to the coding sequence of the nucleic acid molecule of the invention or the nucleic acid molecule used in the method of the invention or another codogenic gene segment. They control in particular transcription and/or translation and/or the transcript stability. The expression level is dependent on the conjunction of further cellular regulatory systems, such as the protein biosynthesis and degradation systems of the cell.

Regulatory sequences include transcription and translation regulating sequences or signals, e.g. sequences located upstream (5'), which concern in particular the regulation of transcription or translation initiation, such as promoters or start codons, and sequences located downstream (3'), which concern in particular the regulation of transcription or translation termination and transcript stability, such as polyadenylation signals or stop codons. Regulatory sequences can also be present in transcribed coding regions as well in transcribed non-coding regions, e.g. in introns, as for example splicing sites. Promoters for the regulation of expression of the nucleic acid molecule according to the invention in a cell and which can be employed are, in principle, all those which are capable of stimulating the transcription of genes in the organisms in question, such as microorganisms or plants. Suitable promoters, which are functional in these organisms are generally known. They may take the form of constitutive or inducible promoters. Suitable promoters can enable the development-and/or tissue-specific expression in multi-celled eukaryotes; thus, leaf-, root-, flower-, seed-, stomata-, tuber- or fruit-specific promoters may advantageously be used in plants.

The regulatory sequences or factors can, as described above, have a positive effect on, the expression of the genes introduced, thus increasing their expression. Thus, an enhancement of the expression can advantageously take place at the transcriptional level by using strong transcription signals such as strong promoters and/or strong enhancers. In addition, enhancement of expression on the translational level is also possible, for example by introducing translation enhancer sequences, e.g., the enhancer e.g. improving the ribosomal binding to the transcript, or by increasing the stability of the mRNA, e.g. by replacing the 3'UTR coding region by a region encoding a 3'UTR known as conferring an high stability of the transcript or by stabilization of the transcript through the elimination of transcript instability, so that the mRNA molecule is translated more often than the wild type. For example in plants AU-rich elements (AREs) and DST (downstream) elements destabilized transcripts. Mutagenesis studies have demonstrated that residues within two of the conserved domains, the ATAGAT and the GTA regions, are necessary for instability function. Therefore removal or mutation of such elements would obviously lead to more stable transcripts, higher transcript rates and higher protein activity. Translation enhancers are also the "overdrive sequence", which comprises the tobacco mosaic virus 5'-untranslated leader sequence and which increases the protein/RNA ratio (Gallie et al., 1987, Nucl. Acids Research 15:8693-8711)

Enhancers are generally defined as cis active elements, which can stimulate gene transcription independent of position and orientation. Different enhancers have been identified in plants, which can either stimulate transcription constitutively or tissue or stimuli specific. Well known examples for constitutive enhancers are the enhancer from the 35S promoter (Odell et al., 1985, Nature 313:810-812) or the ocs enhancer (Fromm et al., 1989, Plant Cell 1: 977-984) Another examples are the G-Box motif tetramer which confers high-level constitutive expression in dicot and monocot plants (Ishige et al., 1999, Plant Journal, 18, 443-448) or the petE, a A/T-rich sequence which act as quantitative enhancers of gene expression in transgenic tobacco and potato plants (Sandhu et al., 1998; Plant Mol Biol. 37(5):885-96). Beside that, a large variety of cis-active elements have been described which contribute to specific expression pattern, like organ specific expression or induced expression in response to biotic or abiotic stress. Examples are elements which provide pathogen or wound-induced expression (Rushton, 2002, Plant Cell, 14, 749-762) or guard cell-specific expression (Plesch, 2001, Plant Journal 28, 455-464).

Advantageous regulatory sequences for the expression of the nucleic acid molecule according to the invention in microorganisms are present for example in promoters such as the cos, tac, rha, trp, tet, trp-tet, lpp, lac, lpp-lac, lacl^{q-,} T7, T5, T3, gal, trc, ara, SP6, λ-P_{R} or λ-P_{L} promoter, which are advantageously used in Gram-negative bacteria. Further advantageous regulatory sequences are present for example in the Gram-positive promoters amy, dnaK, xylS and SPO2, in the yeast or fungal promoters ADC1, MFα, AC, P-60, UASH, MCB, PHO, CYC1, GAPDH, TEF, rp28, ADH. Promoters, which are particularly advantageous, are constitutive, tissue or compartment specific and inducible promoters. In general, "promoter" is understood as meaning, in the present context, a regulatory sequence in a nucleic acid molecule, which mediates the expression of a coding sequence segment of a nucleic acid molecule. In general, the promoter is located upstream to the coding sequence segment. Some elements, for example expression-enhancing elements such as enhancer may, however, also be located downstream or even in the transcribed region.

In principle, it is possible to use natural promoters together with their regulatory sequences, such as those mentioned above, for the novel process. It is also possible advantageously to use synthetic promoters, either additionally or alone, in particular when they mediate seed-specific expression such as described in, for example, WO 99/16890.

The expression of the nucleic acid molecules used in the process may be desired alone or in combination with other genes or nucleic acids. Multiple nucleic acid molecules conferring the expression of advantageous genes can be introduced via the simultaneous transformation of several individual suitable nucleic acid constructs, i.e. expression constructs, or, preferably, by combining several expression cassettes on one construct. It is also possible to transform several vectors with in each case several expression cassettes stepwise into the recipient organisms.

As described above the transcription of the genes introduced should advantageously be terminated by suitable terminators at the 3' end of the biosynthesis genes introduced (behind the stop codon). A terminator, which may be used for this purpose is, for example, the OCS1 terminator, the nos3 terminator or the 35S terminator. As is the case with the promoters, different terminator sequences should be used for each gene. Terminators, which are useful in microorganism are for example the fimA terminator, txn terminator or trp terminator. Such terminators can be rho-dependent or rho-independent.

Different plant promoters such as, for example, the USP, the LegB4-, the DC3 promoter or the ubiquitin promoter from parsley or other herein mentioned promoter and different terminators may advantageously be used in the nucleic acid construct.

In order to ensure the stable integration, into the transgenic plant, of nucleic acid molecules used in the process according to the invention in combination with further biosynthesis genes over a plurality of generations, each of the coding regions used in the process should be expressed under the control of its own, preferably unique, promoter since repeating sequence motifs may lead to recombination events or to silencing or, in plants, to instability of the T-DNA.

The nucleic acid construct is advantageously constructed in such a way that a promoter is followed by a suitable cleavage site for insertion of the nucleic acid to be expressed, advantageously in a polylinker, followed, if appropriate, by a terminator located behind the polylinker. If appropriate, this order is repeated several times so that several genes are combined in one construct and thus can be introduced into the transgenic plant in order to be expressed. The sequence is advantageously repeated up to three times. For the expression, the nucleic acid sequences are inserted via the suitable cleavage site, for example in the polylinker behind the promoter. It is advantageous for each nucleic acid sequence to have its own promoter and, if appropriate, its own terminator, as mentioned above. However, it is also possible to insert several nucleic acid sequences behind a promoter and, if appropriate, before a terminator if a polycistronic transcription is possible in the host or target cells. In this context, the insertion site, or the sequence of the nucleic acid molecules inserted, in the nucleic acid construct is not decisive, that is to say a nucleic acid molecule can be inserted in the first or last position in the cassette without this having a substantial effect on the expression. However, it is also possible to use only one promoter type in the construct. However, this may lead to undesired recombination events or silencing effects, as said.

Accordingly, in a preferred embodiment, the nucleic acid construct according to the invention confers expression of the nucleic acid molecule of the invention or the nucleic acid molecule used in the method of the invention, and, optionally further genes, in a plant and comprises one or more plant regulatory elements. Said nucleic acid construct according to the invention advantageously encompasses a plant promoter or a plant terminator or a plant promoter and a plant terminator.

A "plant" promoter comprises regulatory elements, which mediate the expression of a coding sequence segment in plant cells. Accordingly, a plant promoter need not be of plant origin, but may originate from viruses or microorganisms, in particular for example from viruses which attack plant cells.

The plant promoter can also originates from a plant cell, e.g. from the plant, which is transformed with the nucleic acid construct or vector as described herein.
This also applies to other "plant" regulatory signals, for example in "plant" terminators.

A nucleic acid construct suitable for plant expression preferably comprises regulatory elements which are capable of controlling the expression of genes in plant cells and which are operably linked so that each sequence can fulfill its function. Accordingly, the nucleic acid construct can also comprise transcription terminators. Examples for transcriptional termination are polyadenylation signals. Preferred polyadenylation signals are those which originate from Agrobacterium tumefaciens T-DNA, such as the gene 3 of the Ti plasmid pTiACH5, which is known as octopine synthase (Gielen et al., EMBO J. 3 (1984) 835 et seq.) or functional equivalents thereof, but all the other terminators which are functionally active in plants are also suitable.

The nucleic acid construct suitable for plant expression preferably also comprises other operably linked regulatory elements such as translation enhancers, for example the overdrive sequence, which comprises the tobacco mosaic virus 5'-untranslated leader sequence, which increases the protein/RNA ratio (Gallie et al., 1987, Nucl. Acids Research 15:8693-8711).

Other preferred sequences for use in operable linkage in gene expression constructs are targeting sequences, which are required for targeting the gene product into specific cell compartments (for a review, see Kermode, Crit. Rev. Plant Sci. 15, 4 (1996) 285-423 and references cited therein), for example into the vacuole, the nucleus, all types of plastids, such as amyloplasts, chloroplasts, chromoplasts, the extracellular space, the mitochondria, the endoplasmic reticulum, elaioplasts, peroxisomes, glycosomes, and other compartments of cells or extracellular. Sequences, which must be mentioned in this context are, in particular, the signal-peptide- or transit-peptide-encoding sequences which are known per se. For example, plastid-transit-peptide-encoding sequences enable the targeting of the expression product into the plastids of a plant cell.Targeting sequences are also known for eukaryotic and to a lower extent for prokaryotic organisms and can advantageously be operable linked with the nucleic acid molecule of the present invention to achieve an expression in one of said compartments or extracellular. Especially preferred is the operable linkage of the nucleic acid sequences shown in table I A or IB columns 5 or 7 application no. 2 or no. 3 with a targeting sequence for the plastidial compartment. For expression in plants, the nucleic acid molecule must, as described above, be linked operably to or comprise a suitable promoter which expresses the gene at the right point in time and in a cell- or tissue-specific manner. Usable promoters are constitutive promoters (Benfey et al., EMBO J. 8 (1989) 2195-2202), such as those which originate from plant viruses, such as 35S CAMV (Franck et al., Cell 21 (1980) 285-294), 19S CaMV (see also US 5352605 and WO 84/02913), 34S FMV (Sanger et al., Plant. Mol. Biol., 14, 1990: 433-443), the parsley ubiquitin promoter, or plant promoters such as the Rubisco small subunit promoter described in US 4,962,028 or the plant promoters PRP1 [Ward et al., Plant. Mol. Biol. 22 (1993)], SSU, PGEL1, OCS [Leisner (1988) Proc Natl Acad Sci USA 85(5):2553-2557], lib4, usp, mas [Comai (1990) Plant Mol Biol 15 (3):373-381], STLS1, ScBV (Schenk (1999) Plant Mol Biol 39(6):1221-1230), B33, SAD1 or SAD2 (flax promoters, Jain et al., Crop Science, 39 (6), 1999: 1696-1701) or nos [Shaw et al. (1984) Nucleic Acids Res. 12(20):7831-7846]. Stable, constitutive expression of the proteins according to the invention a plant can be advantageous. However, inducible expression of the polypeptide of the invention or the polypeptide used in the method of the invention is advantageous, if a late expression before the harvest is of advantage, as metabolic manipulation may lead to a plant growth retardation.

The expression of plant genes can also be facilitated as described above via a chemical inducible promoter (for a review, see Gatz 1997, Annu. Rev. Plant Physiol. Plant Mol. Biol., 48:89-108). Chemically inducible promoters are particularly suitable when it is desired to express the gene in a time-specific manner. Examples of such promoters are a salicylic acid inducible promoter (WO 95/19443), and abscisic acid-inducible promoter (EP 335 528), a tetracyclin-inducible promoter (Gatz et al. (1992) Plant J. 2, 397-404), a cyclohexanol- or ethanol-inducible promoter (WO 93/21334) or others as described herein.

Other suitable promoters are those which react to biotic or abiotic stress conditions, for example the pathogen-induced PRP1 gene promoter (Ward et al., Plant. Mol. Biol. 22 (1993) 361-366), the tomato heat-inducible hsp80 promoter (US 5,187,267), the potato chill-inducible alpha-amylase promoter (WO 96/12814) or the wound-inducible pinII promoter (EP-A-0 375 091) or others as described herein.

Preferred promoters are in particular those which bring about gene expression in tissues and organs in which the biosynthesis of nitrogen or nitrogen containing compounds like amino acids or proteins takes place, in seed cells, such as endosperm cells and cells of the developing embryo. Suitable promoters are the oilseed rape napin gene promoter (US 5,608,152), the Vicia faba USP promoter (Baeumlein et al., Mol Gen Genet, 1991, 225 (3):459-67), the Arabidopsis oleosin promoter (WO 98/45461), the Phaseolus vulgaris phaseolin promoter (US 5,504,200), the Brassica Bce4 promoter (WO 91/13980), the bean arc5 promoter, the carrot DcG3 promoter, or the Legumin B4 promoter (LeB4; Baeumlein et al., 1992, Plant Journal, 2 (2):233-9), and promoters which bring about the seed-specific expression in monocotyledonous plants such as maize, barley, wheat, rye, rice and the like. Advantageous seed-specific promoters are the sucrose binding protein promoter (WO 00/26388), the phaseolin promoter and the napin promoter. Suitable promoters which must be considered are the barley lpt2 or lpt1 gene promoter (WO 95/15389 and WO 95/23230), and the promoters described in WO 99/16890 (promoters from the barley hordein gene, the rice glutelin gene, the rice oryzin gene, the rice prolamin gene, the wheat gliadin gene, the wheat glutelin gene, the maize zein gene, the oat glutelin gene, the sorghum kasirin gene and the rye secalin gene). Further suitable promoters are Amy32b, Amy 6-6 and Aleurain [US 5,677,474], Bce4 (oilseed rape) [US 5,530,149], glycinin (soya) [EP 571 741], phosphoenolpyruvate carboxylase (soya) [JP 06/62870], ADR12-2 (soya) [WO 98/08962], isocitrate lyase (oilseed rape) [US 5,689,040] or α-amylase (barley) [EP 781 849]. Other promoters which are available for the expression of genes in plants are leaf-specific promoters such as those described in DE-A 19644478 or light-regulated promoters such as, for example, the pea petE promoter.

Further suitable plant promoters are the cytosolic FBPase promoter or the potato ST-LSI promoter (Stockhaus et al., EMBO J. 8, 1989, 2445), the Glycine max phosphoribosylpyrophosphate amidotransferase promoter (GenBank Accession No. U87999) or the node-specific promoter described in EP-A-0 249 676.

Other promoters, which are particularly suitable, are those which bring about plastid-specific expression. Suitable promoters such as the viral RNA polymerase promoter are described in WO 95/16783 and WO 97/06250, and the Arabidopsis clpP promoter, which is described in WO 99/46394.

Other promoters, which are used for the strong expression of heterologous sequences in as many tissues as possible, in particular also in leaves, are, in addition to several of the abovementioned viral and bacterial promoters, preferably, plant promoters of actin or ubiquitin genes such as, for example, the rice actin1 promoter. Further examples of constitutive plant promoters are the sugarbeet V-ATPase promoters (WO 01/14572). Examples of synthetic constitutive promoters are the Super promoter (WO 95/14098) and promoters derived from G-boxes (WO 94/12015). If appropriate, chemical inducible promoters may furthermore also be used, compare EP-A 388186, EP-A 335528, WO 97/06268.

As already mentioned herein, further regulatory sequences, which may be expedient, if appropriate, also include sequences, which target the transport and/or the localization of the expression products. Sequences, which must be mentioned in this context are, in particular, the signal-peptide- or transit-peptide-encoding sequences which are known per se. For example, plastid-transit-peptide-encoding sequences enable the targeting of the expression product into the plastids of a plant cell.

Preferred recipient plants are, as described above, in particular those plants, which can be transformed in a suitable manner. These include monocotyledonous and dicotyledonous plants. Plants which must be mentioned in particular are agriculturally useful plants such as cereals and grasses, for example Triticum spp., Zea mays, Hordeum vulgare, oats, Secale cereale, Oryza sativa, Pennisetum glaucum, Sorghum bicolor, Triticale, Agrostis spp., Cenchrus ciliaris, Dactylis glomerata, Festuca arundinacea, Lolium spp., Medicago spp. and Saccharum spp., legumes and oil crops, for example Brassica juncea, Brassica napus, Glycine max, Arachis hypogaea, Gossypium hirsutum, Cicer arietinum, Helianthus annuus, Lens culinaris, Linum usitatissimum, Sinapis alba, Trifolium repens and Vicia narbonensis, vegetables and fruits, for example bananas, grapes, Lycopersicon esculentum, asparagus, cabbage, watermelons, kiwi fruit, Solanum tuberosum, Beta vulgaris, cassava and chicory, trees, for example Coffea species, Citrus spp., Eucalyptus spp., Picea spp., Pinus spp. and Populus spp., medicinal plants and trees, and flowers.

One embodiment of the present invention also relates to a method for generating a vector, which comprises the insertion, into a vector, of the nucleic acid molecule characterized herein, the nucleic acid molecule according to the invention or the expression cassette according to the invention. The vector can, for example, be introduced in to a cell, e.g. a microorganism or a plant cell, as described herein for the nucleic acid construct, or below under transformation or transfection or shown in the examples. A transient or stable transformation of the host or target cell is possible, however, a stable transformation is preferred. The vector according to the invention is preferably a vector, which is suitable for expressing the polypeptide according to the invention in a plant. The method can thus also encompass one or more steps for integrating regulatory signals into the vector, in particular signals, which mediate the expression in microorganisms or plants.

Accordingly, the present invention also relates to a vector comprising the nucleic acid molecule characterized herein as part of a nucleic acid construct suitable for plant expression or the nucleic acid molecule according to the invention.

The advantageous vectors of the inventioncomprise the nucleic acid molecules which encode proteins according to the invention, nucleic acid molecules which are used in the process, or nucleic acid construct suitable for plant expression comprising the nucleic acid molecules used, either alone or in combination with further genes such as the biosynthesis or regulatory genes of amino acidmetabolism e.g. with the genes mentioned herein above. In accordance with the invention, the term "vector" refers to a nucleic acid molecule, which is capable of transporting another nucleic acid to which it is linked. One type of vector is a "plasmid", which means a circular double-stranded DNA loop into which additional DNA segments can be ligated. A further type of vector is a viral vector, it being possible to ligate additional nucleic acids segments into the viral genome. Certain vectors are capable of autonomous replication in a host cell into which they have been introduced (for example bacterial vectors with bacterial replication origin). Other preferred vectors are advantageously completely or partly integrated into the genome of a host cell when they are introduced into the host cell and thus replicate together with the host genome. Moreover, certain vectors are capable of controlling the expression of genes with which they are in operable linkage. In the present context, these vectors are referred to as "expression vectors". As mentioned above, they are capable of autonomous replication or may be integrated partly or completely into the host genome. Expression vectors, which are suitable for DNA recombination techniques usually take the form of plasmids. In the present description, "plasmid" and "vector" can be used interchangeably since the plasmid is the most frequently used form of a vector. However, the invention is also intended to encompass these other forms of expression vectors, such as viral vectors, which exert similar functions. The term vector is furthermore also to encompass other vectors which are known to the skilled worker, such as phages, viruses such as SV40, CMV, TMV, transposons, IS elements, phasmids, phagemids, cosmids, and linear or circular DNA.

The recombinant expression vectors which are advantageously used in the process comprise the nucleic acid molecules according to the invention or the nucleic acid construct according to the invention in a form which is suitable for expressing, in a host cell, the nucleic acid molecules according to the invention or described herein. Accordingly, the the recombinant expression vectors comprise one or more regulatory signals selected on the basis of the host cells to be used for the expression, in operable linkage with the nucleic acid sequence to be expressed.

In a recombinant expression vector, "operable linkage" means that the nucleic acid molecule of interest is linked to the regulatory signals in such a way that expression of the nucleic acid molecule is possible: they are linked to one another in such a way that the two sequences fulfill the predicted function assigned to the sequence (for example in an in-vitro transcription/translation system, or in a host cell if the vector is introduced into the host cell).

The term "regulatory sequence" is intended to comprise promoters, enhancers and other expression control elements (for example polyadenylation signals These regulatory sequences are described, for example, in Goeddel: Gene Expression Technology: Methods in Enzymology 185, Academic Press, San Diego, CA (1990), or see: Gruber and Crosby, in: Methods in Plant Molecular Biology and Biotechnolgy, CRC Press, Boca Raton, Florida, Ed.: Glick and Thompson, chapter 7, 89-108, including the references cited therein. Regulatory sequences encompass those, which control the constitutive expression of a nucleotide sequence in many types of host cells and those which control the direct expression of the nucleotide sequence in specific host cells only, and under specific conditions. The skilled worker knows that the design of the expression vector may depend on factors such as the selection of the host cell to be transformed, the extent to which the desired protein is expressed, and the like. A preferred selection of regulatory sequences is described above, for example promoters, terminators, enhancers and the like. The term regulatory sequence is to be considered as being encompassed by the term regulatory signal. Several advantageous regulatory sequences, in particular promoters and terminators are described above. In general, the regulatory sequences described as advantageous for nucleic acid construct suitable for expression are also applicable for vectors.

The recombinant expression vectors used can be designed specifically for the expression, in prokaryotic and/or eukaryotic cells, of nucleic acid molecules used in the process. This is advantageous since intermediate steps of the vector construction are frequently carried out in microorganisms for the sake of simplicity. For example, the genes according to the invention and other genes can be expressed in bacterial cells, insect cells (using baculovirus expression vectors), yeast cells and other fungal cells [Romanos (1992), Yeast 8:423-488; van den Hondel, (1991), in: More Gene Manipulations in Fungi, J.W. Bennet & L.L. Lasure, Ed., pp. 396-428: Academic Press: San Diego; and van den Hondel, C.A.M.J.J. (1991), in: Applied Molecular Genetics of Fungi, Peberdy, J.F., et al., Ed., pp. 1-28, Cambridge University Press: Cambridge], algae [Falciatore et al., 1999, Marine Biotechnology.1, 3:239-251] using vectors and following a transformation method as described in WO 98/01572, and preferably in cells of multi-celled plants [see Schmidt, R. and Willmitzer, L. (1988) Plant Cell Rep.:583-586; Plant Molecular Biology and Biotechnology, C Press, Boca Raton, Florida, chapter 6/7, pp.71-119 (1993); F.F. White, in: Transgenic Plants, Bd. 1, Engineering and Utilization, Ed.: Kung and R. Wu, Academic Press (1993), 128-43; Potrykus, Annu. Rev. Plant Physiol. Plant Molec. Biol. 42 (1991), 205-225 (and references cited therein)]. Suitable host cells are furthermore discussed in Goeddel, Gene Expression Technology: Methods in Enzymology 185, Academic Press, San Diego, CA (1990). As an alternative, the sequence of the recombinant expression vector can be transcribed and translated in vitro, for example using T7 promotor-regulatory sequences and T7 polymerase.

Proteins can be expressed in prokaryotes using vectors comprising constitutive or inducible promoters, which control the expression of fusion proteins or nonfusion proteins. Typical fusion expression vectors are, inter alia, pGEX (Pharmacia Biotech Inc; Smith, D.B., and Johnson, K.S. (1988) Gene 67:31-40), pMAL (New England Biolabs, Beverly, MA) and pRIT5 (Pharmacia, Piscataway, NJ), in which glutathione-S-transferase (GST), maltose-E-binding protein or protein A is fused with the recombinant target protein. Examples of suitable inducible nonfusion E. coli expression vectors are, inter alia, pTrc (Amann et al. (1988) Gene 69:301-315) and pET 11 d [Studier et al., Gene Expression Technology: Methods in Enzymology 185, Academic Press, San Diego, California (1990) 60-89]. The target gene expression of the pTrc vector is based on the transcription of a hybrid trp-lac fusion promoter by the host RNA polymerase. The target gene expression from the pET 11d vector is based on the transcription of a T7-gn10-lac fusion promoter, which is mediated by a coexpressed viral RNA polymerase (T7 gn1). This viral polymerase is provided by the host strains BL21 (DE3) or HMS174 (DE3) by a resident λ-prophage which harbors a T7 gn1 gene under the transcriptional control of the lacUV 5 promoter.

Other vectors which are suitable in prokaryotic organisms are known to the skilled worker; these vectors are for example in E. coli pLG338, pACYC184, the pBR series, such as pBR322, the pUC series such as pUC18 or pUC19, the M113mp series, pKC30, pRep4, pHS1, pHS2, pPLc236, pMBL24, pLG200, pUR290, pIN-III¹¹³-B1, λgt11 or pBdCl, in Streptomyces pIJ101, pIJ364, pIJ702 or pIJ361, in Bacillus pUB110, pC194 or pBD214, in Corynebacterium pSA77 or pAJ667.

In a further embodiment, the expression vector is a yeast expression vector. Examples of vectors for expression in the yeasts S. cerevisiae encompass pYeDesaturasec1 (Baldari et al. (1987) Embo J. 6:229-234), pMFa (Kurjan and Herskowitz (1982) Cell 30:933-943), pJRY88 (Schultz et al. (1987) Gene 54:113-123) and pYES2 (Invitrogen Corporation, San Diego, CA). Vectors and methods for the construction of vectors which are suitable for use in other fungi, such as the filamentous fungi, encompass those which are described in detail in: van den Hondel, C.A.M.J.J. [(1991), J.F. Peberdy, Ed., pp. 1-28, Cambridge University Press: Cambridge; or in: More Gene Manipulations in Fungi; J.W. Bennet & L.L. Lasure, Ed., pp. 396-428: Academic Press: San Diego]. Examples of other suitable yeast vectors are 2uM, pAG-1, YEp6, YEp13 or pEMBLYe23.

Further vectors, which may be mentioned by way of example, are pALS1, pIL2 or pBB116 in fungi or pLGV23, pGHIac⁺, pBIN19, pAK2004 or pDH51 in plants.

As an alternative, the nucleic acid sequences can be expressed in insect cells using baculovirus expression vectors. Baculovirus vectors, which are available for expressing proteins in cultured insect cells (for example Sf9 cells) encompass the pAc series (Smith et al. (1983) Mol. Cell Biol. 3:2156-2165) and the pVL series (Lucklow and Summers (1989) Virology 170:31-39).

The abovementioned vectors are only a small overview of potentially suitable vectors. Further plasmids are known to the skilled worker and are described, for example, in: Cloning Vectors (Ed. Pouwels, P.H., et al., Elsevier, Amsterdam-New York-Oxford, 1985, ISBN 0 444 904018). Further suitable expression systems for prokaryotic and eukaryotic cells, see the chapters 16 and 17 by Sambrook, J., Fritsch, E.F., and Maniatis, T., Molecular Cloning: A Laboratory Manual, 2nd Edition, Cold Spring Harbor Laboratory, Cold Spring Harbor Laboratory Press, Cold Spring Harbor, NY, 1989.

Accordingly, one embodiment of the invention relates to a vector where the nucleic acid molecule according to the invention is linked operably to regulatory sequences which permit the expression in a prokaryotic or eukaryotic or in a prokaryotic and eukaryotic host.

Accordingly, one embodiment of the invention relates to a host cell, which has been transformed stably or transiently with the vector according to the invention or the nucleic acid molecule according to the invention or the nucleic acid construct according to the invention.

The present invention also relates to a process for the production of a polypeptide according to the present invention, the polypeptide being expressed in a host cell according to the invention, preferably in a microorganism or a transgenic plant cell.

In one embodiment, the nucleic acid molecule used in the process for the production of the polypeptide is derived from a microorganism, preferably from a prokaryotic or protozoic cell with an eukaryotic organism as host cell. E.g., in one embodiment the polypeptide is produced in a plant cell or plant with a nucleic acid molecule derived from a prokaryote or a fungus or an alga or an other microorganism but not from plant.

The skilled worker knows that protein and DNA expressed in different organisms differ in many respects and properties, e.g. DNA modulation and imprinting, such as methylation or post-translational modification, as for example glucosylation, phosphorylation, acetylation, myristoylation, ADP-ribosylation, farnesylation, carboxylation, sulfation, ubiquination, etc. though having the same coding sequence. Preferably, the cellular expression control of the corresponding protein differs accordingly in the control mechanisms controlling the activity and expression of an endogenous protein or another eukaryotic protein. One major difference between proteins expressed in prokaryotic or eukaryotic organisms is the amount and pattern of glycosylation. For example in E. coli there are no glycosylated proteins. Proteins expressed in yeasts have high mannose content in the glycosylated proteins, whereas in plants the glycosylation pattern is complex.

The polypeptide of the present invention is preferably produced by recombinant DNA techniques. For example, a nucleic acid molecule encoding the protein is cloned into a vector (as described above), the vector is introduced into a host cell (as described above) and said polypeptide is expressed in the host cell. Said polypeptide can then be isolated from the cells by an appropriate purification scheme using standard protein purification techniques. Alternative to recombinant expression, the polypeptide or peptide of the present invention can be synthesized chemically using standard peptide synthesis techniques.

Moreover, a native polypeptide conferring the increase of nitrogen or nitrogen containing compounds in an organism or part thereof can be isolated from cells (e.g., endothelial cells), for example using the antibody of the present invention as described below, in particular, an antibody against a protein as indicated in Table II, column 3. E.g. an antibody against a polypeptide as indicated in Table II, columns 5 or 7, or an antigenic part thereof which can be produced by standard techniques utilizing polypeptides comprising or consisting of above mentioned sequences, e.g. the polypeptid of the present invention or fragment thereof,. Preferred are monoclonal antibodies specifically binding to polypeptide as indicated in Table II, columns 5 or 7.

In one embodiment, the present invention relates to a polypeptide having the amino acid sequence encoded by a nucleic acid molecule of the invention or the nucleic acid molecule used in the method of the invention or obtainable by a process of the invention. Said polypeptide confers preferably the aforementioned activity, in particular, the polypeptide confers the increase in nitrogen or nitrogen containing compounds in a cell or an organism or a part thereof after increasing the cellular activity, e.g. by increasing the expression or the specific activity of the polypeptide.

In one embodiment, the present invention relates to a polypeptide having a sequence selected from the group as indicated in Table II, columns 5 or 7 or as encoded by a nucleic acid molecule selected from the group as indicated in Table I, columns 5 or 7 or functional homologues thereof.

In one advantageous embodiment, in the method of the present invention the activity of a polypeptide is increased which comprises or consists of a consensus sequence selected from the group as indicated in Table IV, column 7 and in one another embodiment, the present invention relates to a polypeptide comprising or consisting of a consensus sequence as indicated in Table IV, column 7 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, column 7.

In one embodiment not more than 15%, preferably 10%, even more preferred 5%, 4%, 3%, or 2%, most preferred 1% or 0% of the amino acid position indicated by a letter are/is replaced another amino acid or, in another embodiment, are/is absent and/or replaced. In another embodiment the stretches of non-conserved amino acids, indicated by (X), vary in their length by 20%, preferably by 15 or 10 %, even more preferred by 5%, 4%, 3%, 2% or most preferred by only 1 %.

In one embodiment 20 or less, preferably 15 or 10, preferably 9, 8, 7, or 6, more preferred 5 or 4, even more preferred 3, even more preferred 2, even more preferred 1, most preferred 0 amino acids are inserted into the consensus sequence or, in an other embodiment, are absent and/or replaced.

The consensus sequences were derived from multiple alignments of the sequences as listed in table II. The letters represent the one letter amino acid code and indicate that the amino acids are conserved in all aligned proteins. The letter X stands for amino acids, which are not conserved in all sequences. In one example, in the cases where only a small selected subset of amino acids are possible at a certain position these amino acids are given in brackets. The number of given X indicates the distances between conserved amino acid residues, e.g. Y-x(21,23)-F means that conserved tyrosine and phenylalanine residues are separated from each other by minimum 21 and maximum 23 amino acid residues in all investigated sequences.

Conserved domains were identified from all sequences and are described using a subset of the standard Prosite notation, e.g the pattern Y-x(21,23)-[FW] means that a conserved tyrosine is separated by minimum 21 and maximum 23 amino acid residues from either a phenylalanine or tryptophane.

Conserved patterns were identified with the software tool MEME version 3.5.1 or manually. MEME was developed by Timothy L. Bailey and Charles Elkan, Dept. of Computer Science and Engeneering, University of California, San Diego, USA and is described by Timothy L. Bailey and Charles Elkan [Fitting a mixture model by expectation maximization to discover motifs in biopolymers, Proceedings of the Second International Conference on Intelligent Systems for Molecular Biology, pp. 28-36, AAAI Press, Menlo Park, California, 1994]. The source code for the stand-alone program is public available from the San Diego Supercomputer center (http://meme.sdsc.edu).

For identifying common motifs in all sequences with the software tool MEME, the following settings were used: -maxsize 500000, -nmotifs 15, -evt 0.001, - maxw 60, -distance 1e-3, -minsites number of sequences used for the analysis. Input sequences for MEME were non-aligned sequences in Fasta format. Other parameters were used in the default settings in this software version.

Prosite patterns for conserved domains were generated with the software tool Pratt version 2.1 or manually. Pratt was developed by Inge Jonassen, Dept. of Informatics, University of Bergen, Norway and is described by Jonassen et al. [I.Jonassen, J.F.Collins and D.G.Higgins, Finding flexible patterns in unaligned protein sequences, Protein Science 4 (1995), pp. 1587-1595; I.Jonassen, Efficient discovery of conserved patterns using a pattern graph, Submitted to CABIOS Febr. 1997]. The source code (ANSI C) for the stand-alone program is public available, e.g. at establisched Bioinformatic centers like EBI (European Bioinformatics Institute).

For generating patterns with the software tool Pratt, following settings were used: PL (max Pattern Length): 100, PN (max Nr of Pattern Symbols): 100, PX (max Nr of consecutive x's): 30, FN (max Nr of flexible spacers): 5, FL (max Flexibility): 30, FP (max Flex.Product): 10, ON (max number patterns): 50. Input sequences for Pratt were distinct regions of the protein sequences exhibiting high similarity as identified from software tool MEME. The minimum number of sequences, which have to match the generated patterns (CM, min Nr of Seqs to Match) was set to at least 80% of the provided sequences. Parameters not mentioned here were used in their default settings.

The Prosite patterns of the conserved domains can be used to search for protein sequences matching this pattern. Various establisched Bioinformatic centers provide public internet portals for using those patterns in database searches (e.g. PIR [Protein Information Resource, located at Georgetown University Medical Center] or ExPASy [Expert Protein Analysis System]). Alternatively, stand-alone software is available, like the program Fuzzpro, which is part of the EMBOSS software package. For example, the program Fuzzpro not only allows to search for an exact pattern-protein match but also allows to set various ambiguities in the performed search.

The alignment was performed with the software ClustalW (version 1.83) and is described by Thompson et al. [Thompson, J.D., Higgins, D.G. and Gibson, T.J. (1994) CLUSTAL W: improving the sensitivity of progressive multiple sequence alignment through sequence weighting, positions-specific gap penalties and weight matrix choice. Nucleic Acids Research, 22:4673-4680]. The source code for the stand-alone program is public available from the European Molecular Biology Laboratory; Heidelberg, Germany. The analysis was performed using the default parameters of ClustalW v1.83 (gap open penalty: 10.0; gap extension penalty: 0.2; protein matrix: Gonnet; pprotein/DNA endgap: -1; protein/DNA gapdist: 4).

In one advantageous embodiment, the method of the present invention comprises the increasing of a polypeptide comprising or consisting of plant or microorganism specific consensus sequences. Accordingly, in one embodiment, the present invention relates to a polypeptide comprising or consisting of plant or microorganism specific consensus sequences.
In one embodiment, said polypeptide of the invention distinguishes over a sequence as indicated in Table II A or IIB, columns 5 or 7 by one or more amino acids. In one embodiment, polypeptide distinguishes form a sequence as indicated in Table II A or IIB, columns 5 or 7by more than 1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II A or II B, columns 5 or 7 by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II A or II B, columns 5 or 7.

In one embodiment, the polypeptide of the invention comprises any one of the sequences not known to the public before. In one embodiment, the polypeptide of the invention originates from a non-plant cell, in particular from a microorganism, and was expressed in a plant cell. In one embodiment, the present invention relates to a polypeptide encoded by the nucleic acid molecule of the invention or used in the process of the invention for which an activity has not been described yet. In a further preferred embodiment the polypeptide of the invention comprises any of the sequences show in table II column 5 or 7 application no 2 or no. 3 and further a plastidal targeting sequence.

In one embodiment, the invention relates to polypeptide conferring an increase in nitrogen or nitrogen containing compounds in an organism or part thereof being encoded by the nucleic acid molecule of the invention or by a nucleic acid molecule used in the process of the invention.
In one embodiment, the polypeptide of the invention has a sequence which distinguishes from a sequence as indicated in Table II A or II B, columns 5 or 7 by one or more amino acids. In an other embodiment, said polypeptide of the invention does not consist of the sequence as indicated in Table II A or II B, columns 5 or 7. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I A or IB, columns 5 or 7. In one embodiment said polypeptide is a fusionpeptide with a plastidal targeting sequence.

In one embodiment, the present invention relates to a polypeptide having an activity of a protein as indicated in Table II, column 3, which distinguishes over a sequence as indicated in Table II A or II B, columns 5 or 7 by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

The terms "protein" and "polypeptide" used in this application are interchangeable. "Polypeptide" refers to a polymer of amino acids (amino acid sequence) and does not refer to a specific length of the molecule. Thus peptides and oligopeptides are included within the definition of polypeptide. This term does also refer to or include post-translational modifications of the polypeptide, for example, glycosylations, acetylations, phosphorylations and the like. Included within the definition are, for example, polypeptides containing one or more analogs of an amino acid (including, for example, unnatural amino acids, etc.), polypeptides with substituted linkages, as well as other modifications known in the art, both naturally occurring and non-naturally occurring.

Preferably, the polypeptide is isolated. An "isolated" or "purified" protein or nucleic acid molecule or biologically active portion thereof is substantially free of cellular material when produced by recombinant DNA techniques or chemical precursors or other chemicals when chemically synthesized.

The language "substantially free of cellular material" includes preparations of the polypeptide of the invention in which the protein is separated from cellular components of the cells in which it is naturally or recombinantly produced. In one embodiment, the language "substantially free of cellular material" includes preparations having less than about 30% (by dry weight) of "contaminating protein", more preferably less than about 20% of "contaminating protein", still more preferably less than about 10% of "contaminating protein", and most preferably less than about 5% "contaminating protein". The term "Contaminating protein" relates to polypeptides, which are not polypeptides of the present invention. When the polypeptide of the present invention or biologically active portion thereof is recombinantly produced, it is also preferably substantially free of culture medium, i.e., culture medium represents less than about 20%, more preferably less than about 10%, and most preferably less than about 5% of the volume of the protein preparation. The language "substantially free of chemical precursors or other chemicals" includes preparations in which the polypeptide of the present invention is separated from chemical precursors or other chemicals, which are involved in the synthesis of the protein. The language "substantially free of chemical precursors or other chemicals" includes preparations having less than about 30% (by dry weight) of chemical precursors or non-polypeptide of the invention-chemicals, more preferably less than about 20% chemical precursors or non-polypeptide of the invention-chemicals, still more preferably less than about 10% chemical precursors or non-polypeptide of the invention-chemicals, and most preferably less than about 5% chemical precursors or non- polypeptide of the invention-chemicals. In preferred embodiments, isolated proteins or biologically active portions thereof lack contaminating proteins from the same organism from which the polypeptide of the present invention is derived. Typically, such proteins are produced by recombinant techniques.

Non-polypeptide of the invention-chemicals are e.g. polypeptides having not the activity and/or amino acid sequence of a polypeptide indicated in Table II, columns 3, 5 or 7.

A polypeptide of the invention can participate in the process of the present invention. The polypeptide or a portion thereof comprises preferably an amino acid sequence which is sufficiently homologous to an amino acid sequence as indicated in Table II, columns 5 or 7 such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, columns 5 or 7.

Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, columns 5 or 7. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, columns 5 or 7 or which is homologous thereto, as defined above.

Accordingly the polypeptide of the present invention can vary from a sequence as indicated in Table II, columns 5 or 7 in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91 %, 92%, 93%, 94% or 95%, and most preferably at least about 96%, 97%, 98%, 99% or more homologous to an entire amino acid sequence of a sequence as indicated in Table II A or IIB, columns 5 or 7.

For the comparison of amino acid sequences the same algorithms as described above or nucleic acid sequences can be used. Results of high quality are reached by using the algorithm of Needleman and Wunsch or Smith and Waterman. Therefore programs based on said algorithms are preferred. Advantageously the comparisons of sequences can be done with the program PileUp (J. Mol. Evolution., 25, 351-360, 1987, Higgins et al., CABIOS, 5 1989: 151-153) or preferably with the programs Gap and BestFit, which are respectively based on the algorithms of Needleman and Wunsch [J. Mol. Biol. 48; 443-453 (1970)] and Smith and Waterman [Adv. Appl. Math. 2; 482-489 (1981)]. Both programs are part of the GCG software-package [Genetics Computer Group, 575 Science Drive, Madison, Wisconsin, USA 53711 (1991); Altschul et al. (1997) Nucleic Acids Res. 25:3389 et seq.]. Therefore preferably the calculations to determine the percentages of sequence homology are done with the program Gap over the whole range of the sequences. The following standard adjustments for the comparison of amino acid sequences were used: gap weight: 8, length weight: 2, average match: 2.912, average mismatch: -2.003.

Biologically active portions of an polypeptide of the present invention include peptides comprising amino acid sequences derived from the amino acid sequence of the polypeptide of the present invention or used in the process of the present invention, e.g., an amino acid sequence as indicated in Table II, columns 5 or 7 or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

Typically, biologically (or immunologically) active portions i.e. peptides, e.g., peptides which are, for example, 5, 10, 15, 20, 30, 35, 36, 37, 38, 39, 40, 50, 100 or more amino acids in length comprise a domain or motif with at least one activity or epitope of a polypeptide of the present invention or used in the process of the present invention. Moreover, other biologically active portions, in which other regions of the polypeptide are deleted, can be prepared by recombinant techniques and evaluated for one or more of the activities described herein.

Manipulation of the nucleic acid molecule of the invention or the nucleic acid molecule used in the method of the invention may result in the production of a protein having essentially the activity of the polypeptides as indicated in Table II, column 3 but having differences in the sequence from said wild-type protein. These proteins may be improved in efficiency or activity, may be present in greater numbers in the cell than is usual, or may be decreased in efficiency or activity in relation to the wild type protein.

Any mutagenesis strategies for the polypeptide of the present invention or the polypeptide used in the process of the present invention to result in increasing said activity are not meant to be limiting; variations on these strategies will be readily apparent to one skilled in the art. Using such strategies, and incorporating the mechanisms disclosed herein, the nucleic acid molecule and polypeptide of the invention or the polypeptide used in the method of the invention may be utilized to generate plants or parts thereof, expressing one or more wildtype protein(s) or one or more mutated protein encoding nucleic acid molecule(s) or polypeptide molecule(s) of the invention such that the yield, production, and/or efficiency of production of a desired compound is improved.

This desired compound may be any natural product of plants, which includes the final products of biosynthesis pathways and intermediates of naturally-occurring metabolic pathways, as well as molecules which do not naturally occur in the metabolism of said cells, but which are produced by a said cells of the invention. Preferably, the compound is a composition comprising nitrogen or nitrogen containing compounds.

**%**

The invention also provides chimeric or fusion proteins.

As used herein, an "chimeric protein" or "fusion protein" comprises an polypeptide operatively linked to a polypeptide which does not confer above-mentioned activity, in particular, which does not confer an increase of content of nitrogen or nitrogen containing compounds in a cell or an organism or a part thereof, if its activity is increased.

In one embodiment, an reference to a " protein (= polypeptide) of the invention" or as indicated in Table II, columns 5 or 7 refers to a polypeptide having an amino acid sequence corresponding to the polypeptide of the invention or used in the process of the invention, whereas a "non-polypeptide of the invention" or "other polypeptide" not being indicated in Table II, columns 5 or 7refers to a polypeptide having an amino acid sequence corresponding to a protein which is not substantially homologous a polypeptide of the invention, preferably which is not substantially homologous to a polypeptide as indicated in Table II, columns 5 or 7 e.g., a protein which does not confer the activity described herein or annotated or known for as indicated in Table II, column 3 and which is derived from the same or a different organism. In one embodiment, a "non-polypeptide of the invention" or "other polypeptide" not being indicated in Table II, columns 5 or 7 does not confer an increase of nitrogen or nitrogen containing compounds in an organism or part thereof.

Within the fusion protein, the term "operatively linked" is intended to indicate that the polypeptide of the invention or a polypeptide used in the process of the invention and the "other polypeptide" or a part thereof are fused to each other so that both sequences fulfil the proposed function addicted to the sequence used. The "other polypeptide" can be fused to the N-terminus or C-terminus of the polypeptide of the invention or used in the process of the invention. For example, in one embodiment the fusion protein is a GST-LMRP fusion protein in which the sequences of the polypeptide of the invention or the polypeptide used in the process of the invention are fused to the C-terminus of the GST sequences. Such fusion proteins can facilitate the purification of recombinant polypeptides of the invention or a polypeptide useful in the process of the invention.

In another embodiment, the fusion protein is a polypeptide of the invention or a polypeptide used in the process of the invention containing a heterologous signal sequence at its N-terminus. In certain host cells (e.g., mammalian host cells), expression and/or secretion of a polypeptide of the invention or a polypeptide used in the process of the invention can be increased through use of a heterologous signal sequence. As already mentioned above, targeting sequences, are required for targeting the gene product into specific cell compartment (for a review, see Kermode, Crit. Rev. Plant Sci. 15, 4 (1996) 285-423 and references cited therein), for example into the vacuole, the nucleus, all types of plastids, such as amyloplasts, chloroplasts, chromoplasts, the extracellular space, the mitochondria, the endoplasmic reticulum, elaioplasts, peroxisomes, glycosomes, and other compartments of cells or extracellular. Sequences, which must be mentioned in this context are, in particular, the signal-peptide- or transit-peptide-encoding sequences which are known per se. For example, plastid-transit-peptide-encoding sequences enable the targeting of the expression product into the plastids of a plant cell. Targeting sequences are also known for eukaryotic and to a lower extent for prokaryotic organisms and can advantageously be operable linked with the nucleic acid molecule of the present invention to achieve an expression in one of said compartments or extracellular. Therefore In a preferred embodiment the polypeptide of the invention, specifically the polypeptide encompassing a sequence as shown in table II column 5 or 7 are in "operative linkage" with a plastidal targeting sequence, resulting in a functional fusion protein, which is able to direct the fusion protein to the plastidal compartment and which mediates the import of the polypeptide of the invention, specifically the polypeptide encompassing a sequence as shown in table II, column 5 or 7, into the plastidial compartment.

Preferably, a chimeric or fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. The fusion gene can be synthesized by conventional techniques including automated DNA synthesizers. Alternatively, PCR amplification of gene fragments can be carried out using anchor primers, which give rise to complementary overhangs between two consecutive gene fragments which can subsequently be annealed and reamplified to generate a chimeric gene sequence (see, for example, Current Protocols in Molecular Biology, eds. Ausubel et al. John Wiley & Sons: 1992). Moreover, many expression vectors are commercially available that already encode a fusion moiety (e.g., a GST polypeptide). The nucleic acid molecule of the invention or the nucleic acid molecule used in the method of the invention can be cloned into such an expression vector such that the fusion moiety is linked in-frame to the encoded protein.

Furthermore, folding simulations and computer redesign of structural motifs of the protein of the invention can be performed using appropriate computer programs (Olszewski, Proteins 25 (1996), 286-299; Hoffman, Comput. Appl. Biosci. 11 (1995), 675-679). Computer modelling of protein folding can be used for the conformational and energetic analysis of detailed peptide and protein models (Monge, J. Mol. Biol. 247 (1995), 995-1012; Renouf, Adv. Exp. Med. Biol. 376 (1995), 37-45). The appropriate programs can be used for the identification of interactive sites the polypeptide of the invention or polypeptides used in the process of the invention and its substrates or binding factors or other interacting proteins by computer assistant searches for complementary peptide sequences (Fassina, Immunomethods (1994), 114-120). Further appropriate computer systems for the design of protein and peptides are described in the prior art, for example in Berry, Biochem. Soc. Trans. 22 (1994), 1033-1036; Wodak, Ann. N. Y. Acad. Sci. 501 (1987),1-13; Pabo, Biochemistry 25 (1986), 5987-5991. The results obtained from the above-described computer analysis can be used for, e.g., the preparation of peptidomimetics of the protein of the invention or fragments thereof. Such pseudopeptide analogues of the, natural amino acid sequence of the protein may very efficiently mimic the parent protein (Benkirane, J. Biol. Chem. 271 (1996), 33218-33224). For example, incorporation of easily available achiral Q-amino acid residues into a protein of the invention or a fragment thereof results in the substitution of amide bonds by polymethylene units of an aliphatic chain, thereby providing a convenient strategy for constructing a peptidomimetic (Banerjee, Biopolymers 39 (1996), 769-777).

Superactive peptidomimetic analogues of small peptide hormones in other systems are described in the prior art (Zhang, Biochem. Biophys. Res. Commun. 224 (1996), 327-331). Appropriate peptidomimetics of the protein of the present invention can also be identified by the synthesis of peptidomimetic combinatorial libraries through successive amide alkylation and testing the resulting compounds, e.g., for their binding and immunological properties. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715.

Furthermore, a three-dimensional and/or crystallographic structure of the protein of the invention can be used for the design of peptidomimetic inhibitors of the biological activity of the protein of the invention (Rose, Biochemistry 35 (1996), 12933-12944; Rutenber, Bioorg. Med. Chem. 4 (1996),1545-1558).

Furthermore, a three-dimensional and/or crystallographic structure of the protein of the invention and the identification of interactive sites the polypeptide of the invention or the polypeptide used in the method of the invention and its substrates or binding factors can be used for the identification or design of mutants with modulated binding or turn over activities. For example, the active centre of the polypeptide of the present invention can be modelled and amino acid residues participating in the catalytic reaction can be modulated to increase or decrease the binding of the substrate to activate or improve the polypeptide. The identification of the active centre and the amino acids involved in the catalytic reaction facilitates the screening for mutants having an increased activity.

The sequences shown herein have also been described under their protein name as described in the Table I, II, III or IV, column 3.

In an especially preferred embodiment, the polypeptide according to the invention furthermore also does not have the sequences of those proteins which are encoded by the sequences shown in the Table II, column 5 or 7.

One embodiment of the invention also relates to an antibody, which binds specifically to the polypeptide according to the invention or parts, i.e. specific fragments or epitopes of such a protein.

The antibodies of the invention can be used to identify and isolate the polypeptide according to the invention and encoding genes in any organism, preferably plants, prepared in plants described herein. These antibodies can be monoclonal antibodies, polyclonal antibodies or synthetic antibodies as well as fragments of antibodies, such as Fab, Fv or scFv fragments etc. Monoclonal antibodies can be prepared, for example, by the techniques as originally described in Köhler and Milstein, Nature 256 (1975), 495, and Galfr6, Meth. Enzymol. 73 (1981), 3, which comprise the fusion of mouse myeloma cells to spleen cells derived from immunized mammals.

Furthermore, antibodies or fragments thereof to the aforementioned peptides can be obtained by using methods, which are described, e.g., in Harlow and Lane "Antibodies, A Laboratory Manual", CSH Press, Cold Spring Harbor, 1988. These antibodies can be used, for example, for the immunoprecipitation and immunolocalization of proteins according to the invention as well as for the monitoring of the synthesis of such proteins, for example, in recombinant organisms, and for the identification of compounds interacting with the protein according to the invention. For example, surface plasmon resonance as employed in the BIAcore system can be used to increase the efficiency of phage antibodies selections, yielding a high increment of affinity from a single library of phage antibodies, which bind to an epitope of the protein of the invention (Schier, Human Antibodies Hybridomas 7 (1996), 97-105; Malmborg, J. Immunol. Methods 183 (1995), 7-13). In many cases, the binding phenomena of antibodies to antigens are equivalent to other ligand/anti-ligand binding.

In one embodiment, the present invention relates to an antisense nucleic acid molecule comprising the complementary sequence of the nucleic acid molecule of the present invention.

Methods to modify the expression levels and/or the activity are known to persons skilled in the art and include for instance overexpression, co-suppression, the use of ribozymes, sense and anti-sense strategies or other gene silencing approaches like RNA interference (RNAi) or promoter methylation. "Sense strand" refers to the strand of a double-stranded DNA molecule that is homologous to an mRNA transcript thereof. The "anti-sense strand" contains an inverted sequence, which is complementary to that of the "sense strand".
In addition the expression levels and/or the activity can be modified by the introduction of mutations in the regulatory or coding regions of the nucleic acids of the invention. Furthermore antibodies can be expressed which specifically binds to a polypeptide of interest and thereby blocks it activity. The protein-binding factors can, for example, also be aptamers [Famulok M and Mayer G (1999) Curr. Top Microbiol. Immunol. 243: 123-36] or antibodies or antibody fragments or single-chain antibodies. Obtaining these factors has been described, and the skilled worker is familiar therewith. For example, a cytoplasmic scFv antibody has been employed for modulating activity of the phytochrome A protein in genetically modified tobacco plants [Owen M et al. (1992) Biotechnology (NY) 10(7): 790-794; Franken E et al. (1997) Curr. Opin. Biotechnol. 8(4): 411-416; Whitelam (1996) Trend Plant Sci. 1: 286-272]. In a further preferred embodiment of the invention the expression level and/or the activity can be changed through modifications of the internal regulators. The person skilled in the art is familiar with the different options of internal regulators which can be used to modify the expression level of the genes or proteins of the invention. In exemplary embodiment an negative transcriptional regulator, e.g. a repressor of a nucleic acid of the invention is inhibited or knockout, for example through antisense or RNAi inhibition, leading to an enhanced expression of the nucleic acid of the invention. Similarly a allosteric inhibitor of a protein of the invention can be sujected to downregulation by mutation, or antisense or RNAi inhibition.

An "antisense" nucleic acid molecule comprises a nucleotide sequence, which is complementary to a "sense" nucleic acid molecule encoding a protein, e.g., complementary to the coding strand of a double-stranded cDNA molecule or complementary to an encoding mRNA sequence. Accordingly, an antisense nucleic acid molecule can bond via hydrogen bonds to a sense nucleic acid molecule. The antisense nucleic acid molecule can be complementary to an entire coding strand of a nucleic acid molecule conferring the regulation or expression of the polypeptide of the invention or used in the process of the present invention, as the nucleic acid molecule of the invention or the nucleic acid molecule used in the method of the invention coding strand, or to only a portion thereof.

**%**

**%**

**%**

**%**

**%**

**%**

**%**

**%**

**%**

**%**

**%**

**%**

**%**

**%**

**%**

**%,**

**%**

**%.**

**%**

**%**

**%**

**%**

**%**.

**%**.

A further embodiment of the invention also relates to a method for the generation of a transgenic host or host cell, e.g. a eukaryotic or prokaryotic cell, preferably a transgenic microorganism, a transgenic plant cell or a transgenic plant tissue or a transgenic plant, which comprises introducing, into the plant, the plant cell or the plant tissue, the nucleic acid construct according to the invention, the vector according to the invention, or the nucleic acid molecule according to the invention.

A further embodiment of the invention also relates to a method for the transient generation of a host or host cell, eukaryotic or prokaryotic cell, preferably a transgenic microorganism, a transgenic plant cell or a transgenic plant tissue or a transgenic plant, which comprises introducing, into the plant, the plant cell or the plant tissue, the nucleic acid construct according to the invention, the vector according to the invention, the nucleic acid molecule characterized herein as being contained in the nucleic acid construct of the invention or the nucleic acid molecule according to the invention, whereby the introduced nucleic acid molecules, nucleic acid construct and/or vector is not integrated into the genome of the host or host cell. Therefore the transformants are not stable during the propagation of the host in respect of the introduced nucleic acid molecules, nucleic acid construct and/or vector.

In the process according to the invention, transgenic organisms are also to be understood as meaning - if they take the form of plants - plant cells, plant tissues, plant organs such as root, shoot, stem, seed, flower, tuber or leaf, or intact plants which are grown for enhanced nitrogen assimilation, accumulation and/or utilization.

Growing is to be understood as meaning for example culturing the transgenic plant cells, plant tissue or plant organs on or in a nutrient medium or the intact plant on or in a substrate, for example in hydroponic culture, potting compost or on a field soil. In one specfic embodiment growing relates to the grow of the transgenic plants, plant cells, plant tissue under nitrogen limited conditions.

In a further advantageous embodiment of the process, the nucleic acid molecules can be expressed in single-celled plant cells (such as algae), see Falciatore et al., 1999, Marine Biotechnology 1 (3): 239-251 and references cited therein, and plant cells from higher plants (for example spermatophytes such as crops). Examples of plant expression vectors encompass those which are described in detail herein or in: Becker, D. [(1992) Plant Mol. Biol. 20:1195-1197] and Bevan, M.W. [(1984), Nucl. Acids Res. 12:8711-8721; Vectors for Gene Transfer in Higher Plants; in: Transgenic Plants, Vol. 1, Engineering and Utilization, Ed.: Kung and R. Wu, Academic Press, 1993, pp. 15-38]. An overview of binary vectors and their use is also found in Hellens, R. [(2000), Trends in Plant Science, Vol. 5 No.10, 446-451.

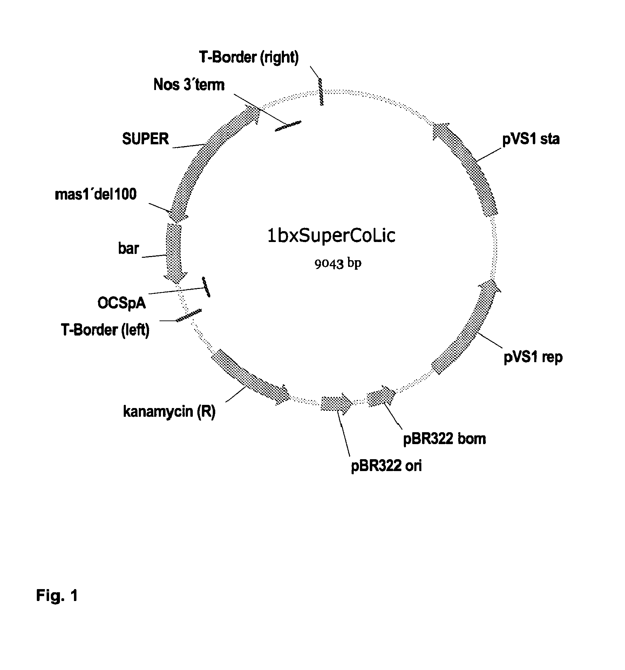

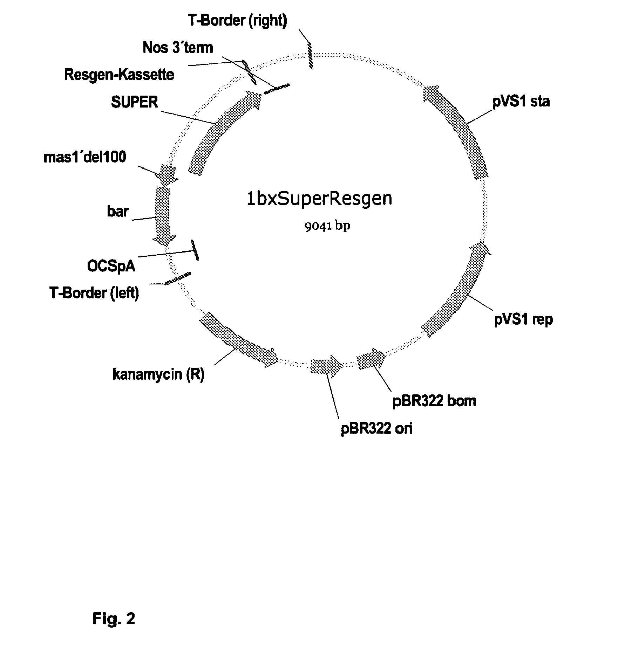

In one embodiment of the invention, plant expression vectors encompass those which are described in the figures: Fig. 1 and/or Fig.2.

Vector DNA can be introduced into prokaryotic or eukaryotic cells via conventional transformation or transfection techniques. The terms "transformation" and "transfection" include conjugation and transduction and, as used in the present context, are intended to encompass a multiplicity of prior-art methods for introducing foreign nucleic acid molecules (for example DNA) into a host cell, including calcium phosphate coprecipitation or calcium chloride coprecipitation, DEAE-dextran-mediated transfection, PEG-mediated transfection, lipofection, natural competence, chemically mediated transfer, electroporation or particle bombardment. Suitable methods for the transformation or transfection of host cells, including plant cells, can be found in Sambrook et al. (Molecular Cloning: A Laboratory Manual., 2nd Ed., Cold Spring Harbor Laboratory, Cold Spring Harbor Laboratory Press, Cold Spring Harbor, NY, 1989) and in other laboratory handbooks such as Methods in Molecular Biology, 1995, Vol. 44, Agrobacterium protocols, Ed.: Gartland and Davey, Humana Press, Totowa, New Jersey.

The above-described methods for the transformation and regeneration of plants from plant tissues or plant cells are exploited for transient or stable transformation of plants. Suitable methods are the transformation of protoplasts by polyethylene-glycol-induced DNA uptake, the biolistic method with the gene gun - known as the particle bombardment method -, electroporation, the incubation of dry embryos in DNA-containing solution, microinjection and the Agrobacterium-mediated gene transfer. The abovementioned methods are described for example in B. Jenes, Techniques for Gene Transfer, in: Transgenic Plants, Vol. 1, Engineering and Utilization, edited by S.D. Kung and R. Wu, Academic Press (1993) 128-143 and in Potrykus Annu. Rev. Plant Physiol. Plant Molec. Biol. 42 (1991) 205-225. The construct to be expressed is preferably cloned into a vector, which is suitable for transforming Agrobacterium tumefaciens, for example pBin19 (Bevan, Nucl. Acids Res. 12 (1984) 8711). Agrobacteria transformed with such a vector can then be used in the known manner for the transformation of plants, in particular crop plants, such as, for example, tobacco plants, for example by bathing scarified leaves or leaf segments in an agrobacterial solution and subsequently culturing them in suitable media. The transformation of plants with Agrobacterium tumefaciens is described for example by Höfgen and Willmitzer in Nucl. Acid Res. (1988) 16, 9877 or known from, inter alia, F.F. White, Vectors for Gene Transfer in Higher Plants; in Transgenic Plants, Vol. 1, Engineering and Utilization, edited by S.D. Kung and R. Wu, Academic Press, 1993, pp. 15-38.

To select for the successful transfer of the nucleic acid molecule, vector or nucleic acid construct of the invention according to the invention into a host organism, it is advantageous to use marker genes as have already been described above in detail. It is known of the stable or transient integration of nucleic acids into plant cells that only a minority of the cells takes up the foreign DNA and, if desired, integrates it into its genome, depending on the expression vector used and the transfection technique used. To identify and select these integrants, a gene encoding for a selectable marker (as described above, for example resistance to antibiotics) is usually introduced into the host cells together with the gene of interest. Preferred selectable markers in plants comprise those, which confer resistance to an herbicide such as glyphosate or gluphosinate. Other suitable markers are, for example, markers, which encode genes involved in biosynthetic pathways of, for example, sugars or amino acids, such as ß-galactosidase, ura3, ilv2 or a mutated acetohydroxyacid synthase (AHAS) gene, also known as acetolactate synthase (ALS) gene or a gene for a D-amino acid metabolizing enzmye. Markers, which encode genes such as luciferase, gfp or other fluorescence genes, are likewise suitable. Additional markers named in the literature sometimes as secondary markers, genes coding for the resistance against herbicides such as phosphinothricin (= glufosinate, BASTA™, Liberty™, encoded by the bar gene), glyphosate (= N-(phosphonomethyl)glycine, Roundup Ready™, encoded by the 5-enolpyruvylshikimate-3-phosphate synthase gene = epsps), sulfonylurea (= Staple™, encoded by the acetolactate synthase gene), imidazolinone [= IMI, imazethapyr, imazamox, Clearfield™, encoded by the acetohydroxyacid synthase (AHAS) gene, also known as acetolactate synthase (ALS) gene] or bromoxynil (= Buctril™, encoded by the oxy gene) or genes coding for antibiotics such as hygromycin or G418 are useful for selection. In addition negative selection markers such as the bacterial cytosine deaminase (encoded by the codA gene) are also useful for the transformation of plastids.
These markers and the aforementioned markers can be used in mutants in whom these genes are not functional since, for example, they have been deleted by conventional methods. Furthermore, nucleic acid molecules, which encode a selectable marker, can be introduced into a host cell on the same vector as those, which encode the polypeptides of the invention or used in the process or else in a separate vector. Cells which have been transfected stably with the nucleic acid introduced can be identified for example by selection (for example, cells which have integrated the selectable marker survive whereas the other cells die).

Since the marker genes, as a rule specifically the gene for resistance to antibiotics and herbicides, are no longer required or are undesired in the transgenic host cell once the nucleic acids have been introduced successfully, the process according to the invention for introducing the nucleic acids advantageously employs techniques which enable the removal, or excision, of these marker genes. One such a method is what is known as cotransformation. The cotransformation method employs two vectors simultaneously for the transformation, one vector bearing the nucleic acid according to the invention and a second bearing the marker gene(s). A large proportion of transformants receives or, in the case of plants, comprises (up to 40% of the transformants and above), both vectors. In case of transformation with Agrobacteria, the transformants usually receive only a part of the vector, the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase resource or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases, the marker gene must be eliminated by performing crosses. In microbiology, techniques were developed which make possible, or facilitate, the detection of such events. A further advantageous method relies on what are known as recombination systems, whose advantage is that elimination by crossing can be dispensed with. The best-known system of this type is what is known as the Cre/lox system. Cre1 is a recombinase, which removes the sequences located between the IoxP sequences. If the marker gene is integrated between the IoxP sequences, it is removed, once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Agrobacteria transformed with an expression vector according to the invention may also be used in the manner known per se for the transformation of plants such as experimental plants like Arabidopsis or crop plants, such as, for example, cereals, maize, oats, rye, barley, wheat, soya, rice, cotton, sugarbeet, canola, sunflower, flax, hemp, potato, tobacco, tomato, carrot, bell peppers, oilseed rape, tapioca, cassava, arrow root, tagetes, alfalfa, lettuce and the various tree, nut, and grapevine species, in particular oil-containing crop plants such as soya, peanut, castor-oil plant, sunflower, maize, cotton, flax, oilseed rape, cotton, coconut, oil palm, safflower (Carthamus tinctorius) or cocoa beans, for example by bathing scarified leaves or leaf segments in an agrobacterial solution and subsequently growing them in suitable media.

In addition to the transformation of somatic cells, which then has to be regenerated into intact plants, it is also possible to transform the cells of plant meristems and in particular those cells which develop into gametes. In this case, the transformed gametes follow the natural plant development, giving rise to transgenic plants. Thus, for example, seeds of Arabidopsis are treated with agrobacteria and seeds are obtained from the developing plants of which a certain proportion is transformed and thus transgenic (Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289). Alternative methods are based on the repeated removal of the influorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of Arabidopsis, intact plants under reduced pressure are treated with an agrobacterial suspension (Bechthold, N (1993). C R Acad Sci Paris Life Sci, 316: 1194-1199), while in the case of the"floral dip" method the developing floral tissue is incubated briefly with a surfactant-treated agrobacterial suspension (Clough, SJ und Bent, AF (1998). The Plant J. 16, 735-743). A certain proportion of transgenic seeds are harvested in both cases, and these seeds can be distinguished from nontransgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process, which has been schematically displayed in Klaus et al., 2004 (Nature Biotechnology 22(2), 225-229). Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview can be taken from Bock (2001) Transgenic plastids in basic research and plant biotechnology. J Mol Biol. 2001 Sep 21; 312 (3): 425-38 or Maliga, P (2003) Progress towards commercialization of plastid transformation technology. Trends Biotechnol. 21, 20-28. Further biotechnological progress has recently been reported in form of marker free plastid transformants, which can be produced by a transient cointegrated maker gene (Klaus et al., 2004, Nature Biotechnology 22 (2), 225-229).

The genetically modified plant cells can be regenerated via all methods with which the skilled worker is familiar. Suitable methods can be found in the abovementioned publications by S.D. Kung and R. Wu, Potrykus or Höfgen and Willmitzer.

Accordingly, the present invention thus also relates to a plant cell comprising the nucleic acid construct according to the invention, the nucleic acid molecule according to the invention or the vector according to the invention.

Accordingly the present invention relates to any cell transgenic for any nucleic acid characterized as part of the invention, e.g. conferring the increase of nitrogen or nitrogen containing compounds in a cell or an organism or a part thereof, e.g. the nucleic acid molecule of the invention or the nucleic acid molecule used in the method of the invention, the nucleic acid construct of the invention, the antisense molecule of the invention, the vector of the invention or a nucleic acid molecule encoding the polypeptide of the invention or the polypeptide used in the method of the invention, e.g. the polypeptide as indicated in Table II, columns 5 or 7. Due to the above mentioned activity nitrogen or nitrogen containing compound content in a cell or an organism is increased. For example, due to modulation or manipulation, the cellular activity of the polypeptide of the invention or the polypeptide used in the method of the invention or the nucleic acid molecule of the invention or the nucleic acid molecule used in the method of the invention is increased, e.g. due to an increased expression or specific activity of the subject matters of the invention in a cell or an organism or a part thereof. In one embodiment, transgenic for a polypeptide having an activity of a polypeptide as indicated in Table II, columns 5 or 7 means herein that due to modulation or manipulation of the genome, an activity as annotated for a polypeptide as indicated in Table II, column 3, e.g. having a sequence as indicated in Table II, columns 5 or 7 is increased in a cell or an organism or a part thereof. Examples are described above in context with the process of the invention

"Transgenic", for example regarding a nucleic acid molecule, an nucleic acid construct or a vector comprising said nucleic acid molecule or an organism transformed with said nucleic acid molecule, nucleic acid construct or vector, refers to all those subjects originating by recombinant methods in which either
**a)** the nucleic acid sequence, or
**b)** a genetic control sequence linked operably to the nucleic acid sequence, for example a promoter, or
**c)** (a) and (b)
are not located in their natural genetic environment or have been modified by recombinant methods, an example of a modification being a substitution, addition, deletion, inversion or insertion of one or more nucleotide residues. Natural genetic environment refers to the natural chromosomal locus in the organism of origin, or to the presence in a genomic library. In the case of a genomic library, the natural genetic environment of the nucleic acid sequence is preferably retained, at least in part. The environment flanks the nucleic acid sequence at least at one side and has a sequence of at least 50 bp, preferably at least 500 bp, especially preferably at least 1000 bp, very especially preferably at least 5000 bp, in length.

A naturally occurring expression cassette - for example the naturally occurring combination of a promoter of a polypeptide of the invention with the corresponding protein-encoding sequence - becomes a transgenic expression cassette when it is modified by non-natural, synthetic "artificial" methods such as, for example, mutagenization. Such methods have been described (US 5,565,350; WO 00/15815; also see above).

Further, the plant cell, plant tissue or plant can also be transformed such that further enzymes and proteins are (over)expressed which expression supports an increase of nitrogen or nitrogen containing compounds.

However, transgenic also means that the nucleic acids according to the invention are located at their natural position in the genome of an organism, but that the sequence has been modified in comparison with the natural sequence and/or that the regulatory sequences of the natural sequences have been modified. Preferably, transgenic/recombinant is to be understood as meaning the transcription of the nucleic acids used in the process according to the invention occurs at a non-natural position in the genome, that is to say the expression of the nucleic acids is homologous or, preferably, heterologous. This expression can be transiently or of a sequence integrated stably into the genome.

The term "transgenic plants" used in accordance with the invention also refers to the progeny of a transgenic plant, for example the T₁, T₂, T₃ and subsequent plant generations or the BC₁, BC₂, BC₃ and subsequent plant generations. Thus, the transgenic plants according to the invention can be raised and selfed or crossed with other individuals in order to obtain further transgenic plants according to the invention. Transgenic plants may also be obtained by propagating transgenic plant cells vegetatively. The present invention also relates to transgenic plant material, which can be derived from a transgenic plant population according to the invention. Such material includes plant cells and certain tissues, organs and parts of plants in all their manifestations, such as seeds, leaves, anthers, fibers, tubers, roots, root hairs, stems, embryo, calli, cotelydons, petioles, harvested material, plant tissue, reproductive tissue and cell cultures, which are derived from the actual transgenic plant and/or can be used for bringing about the transgenic plant.

Any transformed plant obtained according to the invention can be used in a conventional breeding scheme or in *in vitro* plant propagation to produce more transformed plants with the same characteristics and/or can be used to introduce the same characteristic in other varieties of the same or related species. Such plants are also part of the invention. Seeds obtained from the transformed plants genetically also contain the same characteristic and are part of the invention. As mentioned before, the present invention is in principle applicable to any plant and crop that can be transformed with any of the transformation method known to those skilled in the art.

In an especially preferred embodiment, the organism, the host cell, plant cell, plant, microorganism or plant tissue according to the invention is transgenic.

Accordingly, the invention therefore relates to transgenic organisms transformed with at least one nucleic acid molecule, nucleic acid construct or vector according to the invention, and to cells, cell cultures, tissues, parts - such as, for example, in the case of plant organisms, plant tissue, for example leaves, roots and the like - or propagation material derived from such organisms, or intact plants. The terms "recombinant (host)", and "transgenic (host)" are used interchangeably in this context. Naturally, these terms refer not only to the host organism or target cell in question, but also to the progeny, or potential progeny, of these organisms or cells. Since certain modifications may occur in subsequent generations owing to mutation or environmental effects, such progeny is not necessarily identical with the parental cell, but still comes within the scope of the term as used herein.

Suitable organisms for the process according to the invention or as hosts are all these eukaryotic or prokaryotic organisms, which are capable of accumulating or assimilating nitrogen or nitrogen containing compounds respectively. The organisms used as hosts are microorganisms, such as bacteria, fungi, yeasts or algae, non-human animals, or plants, such as dictotyledonous or monocotyledonous plants.

In principle all plants can be used as host organism, especially the plants mentioned above as source organism. Preferred transgenic plants are, for example, selected from the families Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae or Poaceae and preferably from a plant selected from the group of the families Apiaceae, Asteraceae, Brassicaceae, Cucurbitaceae, Fabaceae, Papaveraceae, Rosaceae, Solanaceae, Liliaceae or Poaceae. Preferred are crop plants such as plants advantageously selected from the group of the genus peanut, oilseed rape, canola, sunflower, safflower, olive, sesame, hazelnut, almond, avocado, bay, pumpkin/squash, linseed, soya, pistachio, borage, maize, wheat, rye, oats, sorghum and millet, triticale, rice, barley, cassava, potato, sugarbeet, egg plant, alfalfa, and perennial grasses and forage plants, oil palm, vegetables (brassicas, root vegetables, tuber vegetables, pod vegetables, fruiting vegetables, onion vegetables, leafy vegetables and stem vegetables), buckwheat, Jerusalem artichoke, broad bean, vetches, lentil, dwarf bean, lupin, clover and Lucerne for mentioning only some of them.

Preferred plant cells, plant organs, plant tissues or parts of plants originate from the under source organism mentioned plant families, preferably from the abovementioned plant genus, more preferred from abovementioned plants species.

Transgenic plants comprising the nitrogen or nitrogen containing compounds, for example the amino acids or proteins synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. The nitrogen accumulated or assimilated in the process according to the invention may, however, also be isolated from the plant in the form of their free amino acids or bound in proteins. Amino acids produced by this process can be harvested by harvesting the organisms either from the culture in which they grow or from the field. This can be done via expressing, grinding and/or extraction, salt precipitation and/or ion-exchange chromatography of the plant parts, preferably the plant seeds, plant fruits, plant tubers and the like.

In yet another aspect, the invention also relates to harvestable parts and to propagation material of the transgenic plants according to the invention which either contain transgenic plant cells expressing a nucleic acid molecule according to the invention or which contains cells which show an increased cellular activity of the polypeptide of the invention or the polypeptide used in the method of the invention, e.g. an increased expression level or higher activity of the described protein.

Harvestable parts can be in principle any useful parts of a plant, for example, flowers, pollen, seedlings, tubers, leaves, stems, fruit, seeds, roots etc. Propagation material includes, for example, seeds, fruits, cuttings, seedlings, tubers, rootstocks etc. Preferred are seeds, fruits, seedlings or tubers as harvestable or propagation material.

The invention furthermore relates to the use of the transgenic organisms according to the invention and of the cells, cell cultures, parts - such as, for example, roots, leaves and the like as mentioned above in the case of transgenic plant organisms - derived from them, and to transgenic propagation material such as seeds or fruits and the like as mentioned above, for the production of foodstuffs or feeding stuffs, pharmaceuticals or fine chemicals, preferably feedstuffs.

Accordingly in another embodiment, the present invention relates to the use of the nucleic acid molecule, the organism, e.g. the microorganism, the plant, plant cell or plant tissue, the vector, or the polypeptide of the present invention for making fatty acids, carotenoids, isoprenoids, vitamins, lipids, wax esters, (poly)saccharides and/or polyhydroxyalkanoates, and/or its metabolism products, in particular, steroid hormones, cholesterol, prostaglandin, triacylglycerols, bile acids and/or ketone bodies producing cells, tissues and/or plants. There are a number of mechanisms by which the yield, production, and/or efficiency of production of fatty acids, carotenoids, isoprenoids, vitamins, wax esters, lipids, (poly)saccharides and/or polyhydroxyalkanoates, and/or its metabolism products, in particular, steroid hormones, cholesterol, triacylglycerols, prostaglandin, bile acids and/or ketone bodies or further of above defined fine chemicals incorporating such an altered protein can be affected. In the case of plants, by e.g. increasing the expression of acetyl-CoA which is the basis for many products, e.g., fatty acids, carotenoids, isoprenoids, vitamines, lipids, (poly)saccharides, wax esters, and/or polyhydroxyalkanoates, and/or its metabolism products, in particular, prostaglandin, steroid hormones, cholesterol, triacylglycerols, bile acids and/or ketone bodies in a cell, it may be possible to increase the amount of the produced said compounds thus permitting greater ease of harvesting and purification or in case of plants more efficient partitioning. Further, one or more of said metabolism products, increased amounts of the cofactors, precursor molecules, and intermediate compounds for the appropriate biosynthetic pathways maybe required. Therefore, by increasing the number and/or activity of transporter proteins involved in the import of nutrients, such as carbon sources (i.e., sugars), nitrogen sources (i.e., amino acids, ammonium salts), phosphate, and sulfur, it may be possible to improve the production of acetyl CoA and its metabolism products as mentioned above, due to the removal of any nutrient supply limitations on the biosynthetic process. In particular, it may be possible to increase the yield, production, and/or efficiency of production of said compounds, e.g. fatty acids, carotenoids, isoprenoids, vitamins, was esters, lipids, (poly)saccharides, and/or polyhydroxyalkanoates, and/or its metabolism products, in particular, steroid hormones, cholesterol, prostaglandin, triacylglycerols, bile acids and/or ketone bodies molecules etc. in plants.

The organisms, preferably plants of the present invention show enhanced nitrogen assimilation, accumulation and/or utilization even under conditions of limited nitrogen. In consequence the organims, preferably plants of the present invention show also enhanced growth, biomass and/or yield, preferably under conditions of limited nitrogen.

In one embodiment, the present invention relates to a method for the identification of a gene product conferring an enhanced nitrogen assimilation, accumulation and/or utilization in a organism, comprising the following steps:

contacting, e.g. hybridising, the nucleic acid molecules of a sample, e.g. cells, tissues, plants or microorganisms or a nucleic acid library , which can contain a candidate gene encoding a gene product conferring an enhanced nitrogen assimilation, accumulation and/or utilization after expression, with the nucleic acid molecule of the present invention;

identifying the nucleic acid molecules, which hybridize under relaxed stringent conditions with the nucleic acid molecule of the present invention in particular to a nucleic acid molecule sequence as indicated in Table I, columns 5 or 7, preferably in Table I B, columns 5 or 7and, optionally, isolating the full length cDNA clone or complete genomic clone;

introducing the candidate nucleic acid molecules in host cells, preferably in a plant cell or a microorganism, appropriate for enhanced nitrogen assimilation, accumulation and/or utilization;
**a)** expressing the identified nucleic acid molecules in the host cells;
**b)** assaying enhanced nitrogen assimilation, accumulation and/or utilization levels in the host cells; and
**c)** identifying the nucleic acid molecule and its gene product which expression confers an enhanced nitrogen assimilation, accumulation and/or utilization in the host cell after expression compared to the wild type.

Relaxed hybridisation conditions are: After standard hybridisation procedures washing steps can be performed at low to medium stringency conditions usually with washing conditions of 40°-55°C and salt conditions between 2xSSC and 0,2x SSC with 0,1% SDS in comparison to stringent washing conditions as e.g. 60°-68°C with 0,1% SDS. Further examples can be found in the references listed above for the stringent hybridization conditions. Usually washing steps are repeated with increasing stringency and length until a useful signal to noise ratio is detected and depend on many factors as the target, e.g. its purity, GC-content, size etc, the probe, e.g. its length, is it a RNA or a DNA probe, salt conditions, washing or hybridisation temperature, washing or hybridisation time etc.

In an other embodiment, the present invention relates to a method for the identification of a gene product conferring an enhanced nitrogen assimilation, accumulation and/or utilization in a organism, comprising the following steps:

identifying nucleic acid molecules of an organism; which can contain a candidate gene encoding a gene product conferring an enhanced nitrogen assimilation, accumulation and/or utilization after expression, which are at least 20%, preferably 25%, more preferably 30%, even more preferred are 35%. 40% or 50%, even more preferred are 60%, 70% or 80%, most preferred are 90% or 95% or more homology to the nucleic acid molecule of the present invention, for example via homology search in a data bank;

introducing the candidate nucleic acid molecules in host cells, preferably in a plant cells or microorganisms, appropriate enhancing nitrogen assimilation, accumulation and/or utilization;
**a)** expressing the identified nucleic acid molecules in the host cells;
**b)** assaying the enhanced nitrogen assimilation, accumulation and/or utilization level in the host cells; and
**c)** identifying the nucleic acid molecule and its gene product which expression confers an enhanced nitrogen assimilation, accumulation and/or utilization in the host cell after expression compared to the wild type.
   Eventually gene products conferring the increase in enhanced nitrogen assimilation, accumulation and/or utilization can also be identify according to a identical or similar 3D structure in step (a) and by the above described method.

The nucleic acid molecules identified can then be used for the production of or organisms with enhanced nitrogen assimilation, accumulation and/or utilization in the same way as the nucleic acid molecule of the present invention.

Accordingly, in one embodiment, the present invention relates to a process for the accumulation or production of nitrogen or nitrogen containing compounds respectively , comprising (a) identifying a nucleic acid molecule according to aforementioned steps (a) to (f) or (a) to (e) and recovering the free or bound nitrogen containing compounds, especially proteins, from a organism having an increased cellular activity of a polypeptide encoded by the isolated nucleic acid molecule compared to a wild type.

Furthermore, in one embodiment, the present invention relates to a method for the identification of a compound stimulating enhanced nitrogen assimilation, accumulation and/or utilization to said plant comprising:
**a)** contacting cells which express the polypeptide of the present invention or its mRNA with a candidate compound under cell cultivation conditions;
**b)** assaying an increase in expression of said polypeptide or said mRNA;
**c)** comparing the expression level to a standard response made in the absence of said candidate compound; whereby, an increased expression over the standard indicates that the compound is stimulating enhanced nitrogen assimilation, accumulation and/or utilization.

Furthermore, in one embodiment, the present invention relates to a method for the screening for agonists or an antagonist of the activity of the polypeptide of the present invention or used in the process of the present invention comprising:
**a)** contacting cells, tissues , plants or microorganisms which express the polypeptide according to the invention with a candidate compound or a sample comprising a plurality of compounds under conditions which permit the expression the polypeptide of the present invention or used in the process of the present invention;
**b)** assaying enhanced nitrogen assimilation, accumulation and/or utilization level or the polypeptide expression level in the cell, tissue, plant or microorganism or the media the cell, tissue, plant or microorganisms is cultured or maintained in; and
**c)** identifying a agonist or antagonist by comparing the measured nitrogen assimilation, accumulation and/or utilization level or polypeptide of the invention or used in the invention expression level with a standard nitrogen assimilation, accumulation and/or utilization or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.

Furthermore, in one embodiment, the present invention relates to process for the identification of a compound conferring enhanced nitrogen assimilation, accumulation and/or utilization in a plant or microorganism, comprising the steps:
**a)** culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
**b)** identifying if the compound is an effective agonist by detecting the presence or absence or increase of a signal produced by said readout system.

The screen for a gene product or an agonist conferring an increase in nitrogen or nitrogen containing compounds production can be performed by growth of an organism for example a microorganism in the presence of growth reducing amounts of an inhibitor of the accumulation or synthesis of nitrogen or nitrogen containing compounds respectively. Better growth, e.g. higher dividing rate or high dry mass in comparison to the control under such conditions would identify a gene or gene product or an agonist conferring an enhanced nitrogen assimilation, accumulation and/or utilization.

One can think to screen for enhanced nitrogen assimilation, accumulation and/or utilization by for example searching for a resistance to a drug blocking the synthesis of nitrogen or nitrogen containing compounds and looking whether this effect is dependent on the activity or expression of a polypeptide as indicated in Table II, columns 5 or 7 or a homolog thereof, e.g. comparing the phenotype of nearly identical organisms with low and high activity of a protein as indicated in Table II, columns 5 or 7after incubation with the drug.

Said compound may be chemically synthesized or microbiologically produced and/or comprised in, for example, samples, e.g., cell extracts from, e.g., plants, animals or microorganisms, e.g. pathogens. Furthermore, said compound(s) may be known in the art but hitherto not known to be capable of suppressing or activating the polypeptide of the present invention. The reaction mixture may be a cell free extract or may comprise a cell or tissue culture. Suitable set ups for the method of the invention are known to the person skilled in the art and are, for example, generally described in Alberts et al., Molecular Biology of the Cell, third edition (1994), in particular Chapter 17. The compounds may be, e.g., added to the reaction mixture, culture medium, injected into the cell or sprayed onto the plant.

If a sample containing a compound is identified in the method of the invention, then it is either possible to isolate the compound from the original sample identified as containing the compound capable of activating or increasing the content of nitrogen or nitrogen containing compounds in an organism or part thereof, or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical. Preferably, the compound identified according to the above described method or its derivative is further formulated in a form suitable for the application in plant breeding or plant cell and tissue culture.

The compounds which can be tested and identified according to a method of the invention may be expression libraries, e.g., cDNA expression libraries, peptides, proteins, nucleic acids, antibodies, small organic compounds, hormones, peptidomimetics, PNAs or the like (Milner, Nature Medicine 1 (1995), 879-880; Hupp, Cell 83 (1995), 237-245; Gibbs, Cell 79 (1994), 193-198 and references cited supra). Said compounds can also be functional derivatives or analogues of known inhibitors or activators. Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art. Furthermore, peptidomimetics and/or computer aided design of appropriate derivatives and analogues can be used, for example, according to the methods described above. The cell or tissue that may be employed in the method of the invention preferably is a host cell, plant cell or plant tissue of the invention described in the embodiments hereinbefore.

Thus, in a further embodiment the invention relates to a compound obtained or identified according to the method for identifying an agonist of the invention said compound being an agonist of the polypeptide of the present invention or used in the process of the present invention.

Accordingly, in one embodiment, the present invention further relates to a compound identified by the method for identifying a compound of the present invention.

Said compound is, for example, a homologous of the polypeptide of the present invention. Homologues of the polypeptid of the present invention can be generated by mutagenesis, e.g., discrete point mutation or truncation of the polypeptide of the present invention. As used herein, the term "homologue" refers to a variant form of the protein, which acts as an agonist of the activity of the polypeptide of the present invention. An agonist of said protein can retain substantially the same, or a subset, of the biological activities of the polypeptide of the present invention. In particular, said agonist confers the increase of the expression level of the polypeptide of the present invention and/or the expression of said agonist in an organisms or part thereof confers the enhanced nitrogen assimilation, accumulation and/or utilization in the organism or part thereof.

In one embodiment, the invention relates to an antibody specifically recognizing the compound or agonist of the present invention.

The invention also relates to a diagnostic composition comprising at least one of the aforementioned nucleic acid molecules, vectors, proteins, antibodies or compounds of the invention and optionally suitable means for detection.

The diagnostic composition of the present invention is suitable for the isolation of mRNA from a cell and contacting the mRNA so obtained with a probe comprising a nucleic acid probe as described above under hybridizing conditions, detecting the presence of mRNA hybridized to the probe, and thereby detecting the expression of the protein in the cell. Further methods of detecting the presence of a protein according to the present invention comprise immunotechniques well known in the art, for example enzyme linked immunosorbent assay. Furthermore, it is possible to use the nucleic acid molecules according to the invention as molecular markers or primer in plant breeding. Suitable means for detection are well known to a person skilled in the arm, e.g. buffers and solutions for hydridization assays, e.g. the aforementioned solutions and buffers, further and means for Southern-, Western-, Northern- etc. -blots, as e.g. described in Sambrook et al. are known.

In another embodiment, the present invention relates to a kit comprising the nucleic acid molecule, the vector, the host cell, the polypeptide, the antisense nucleic acid, the antibody, plant cell, the plant or plant tissue, the harvestable part, the propagation material and/or the compound or agonist or antagonists identified according to the method of the invention.

The compounds of the kit of the present invention may be packaged in containers such as vials, optionally with/in buffers and/or solution. If appropriate, one or more of said components might be packaged in one and the same container. Additionally or alternatively, one or more of said components might be adsorbed to a solid support as, e.g. a nitrocellulose filter, a glass plate, a chip, or a nylon membrane or to the well of a micro titerplate. The kit can be used for any of the herein described methods and embodiments, e.g. for the production of the host cells, transgenic plants, pharmaceutical compositions, detection of homologous sequences, identification of antagonists or agonists, as food or feed or as a supplement thereof, as supplement for the treating of plants, etc.

Further, the kit can comprise instructions for the use of the kit for any of said embodiments, in particular for the use for producing organisms or part thereof having enhanced nitrogen assimilation, accumulation and/or utilization.

In one embodiment said kit comprises further a nucleic acid molecule encoding one or more of the aforementioned protein, and/or an antibody, a vector, a host cell, an antisense nucleic acid, a plant cell or plant tissue or a plant.

In a further embodiment, the present invention relates to a method for the production of a agricultural composition providing the nucleic acid molecule, the vector or the polypeptide of the invention or the polypeptide used in the method of the invention or comprising the steps of the method according to the invention for the identification of said compound, agonist or antagonist; and formulating the nucleic acid molecule, the vector or the polypeptide of the invention or the polypeptide used in the method of the invention or the agonist, or compound identified according to the methods or processes of the present invention or with use of the subject matters of the present invention in a form applicable as plant agricultural composition.

In another embodiment, the present invention relates to a method for the production of a "nitrogen containing compounds "-production supporting plant culture composition comprising the steps of the method for of the present invention; and formulating the compound identified in a form acceptable as agricultural composition.

Under "acceptable as agricultural composition" is understood, that such a composition is in agreement with the laws regulating the content of fungicides, plant nutrients, herbicides, etc. Preferably such a composition is without any harm for the protected plants and the animals (humans included) fed therewith.

The present invention also pertains to several embodiments relating to further uses and methods. The nucleic acid molecule, polypeptide, protein homologues, fusion proteins, primers, vectors, host cells, described herein can be used in one or more of the following methods: identification of plants useful for nitrogen or nitrogen containing compounds production as mentioned and related organisms; mapping of genomes; identification and localization of sequences of interest; evolutionary studies; determination of regions required for function; modulation of an activity.

The nucleic acid molecules of the present invention have a variety of uses. First, they may be used to identify an organism or a close relative thereof. Also, they may be used to identify the presence thereof or a relative thereof in a mixed population of microorganisms or plants. By probing the extracted genomic DNA of a culture of a unique or mixed population of plants under stringent conditions with a probe spanning a region of the gene of the present invention which is unique to this, one can ascertain whether the present invention has been used or whether it or a close relative is present.

Further, the nucleic acid molecule of the invention or the nucleic acid molecule used in the method of the invention may be sufficiently homologous to the sequences of related species such that these nucleic acid molecules may serve as markers for the construction of a genomic map in related organism.

Accordingly, the present invention relates to a method for breeding plants with enhanced nitrogen assimilation, accumulation and/or utilization, comprising
(a providing a first plant variety produced according to the process of the invention preferably (over)expressing the nucleic acid molecule of the invention or the nucleic acid molecule used in the method of the invention;
(b crossing the first plant variety with a second plant variety; and
   selecting the offspring plants which overaccumulates nitrogen or overproduce nitrogen containing compounds by means of analysis the distribution of a molecular marker in the offspring representing the first plant variety and its capability to (over)accumulate nitrogen or (over)produce nitrogen containing compounds.

Further, the nucleic acid molecule disclosed herein, in particular the nucleic acid molecule shown column 5 or 7 of Table I A or B, may be sufficiently homologous to the sequences of related species such that these nucleic acid molecules may serve as markers for the construction of a genomic map in related organism or for association mapping. Furthermore natural variation in the genomic regions corresponding to nucleic acids disclosed herein, in particular the nucleic acid molecule shown column 5 or 7 of Table I A or B, or homologous thereof may lead to variation in the activity of the proteins disclosed herein, in particular the proteins comprising polypeptides as shown in column 5 or 7 of Table II A or B or comprising the consensus sequence or the polypeptide motif as shown in column 7 of Table IV, and their homolgous and in consequence in natural variation in the production of nitrogen or nitrogen containing compounds. In consequnce natural variation eventually also exists in form of more active allelic variants leading already to a relative increase in the production of nitrogen or nitrogen containing compounds. Different variants of the nucleic acids molecule disclosed herein, in particular the nucleic acid comprising the nucleic acid molecule as shown column 5 or 7 of Table I A or B, which corresponds to different production levels of nitrogen or nitrogen containing compounds can be indentified and used for marker assisted breeding for increase production of nitrogen or nitrogen containing compounds.

Accordingly, the present invention relates to a method for breeding plants for increased production of nitrogen or nitrogen containing compounds, comprising
a) selecting a first plant variety with increased production of nitrogen or nitrogen containing compounds based on increased expression of a nucleic acid of the invention as disclosed herein, in particular of a nucleic acid molecule comprising a nucleic acid molecule as shown in column 5 or 7 of Table I A or B or a polypeptide comprising a polypeptide as shown in column 5 or 7 of Table II A or B or comprising a consensus sequence or a polypeptide motif as shown in column 7 of Table IV, or a homologue thereof as described herein;
b) associating the production level of nitrogen or nitrogen containing compounds with the expression level or the genomic structure of a gene encoding said polypeptide or said nucleic acid molecule;
c) crossing the first plant variety with a second plant variety, which significantly differs in its production level of nitrogen or nitrogen containing compounds and
d)identifying, which of the offspring varieties has got increased production level for nitrogen or nitrogen containing compounds by the expression level of said polypeptide or nucleic acid molecule or the genomic structure of the genes encoding said polypeptide or nucleic acid molecule of the invention.
In one embodiment, the expression level of the gene according to step (b) is increased

Details about the use of molecular markers in breeding can be found in Kumar et al., 1999 (Biotech Adv., 17:143-182) and Peleman and van der Voort 2003 (Trends Plant Sci. 2003 Jul;8(7):330-334)
The molecular marker can e.g. relate to the nucleic acid molecule of the invention or the nucleic acid molecule used in the method of the invention and/or its expression level. Accordingly, the molecular marker can be a probe or a PCR primer set useful for identification of the genomic existence or genomic localisation of the nucleic acid molecule of the invention or the nucleic acid molecule used in the method of the invention, e.g. in a Southern blot analysis or a PCR or its expression level, i.g. in a Northern Blot analysis or a quantitative PCR.
Accordingly, in one embodiment, the present invention relates to the use of the nucleic acid molecule of the present invention or encoding the polypeptide of the present invention as molecular marker for breeding, especially for breeding for a high or low respective fine chemical production.

The nucleic acid molecules of the invention are also useful for evolutionary and protein structural studies. By comparing the sequences of the invention or used in the process of the invention to those encoding similar enzymes from other organisms, the evolutionary relatedness of the organisms can be assessed. Similarly, such a comparison permits an assessment of which regions of the sequence are conserved and which are not, which may aid in determining those regions of the protein which are essential for the functioning of the enzyme. This type of determination is of value for protein engineering studies and may give an indication of what the protein can tolerate in terms of mutagenesis without losing function.

Accordingly, the nucleic acid molecule of the invention or the nucleic acid molecule used in the method of the invention can be used for the identification of other nucleic acids conferring an increase of nitrogen or nitrogen containing compounds after expression.

Further, the nucleic acid molecule of the invention or the nucleic acid molecule used in the method of the invention or a fragment of a gene conferring the expression of the polypeptide of the invention or the polypeptide used in the method of the invention, preferably comprising the nucleic acid molecule of the invention, can be used for marker assisted breeding or association mapping of nitrogen or nitrogen containing compounds derived traits.

Accordingly, the nucleic acid of the invention, the polypeptide of the invention or the polypeptide used in the method of the invention, the nucleic acid construct of the invention, the organisms, the host cell, the microorganisms, the plant, plant tissue, plant cell, or the part thereof of the invention, the vector of the invention, the agonist identified with the method of the invention, the nucleic acid molecule identified with the method of the present invention, can be used for the production of N-containing compound or of the N-containing compound and one or more other fine chemicals.
Accordingly, the nucleic acid of the invention, or the nucleic acid molecule identified with the method of the present invention or the complement sequences thereof, the polypeptide of the invention or the polypeptide used in the method of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reductionof nitrogen or nitrogen containing compounds in a organism or part thereof, e.g. in a cell.

Further, the nucleic acid of the invention, the polypeptide of the invention or the polypeptide used in the method of the invention, the nucleic acid construct of the invention, the organisms, the host cell, the microorganisms, the plant, plant tissue, plant cell, or the part thereof of the invention, the vector of the invention, the antagonist or the agonist identified with the method of the invention, the antibody of the present invention, the antisense molecule of the present invention or the nucleic acid molecule identified with the method of the present invention, can be used for the preparation of an agricultural composition.

Furthermore, the nucleic acid of the invention, the polypeptide of the invention or the polypeptide used in the method of the invention, the nucleic acid construct of the invention, the organisms, the host cell, the microorganisms, the plant, plant tissue, plant cell, or the part thereof of the invention, the vector of the invention, antagonist or the agonist identified with the method of the invention, the antibody of the present invention, the antisense molecule of the present invention or the nucleic acid molecule identified with the method of the present invention, can be used for the identification and production of compounds capable of conferring a modulation of nitrogen or nitrogen containing compounds levels in an organism or parts thereof, preferably to identify and produce compounds conferring an increase of nitrogen or nitrogen containing compounds levels in an organism or parts thereof, if said identified compound is applied to the organism or part thereof, i.e. as part of its food, or in the growing or culture media.

These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the Internet, for example under hftp://www.ncbi.nlm.nih.gov/PubMed/medline.html. Further databases and addresses, such as hftp://www.ncbi.nlm.nih.gov/, hftp://www.infobiogen.fr/, hftp://www.fmi.ch/biology/research-tools.html, hftp://www.tigr.org/, are known to the person skilled in the art and can also be obtained using, e.g., hftp://www.lycos.com. An overview of patent information in biotechnology and a survey of relevant sources of patent information useful for retrospective searching and for current awareness is given in Berks, TIBTECH 12 (1994), 352-364.

The present invention is illustrated by the examples, which follow. The present examples illustrate the basic invention without being intended as limiting the subject of the invention. The content of all of the references, patent applications, patents and published patent applications cited in the present patent application is herewith incorporated by reference.

Example 1: Cloning SEQ ID NO: 689 from Saccharomyces cerevisiae for the expression in plants

Unless otherwise specified, standard methods as described in Sambrook et al., Molecular Cloning: A laboratory manual, Cold Spring Harbor 1989, Cold Spring Harbor Laboratory Press are used.

SEQ ID NO: 689 is amplified by PCR as described in the protocol of the Pfu Turbo or DNA Herculase polymerase (Stratagene).

The composition for the protocol of the Pfu Turbo DNA polymerase was as follows: 1x PCR buffer (Stratagene), 0.2 mM of each dNTP, 100 ng genomic DNA of Saccharomyces cerevisiae (strain S288C; Research Genetics, Inc., now Invitrogen), 50 pmol forward primer, 50 pmol reverse primer, 2.5 u Pfu Turbo DNA polymerase. The amplification cycles were as follows:

1 cycle of 3 minutes at 94-95°C, followed by 25-36 cycles of in each case 1 minute at 95°C or 30 seconds at 94°C, 45 seconds at 50°C, 30 seconds at 50°C or 30 seconds at 55°C and 210-480 seconds at 72°C, followed by 1 cycle of 8 minutes at 72°C, then 4°C. The composition for the protocol of the Herculase polymerase was as follows: 1x PCR buffer (Stratagene), 0.2 mM of each dNTP, 100 ng genomic DNA of Saccharomyces cerevisiae (strain S288C; Research Genetics, Inc., now Invitrogen), 50 pmol forward primer, 50 pmol reverse primer, 2.5 u Herculase polymerase. The amplification cycles were as follows:

1 cycle of 2-3 minutes at 94°C, followed by 25-30 cycles of in each case 30 seconds at 94°C, 30 seconds at 55-60°C and 5-10 minutes at 72°C, followed by 1 cycle of 10 minutes at 72°C, then 4°C.

The following primer sequences were selected for the gene SEQ ID NO: 689
i) forward primer (SEQ ID NO: 949)
   atggctcggg gtgacggaca t
ii) reverse primer (SEQ ID NO: 950)
   tcatgcttct tttgcgtgat gcaat

Thereafter, the amplificate was purified over QIAquick columns following the standard protocol (Qiagen).

For the cloning of PCR-products, produced by Pfu Turbo DNA polymerase, the vector DNA (30 ng) was restricted with Smal following the standard protocol (MBI Fermentas) and stopped by addition of high-salt buffer. The restricted vector fragments were purified via Nucleobond columns using the standard protocol (Macherey-Nagel). Thereafter, the linearized vector was dephosphorylated following the standard protocol (MBI Fermentas).

The PCR-products, produced by Pfu Turbo DNA polymerase, were phosphorylated using a T4 DNA polymerase using a standard protocol (e.g. MBI Fermentas) and cloned into the processed binary vector.

The DNA termini of the PCR-products, produced by Herculase DNA polymerase, were blunted in a second synthesis reaction using Pfu Turbo DNA polymerase. The composition for the protocol of the blunting the DNA-termini was as follows: 0.2 mM blunting dTTP and 1.25 u Pfu Turbo DNA polymerase. The reaction was incubated at 72°C for 30 minutes. Then the PCR-products were phosphorylated using a T4 DNA polymerase using a standard protocol (e.g. MBI Fermentas) and cloned into the processed vector as well.

A binary vector comprising a selection cassette (promoter, selection marker, terminator) and an expression cassette with promoter, cloning cassette and terminator sequence between the T-DNA border sequences was used. In addition to those within the cloning cassette, the binary vector has no Smal cleavage site. Binary vectors which can be used are known to the skilled worker; an overview of binary vectors and their use can be found in Hellens, R., Mullineaux, P. and Klee H., [(2000) "A guide to Agrobacterium binary vectors", Trends in Plant Science, Vol. 5 No.10, 446-451. Depending on the vector used, cloning may advantageously also be carried out via other restriction enzymes. Suitable advantageous cleavage sites can be added to the ORF by using suitable primers for the PCR amplification.

Approximately 30 ng of prepared vector and a defined amount of prepared amplificate were mixed and ligated by addition of ligase.

The ligated vectors were transformed in the same reaction vessel by addition of competent E. coli cells (strain DH5alpha) and incubation for 20 minutes at 1°C followed by a heat shock for 90 seconds at 42°C and cooling to 4°C. Then, complete medium (SOC) was added and the mixture was incubated for 45 minutes at 37°C. The entire mixture was subsequently plated onto an agar plate with antibiotics (selected as a function of the binary vector used) and incubated overnight at 37°C.

The outcome of the cloning step was verified by amplification with the aid of primers which bind upstream and downstream of the integration site, thus allowing the amplification of the insertion. In addition combinations of the above mentioned gene specific primers and upstream and downstream primers were used in PCR reactions to identify clones with the correct insert orientation. The amplifications were carried as described in the protocol of Taq DNA polymerase (Gibco-BRL).

The amplification cycles were as follows: 1 cycle of 5 minutes at 94°C, followed by 35 cycles of in each case 15 seconds at 94°C, 15 seconds at 50-66°C and 5 minutes at 72°C, followed by 1 cycle of 10 minutes at 72°C, then 4°C.

Several colonies were checked, but only one colony for which a PCR product of the expected size was detected was used in the following steps.

A portion of this positive colony was transferred into a reaction vessel filled with complete medium (LB) and incubated overnight at 37°C. The LB medium contained an antibiotic chosen to suit the binary vector (see above) used and the resistance gene present therein in order to select the clone.

The plasmid preparation was carried out as specified in the Qiaprep standard protocol (Qiagen).

Example 2: Generation of transgenic plants which express SEQ ID NO: 689

1 ng of the plasmid DNA isolated was transformed by electroporation into competent cells of Agrobacterium tumefaciens, of strain GV 3101 pMP90 (Koncz and Schell, Mol. Gen. Gent. 204, 383-396, 1986) . The choice of the agrobacterial strain depends on the choice of the binary vector. An overview of possible strains and their properties is found in Hellens, R., Mullineaux, P. and Klee H., (2000) " A guide to Agrobacterium binary vectors, Trends in Plant Science, Vol. 5 No.10, 446-451. Thereafter, complete medium (YEP) was added and the mixture was transferred into a fresh reaction vessel for 3 hours at 28°C. Thereafter, all of the reaction mixture was plated onto YEP agar plates supplemented with the respective antibiotics, for example rifampicin and gentamycin for GV3101 pMP90, and a further antibiotic for the selection onto the binary vector, was plated, and incubated for 48 hours at 28°C.

The agrobacteria generated in Example 2, which contains the plasmid construct were then used for the transformation of plants.

A colony was picked from the agar plate with the aid of a pipette tip and taken up in 3 ml of liquid TB medium, which also contained suitable antibiotics, depending on the agrobacterial strain and the binary plasmid. The preculture was grown for 48 hours at 28°C and 120 rpm.

400 ml of LB medium containing the same antibiotics as above were used for the main culture. The preculture was transferred into the main culture. It was grown for 18 hours at 28°C and 120 rpm. After centrifugation at 4 000 rpm, the pellet was resuspended in infiltration medium (MS medium, 10% sucrose).

In order to grow the plants for the transformation, dishes (Piki Saat 80, green, provided with a screen bottom, 30 x 20 x 4.5 cm, from Wiesauplast, Kunststofftechnik, Germany) were half-filled with a GS 90 substrate (standard soil, Werkverband E.V., Germany). The dishes were watered overnight with 0.05% Proplant solution (Chimac-Apriphar, Belgium). Arabidopsis thaliana C24 seeds (Nottingham Arabidopsis Stock Centre, UK ; NASC Stock N906) were scattered over the dish, approximately 1 000 seeds per dish. The dishes were covered with a hood and placed in the stratification facility (8 h, 110 µmol/m2/s-1, 22°C; 16 h, dark, 6°C). After 5 days, the dishes were placed into the short-day controlled environment chamber (8 h 130 µmol/m2/s-1, 22°C; 16 h, dark 20°C), where they remained for approximately 10 days until the first true leaves had formed.

The seedlings were transferred into pots containing the same substrate (Teku pots, 7 cm, LC series, manufactured by Pöppelmann GmbH & Co, Germany). Five plants were pricked out into each pot. The pots were then returned into the short-day controlled environment chamber for the plant to continue growing.

After 10 days, the plants were transferred into the greenhouse cabinet (supplementary illumination, 16 h, 340 µE, 22°C; 8 h, dark, 20°C), where they were allowed to grow for further 17 days.

For the transformation, 6-week-old Arabidopsis plants which had just started flowering were immersed for 10 seconds into the above-described agrobacterial suspension which had previously been treated with 10µl Silwett L77 (Crompton S.A., Osi Specialties, Switzerland). The method in question is described in Clough and Bent, 1998 (Clough, JC and Bent, AF. 1998 Floral dip: a simplified method for Agrobacterium-mediated transformation of Arabidopsis thaliana, Plant J. 16:735-743.

The plants were subsequently placed for 18 hours into a humid chamber. Thereafter, the pots were returned to the greenhouse for the plants to continue growing. The plants remained in the greenhouse for another 10 weeks until the seeds were ready for harvesting.

Depending on the resistance marker used for the selection of the transformed plants the harvested seeds were planted in the greenhouse and subjected to a spray selection or else first sterilized and then grown on agar plates supplemented with the respective selection agent. In case of BASTA®-resistance, plantlets were sprayed four times at an interval of 2 to 3 days with 0.02 % BASTA® and transformed plants were allowed to set seeds. The seeds of the transgenic A. thaliana plants were stored in the freezer (at -20°C).

Eample 3: Nitrogen Content Analysis

The determination of nitrogen in the samples is performed using the Dumas method which relies on the complete combustion of the test material. The sample is heated in a high temperature furnace and rapidly combusted in the presence of pure oxygen. The combustion products (mainly CO2, H2O, NOx, and N2) are collected and allowed to equilibrate. An aliquot of the gas mixture is passed over hot copper to remove any oxygen and convert NO2 to N2. The sample is then passed through a trap that removes CO2 and H2O. The remaining nitrogen is measured by a thermal conductivity detector.

For the analysis of leaf material or for seed kernels, homogenized freeze-dryed material is used. In the case of Arabidopsis seeds, the seeds are analyzed directly without pretreatment.

4 - 7 mg of the sample were weighed into a tin foil cup together with 15 mg of tungsten(VI)-oxide (WO3). Analysis was performed using a commercial elementar analyzer (e.g. ELEMENTAR vario EL III, ELEMENTAR, Hanau, Germany).

Table VI shows the increased total nitrogen content of seeds of transgenic plants transformed with the yeast ORF YPR138c, corresponding to SeqID NO: 689 under control of the double 35S promotor. Column 1 shows the measured elements, column 2 shows the wild type variability as relative standard deviation, column 3 shows the mean change in the element content for different transgenic lines transformed with SEQ ID NO: 689 relative to the wildtype control which is standardized as "1", column 4 shows the standard deviation for the different transgenic lines and column 5 shows the maximal observed change. As expected, the relative increase in nitrogen corresponds to a relative decrease in carbon content.

| Parameter | WT variability (RSD; %) | Mean | SD | Max. Change |
|---|---|---|---|---|
| %N | 0.05 | 1.17 | 0.06 | 1.24 |
| %C | 0.01 | 0.90 | 0.03 | 0.87 |

Example 4: Enhanced nitrogen use efficiency

### Plant screening (Arabidopsis) for growth under limited nitrogen supply

For screening of transgenic plants a specific culture facility was used. For high-throughput purposes plants were screened for biomass production on agar plates with limited supply of nitrogen (adapted from Estelle and Somerville, 1987). The screening pipeline consists of three level. Transgenic lines are subjected to subsequent level if biomass production was significantly improved in comparison to wild type plants. With each level number of replicates and statistical stringency was increased.
For the sowing, the seeds, which had been stored in the refrigerator (at -20°C), were removed from the Eppendorf tubes with the aid of a toothpick and transferred onto plates. In total, approximately 15-30 seeds were distributed horizontally on each plate (12 x 12 cm).
After the seeds had been sown, plates are subjected to stratification for 2-4 days in the dark at 4°C. After the stratification, the test plants were grown for 22 to 25 days at a 16-h-light, 8-h-dark rhythm at 20°C, an atmospheric humidity of 60% and a CO2 concentration of approximately 400 ppm. The light sources used generate a light resembling the solar color spectrum with a light intensity of approximately 100 µE/m2/s-1.
Improved growth under nitrogen limited conditions was assessed by biomass production of shoots and roots of transgenic plants in comparison to wild type control plants after 20-25 days growth.
Transgenic lines expressing the yeast ORF YPR138c, corresponding to SEQ ID NO: : 689 under control of a strong constitutive promotor like the double 35S promotor, showed enhanced biomass production of shoots and roots under nitrogen limited conditions in comparison to wilde type control plants.

Example 5: Engineering ryegrass plants by over-expressing the polynucleotide characterized in the invention, e.g. derived from Saccharomyces, E.coli or an other organism

Seeds of several different ryegrass varieties can be used as explant sources for transformation, including the commercial variety Gunne available from Svalof Weibull seed company or the variety Affinity. Seeds are surface-sterilized sequentially with 1% Tween-20 for 1 minute, 100 % bleach for 60 minutes, 3 rinses with 5 minutes each with de-ionized and distilled H2O, and then germinated for 3-4 days on moist, sterile filter paper in the dark. Seedlings are further sterilized for 1 minute with 1% Tween-20, 5 minutes with 75% bleach, and rinsed 3 times with ddH2O, 5 min each.

Surface-sterilized seeds are placed on the callus induction medium containing Murashige and Skoog basal salts and vitamins, 20 g/l sucrose, 150 mg/l asparagine, 500 mg/l casein hydrolysate, 3 g/l Phytagel, 10 mg/l BAP, and 5 mg/l dicamba. Plates are incubated in the dark at 25°C for 4 weeks for seed germination and embryogenic callus induction.

After 4 weeks on the callus induction medium, the shoots and roots of the seedlings are trimmed away, the callus is transferred to fresh media, is maintained in culture for another 4 weeks, and is then transferred to MSO medium in light for 2 weeks. Several pieces of callus (11-17 weeks old) are either strained through a 10 mesh sieve and put onto callus induction medium, or are cultured in 100 ml of liquid ryegrass callus induction media (same medium as for callus induction with agar) in a 250 ml flask. The flask is wrapped in foil and shaken at 175 rpm in the dark at 23°C for 1 week. Sieving the liquid culture with a 40-mesh sieve is collected the cells. The fraction collected on the sieve is plated and is cultured on solid ryegrass callus induction medium for 1 week in the dark at 25°C. The callus is then transferred to and is cultured on MS medium containing 1% sucrose for 2 weeks.

Transformation can be accomplished with either Agrobacterium or with particle bombardment methods. An expression vector is created containing a constitutive plant promoter and the cDNA of the gene in a pUC vector. The plasmid DNA is prepared from E. coli cells using with Qiagen kit according to manufacturer's instruction. Approximately 2 g of embryogenic callus is spread in the center of a sterile filter paper in a Petri dish. An aliquot of liquid MSO with 10 g/l sucrose is added to the filter paper. Gold particles (1.0 µm in size) are coated with plasmid DNA according to method of Sanford et al., 1993 and are delivered to the embryogenic callus with the following parameters: 500 µg particles and 2 µg DNA per shot, 1300 psi and a target distance of 8.5 cm from stopping plate to plate of callus and 1 shot per plate of callus.

After the bombardment, calli are transferred back to the fresh callus development medium and maintained in the dark at room temperature for a 1-week period. The callus is then transferred to growth conditions in the light at 25 °C to initiate embryo differentiation with the appropriate selection agent, e.g. 250 nM Arsenal, 5 mg/l PPT or 50 mg/L Kanamycin. Shoots resistant to the selection agent are appearing and once rooted are transferred to soil.

Samples of the primary transgenic plants (T0) are analyzed by PCR to confirm the presence of T-DNA. These results are confirmed by Southern hybridization in which DNA is electrophoresed on a 1% agarose gel and transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.

Transgenic T0 ryegrass plants are propagated vegetatively by excising tillers. The transplanted tillers are maintained in the greenhouse for 2 months until well established. The shoots are defoliated and allowed to grow for 2 weeks.

Seeds of transgenic ryegrass can be analyzed for increased nitrogen content with the elementar analyzer as described under example 3.

Example 6: Engineering soybean plants by over-expressing the polynucleotide characterized in the invention, e.g. derived from Saccharomyces, E. coli or another organism

Soybean can be transformed according to the following modification of the method described in the Texas A&M patent US 5,164,310. Several commercial soybean varieties are amenable to transformation by this method. The cultivar Jack (available from the Illinois Seed Foundation) is commonly used for transformation. Seeds are sterilized by immersion in 70% (v/v) ethanol for 6 min and in 25 % commercial bleach (NaOCl) supplemented with 0.1 % (v/v) Tween for 20 min, followed by rinsing 4 times with sterile double distilled water. Removing the radicle, hypocotyl and one cotyledon from each seedling propagates seven-day seedlings. Then, the epicotyl with one cotyledon is transferred to fresh germination media in petri dishes and incubated at 25 °C under a 16-hr photoperiod (approx. 100 µE-m-2s-1) for three weeks. Axillary nodes (approx. 4 mm in length) are cut from 3 - 4 week-old plants. Axillary nodes are excised and incubated in Agrobacterium LBA4404 culture.

Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used as described above, including the Arabidopsis gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription as described above. In this example, the 34S promoter (GenBank Accession numbers M59930 and X16673) is used to provide constitutive expression of the trait gene.

After the co-cultivation treatment, the explants are washed and transferred to selection media supplemented with 500 mg/L timentin. Shoots are excised and placed on a shoot elongation medium. Shoots longer than 1 cm are placed on rooting medium for two to four weeks prior to transplanting to soil.

The primary transgenic plants (T0) are analyzed by PCR to confirm the presence of T-DNA. These results are confirmed by Southern hybridization in which DNA is electrophoresed on a 1 % agarose gel and transferred to a positively charged nylon membrane (Roche Diagnostics). The PCR DIG Probe Synthesis Kit (Roche Diagnostics) is used to prepare a digoxigenin-labelled probe by PCR, and is used as recommended by the manufacturer.

Seeds of transgenic soybean can be analyzed for increased nitrogen content with the elementar analyzer as described under example 3.

Example 7 Engineering corn plants by over-expressing the polynucleotide characterized in the invention, e.g. derived from Saccharomyces, E.coli or another organism

Transformation of maize (Zea Mays L.) can be performed with a modification of the method described by Ishida et al. (1996. Nature Biotech 14745-50). Transformation is genotype-dependent in corn and only specific genotypes are amenable to transformation and regeneration. The inbred line A188 (University of Minnesota) or hybrids with A188 as a parent are good sources of donor material for transformation (Fromm et al. 1990 Biotech 8:833-839), but other genotypes can be used successfully as well. Ears are harvested from corn plants at approximately 11 days after pollination (DAP) when the length of immature embryos is about 1 to 1.2 mm. Immature embryos are co-cultivated with Agrobacterium tumefaciens that carry "super binary" vectors and transgenic plants are recovered through organogenesis. The super binary vector system of Japan Tobacco is described in WO patents WO94/00977 and WO95/06722. Vectors can be constructed as described. Various selection marker genes can be used including the maize gene encoding a mutated acetohydroxy acid synthase (AHAS) enzyme (US patent 6025541). Similarly, various promoters can be used to regulate the trait gene to provide constitutive, developmental, tissue or environmental regulation of gene transcription. In this example, the 34S promoter (GenBank Accession numbers M59930 and X16673 can be used to provide constitutive expression of the trait gene.

Excised embryos can be grown on callus induction medium, then maize regeneration medium, containing imidazolinone as a selection agent. The Petri plates can be incubated in the light at 25 °C for 2-3 weeks, or until shoots develop. The green shoots can be transferred from each embryo to maize rooting medium and incubated at 25 °C for 2-3 weeks, until roots develop. The rooted shoots can be transplanted to soil in the greenhouse. T1 seeds can be produced from plants that exhibit tolerance to the imidazolinone herbicides and which can be PCR positive for the transgenes.

The T1 generation of single locus insertions of the T-DNA can segregate for the transgene in a 3:1 ratio. Those progeny containing one or two copies of the transgene can be tolerant of the imidazolinone herbicide. Seeds of transgenic corn can be analyzed for increased nitrogen content with the elementar analyzer as described under example 3.

Example 8: Engineering wheat plants by over-expressing the polynucleotide characterized in the invention, e.g. derived from Saccharomyces, E.coli or another organism

Transformation of wheat can be performed with the method described by Ishida et al. (1996 Nature Biotech. 14745-50). The cultivar Bobwhite (available from CYMMIT, Mexico) can commonly be used in transformation. Immature embryos can be co-cultivated with Agrobacterium tumefaciens that carry "super binary" vectors, and transgenic plants are recovered through organogenesis. The super binary vector system of Japan Tobacco is described in WO patents WO94/00977 and WO95/06722. Vectors can be constructed as described. Various selection marker genes can be used including the maize gene encoding a mutated acetohydroxy acid synthase (AHAS) enzyme (US patent 6025541). Similarly, various promoters can be used to regulate the trait gene to provide constitutive, developmental, tissue or environmental regulation of gene transcription. The 34S promoter (GenBank Accession numbers M59930 and X16673) can be used to provide constitutive expression of the trait gene.

After incubation with Agrobacterium, the embryos can be grown on callus induction medium, then regeneration medium, containing imidazolinone as a selection agent. The Petri plates can be incubated in the light at 25 °C for 2-3 weeks, or until shoots develop. The green shoots can be transferred from each embryo to rooting medium and incubated at 25 °C for 2-3 weeks, until roots develop. The rooted shoots can be transplanted to soil in the greenhouse. T1 seeds can be produced from plants that exhibit tolerance to the imidazolinone herbicides and which are PCR positive for the transgenes.

The T1 generation of single locus insertions of the T-DNA can segregate for the transgene in a 3:1 ratio. Those progeny containing one or two copies of the transgene can be tolerant of the imidazolinone herbicide. Homozygous T2 plants exhibited similar phenotypes. Seeds of transgenic wheat can be analyzed for increased nitrogen content with the elementar analyzer as described under example 3.

Example 9: Engineering Rapeseed/Canola plants by over-expressing the polynucleotide characterized in the invention, e.g. derived from Saccharomyces cerevisiae, E.coli or another organism

Cotyledonary petioles and hypocotyls of 5-6 day-old young seedlings can be used as explants for tissue culture and transformed according to Babic et al.(1998, Plant Cell Rep 17: 183-188). The commercial cultivar Westar (Agriculture Canada) can be the standard variety used for transformation, but other varieties can be used.

Agrobacterium tumefaciens LBA4404 containing a binary vector can be used for canola transformation. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette can consist of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription. The 34S promoter (GenBank Accession numbers M59930 and X16673) can be used to provide constitutive expression of the trait gene.

Canola seeds can be surface-sterilized in 70% ethanol for 2 min., and then in 30% Clorox with a drop of Tween-20 for 10 min, followed by three rinses with sterilized distilled water. Seeds can be then germinated in vitro 5 days on half strength MS medium without hormones, 1% sucrose, 0.7% Phytagar at 23oC, 16 hr. light. The cotyledon petiole explants with the cotyledon attached can be excised from the in vitro seedlings, and can be inoculated with Agrobacterium by dipping the cut end of the petiole explant into the bacterial suspension. The explants can be then cultured for 2 days on MSBAP-3 medium containing 3 mg/l BAP, 3 % sucrose, 0.7 % Phytagar at 23 °C, 16 hr light. After two days of co-cultivation with Agrobacterium, the petiole explants can be transferred to MSBAP-3 medium containing 3 mg/l BAP, cefotaxime, carbenicillin, or timentin (300 mg/l) for 7 days, and can then be cultured on MSBAP-3 medium with cefotaxime, carbenicillin, or timentin and selection agent until shoot regeneration. When the shoots are 5 - 10 mm in length, they can be cut and transferred to shoot elongation medium (MSBAP-0.5, containing 0.5 mg/l BAP). Shoots of about 2 cm in length can be transferred to the rooting medium (MS0) for root induction.

Samples of the primary transgenic plants (T0) can be analyzed by PCR to confirm the presence of T-DNA. These results can be confirmed by Southern hybridization in which DNA is electrophoresed on a 1 % agarose gel and are transferred to a positively charged nylon membrane (Roche Diagnostics). The PCR DIG Probe Synthesis Kit (Roche Diagnostics) can be used to prepare a digoxigenin-labelled probe by PCR, and used as recommended by the manufacturer.

Seeds of transgenic canola can be analyzed for increased nitrogen content with the elementar analyzer as described under example 3.

Example 10: Engineering alfalfa plants by over-expressing the polynucleotide characterized in the invention, e.g. derived from Saccharomyces, E.coli or another organism.

A regenerating clone of alfalfa (Medicago sativa) can be transformed using the method of (McKersie et al., 1999 Plant Physiol 119: 839-847). Regeneration and transformation of alfalfa can be genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) can be selected for use in tissue culture (Walker et al., 1978 Am J Bot 65:654-659).

Petiole explants can be cocultivated with an overnight culture of Agrobacterium tumefaciens C58C1 pMP90 (McKersie et al., 1999 Plant Physiol 119: 839-847) or LBA4404 containing a binary vector. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette can consist of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene that provides constitutive, developmental, tissue or environmental regulation of gene transcription. The 34S promoter (GenBank Accession numbers M59930 and X16673) can be used to provide constitutive expression of the trait gene.

The explants can be cocultivated for 3 d in the dark on SH induction medium containing 288 mg/ L Pro, 53 mg/ L thioproline, 4.35 g/ L K2SO4, and 100 µm acetosyringinone. The explants can be washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos can be transferred to BOi2Y development medium containing no growth regulators, no antibiotics, and 50 g/ L sucrose. Somatic embryos are subsequently germinated on half-strength Murashige-Skoog medium. Rooted seedlings can be transplanted into pots and grown in a greenhouse.

The T0 transgenic plants are propagated by node cuttings and rooted in Turface growth medium. The plants are defoliated and grown to a height of about 10 cm (approximately 2 weeks after defoliation).

Seeds of transgenic alfalfa can be analyzed for increased nitrogen content with the elementar analyzer as described under example 3.

Example 11: Preparation of homologous sequences from plants Different plants can be grown under standard or varying conditions in the greenhouse. RNA can be extracted following the protocol of Jones, Dunsmuir and Bedbrook (1985) EMBO J. 4: 2411-2418. Approx. 1 gram of tissue material from various organs is ground in liquid nitrogen. The powder is transferred to a 13 ml Falcon tube containing 4.5 ml NTES buffer (100 mM NaCl, 10 mM Tris/HCl pH 7.5, 1 mM EDTA, 1 % SDS; in RNase-free water) and 3 ml phenol/chloroform/isoamylalcohol (25/24/1), immediately mixed and stored on ice. The mixture is spun for 10 minutes at 7000 rpm using a centrifuge (Sorval; SM24 or SS34 rotor). The supernatant is transferred to a new tube, 1/10th volume of 3 M NaAcetate (pH 5.2; in RNase-free water) and 1 volume of isopropanol is added, mixed at stored for 1 hour or overnight at -20 °C. The mixture is spun for 10 minutes at 7000 rpm. The supernatant is discarded and the pellet washed with 70 % ethanol (v/v). The mixture is spun for 5 minutes at 7000 rpm, the supernatant is discarded and the pellet is air-dried. 1 ml RNase-free water is added and allow the DNA/RNA pellet to dissolve on ice at 4 C. The nucleic acid solution is transferred to a 2 ml Eppendorf tube and 1 ml of 4 M LiAcetate is added. After mixing the solution is kept for at least 3 hours, or overnight, at 4 C. The mixture is spun for 10 minutes at 14000 rpm, the supernatant discarded, the pellet washed with 70 % Ethanol, air-dried and dissolved in 200 µl of RNase-free water.
Total RNA can be used to construct a cDNA-library according to the manufacturer's protocol (for example using the ZAP-cDNA synthesis and cloning kit of Stratagene, La Jolla, USA). Basically, messenger RNA (mRNA) is primed in the first strand synthesis with a oligo(dT) linker-primer and is reverse-transcribed using reverse transcriptase. After second strand cDNA synthesis, the double-stranded cDNA is ligated into the Uni-ZAP XR vector. The Uni-ZAP XR vector allows in vivo excision of the pBluescript phagemid. The polylinker of the pBluescript phagemid has 21 unique cloning sites flanked by T3 and T7 promoters and a choice of 6 different primer sites for DNA sequencing. Systematic single run sequencing of the expected 5 prime end of the clones can allow preliminary annotation of the sequences for example with the help of the pedant pro Software package (Biomax, München). Clones for the nucleic acids of the invention or used in the process according to the invention can be identified based on homology search with standard algorithms like blastp or gap. Identified putative full length clones with identity or high homology can be subjected to further sequencing in order to obtain the complete sequence.
Additional new homologous sequences can be identified in a similar manner by preparing respective cDNA libraries from various plant sources as described above. Libraries can then be screened with available sequences of the invention under low stringency conditions for example as described in Sambrook et al., Molecular Cloning: A laboratory manual, Cold Spring Harbor 1989, Cold Spring Harbor Laboratory Press. Purified positive clones can be subjected to the in vivo excision and complete sequencing. A pairwise sequence alignment of the original and the new sequence using the blastp or gap program allows the identification of orthologs, meaning homologous sequences from different organisms, which should have a sequence identity of at least 30%. Furthermore the conservation of functionally important amino acid residues or domains, which can be identified by the alignment of several already available paralogs, can identify a new sequence as an new orthologs..

Alternatively libraries can be subjected to mass sequencing and obtained sequences can be stored in a sequence database, which then can be screened for putative orthologs by different search algorithms, for example the tbastn algorithm to search the obtained nucleic acid sequences with a amino acid sequence of the invention. Clones with the highest sequence identity are used for a complete sequence determination and orthologs can be identified as described above.

The oxidative pentose phosphate pathway is known as a major source of reducing power for biosynthetic processes such as fatty-acid synthesis and the assimilation of nitrogen (Neuhaus and Emes, Annu Rev Plant Physiol Plant Mol Biol 200, 51:111-140). In addition it provides metabolic intermediates for biosynthetic processes. Enzymes for this pathway are found both in the cytosol and in the plastids, whereby the precise distribution of the activities varies among plant species and growth conditions. One of the important enzymes of the oxidative pentose phosphate pathway is the glucose-6-phosophate dehydrogenase, with different genes encoding discrete cytosolic and plastidic isoenzymes. Two classes of plastidic G6PDH have been distinguished in higher plants. Although both plastidic activities are inhibited by increased NADPH/NADP+ and inactivated by dithiothreitol, one of the enzymes is striking less sensitive to both forms of modulation. The involvement of the less sensitive isoenzyme of G6PDH in the provision of the NADPH for ferredoxin-dependent reactions is suggested by an increase in the expression of genes encoding these isoforms in Arabidopsis after transfer from a medium containing ammonium to one including nitrate (Wang et al., Plant Cell 2000, 12:1491-15009) Additionally recent studies on barley roots have identified a similar plastidic isoform in barley to be induced in response to exposure to ammonium or glutamate (Esposito et al., Physiol Plant 2001, 113:469-476).

Although the basic features of the oxidative pentose phosphate pathway are well established, details of how the pathway operates in plants and how it influences other processes (like nitrogen fixation) remain largely conjecture (reviewed in Krüger and von Schaewen, Current Opinion in Plant Biology, 2003, 6:236-264).

Farnesyl pyrophosphate synthetase (FPP synthase) is known as a cytosolic enzyme that has dimethyl-allyl-transtransferase and geranyl-transtransferase activities (Wang et al., Plant Journal, 43, 413-424, 2005; Barth et al., Physiologica Plantarium, 121, 282-293, 2004; Glichet et al., Curr. Opinion Plant Biol., 6, 530-535, 2003). FPP synthase catalyzes the sequential 1'-4 coupling of isopentenyl diphosphate (IPP) with dimethylallyl diphosphate and geranyl diphosphate, forming C15 farnesyl pyrophosphate units for isoprenoid and sterol biosynthesis. It is unclear how sterol biosynthesis or protein farnesylation might be involved in increasing the nitrogen content in a cell, preferably in plants or parts thereof, particularly by generating or increasing the FPP synthase activity in an organelle, preferably in plastids.

L-lactate cytochrome c oxidoreductase/cytochrome b2 is known as a component of the mitochondrial intermembrane space, required for lactate utilization. It is unclear how cytochrome b2 activity might be involved in increasing the nitrogen content in a cell, preferably in plants or parts thereof, particularly by generating or increasing the cytochrome bs2 activity in an organelle, preferably in plastids.

Accordingly, in one embodiment this is achieved by the accumulation or production of a nitrogen or nitrogen containing compounds. In one embodiment the term "nitrogen or nitrogen containing compounds " as used herein relates to protein, containing"amino acids", or other nitrogen containing compounds like "heme-complex", "purine" and/or "pyrimidine"-containing compounds and/or derivates .Further, in another embodiment the term "nitrogen or nitrogen containing compounds" as used herein also relates to compositions of fine chemicals comprising N-containing compounds..

Accordingly, the present invention relates to a process comprising
**(a)** increasing or generating the activity of one or more of the of a protein as shown in table II, application no. 2 and/or application no. 3, column 3 encoded by the nucleic acid sequences as shown in table I, application no. 2 and/or application no. 3, column 5, in an organelle of a microorganism or plant, or
**(b)** increasing or generating the activity of a protein as shown in table II, application no. 2 and/or application no. 3, column 3 encoded by the nucleic acid sequences as shown in table I, application no. 2 and/or application no. 3, column 5 in the plastid of a microorganism or plant, or in one or more parts thereof; and
**(c)** growing the organism under conditions which permit the accumulation and/or production of nitrogen or nitrogen containing compounds , thus, N-containing compound, in said organism or in the culture medium surrounding the organism.

Accordingly, the present invention relates to a process for the accumulation and/or production of nitrogen or nitrogen containing compounds respectively comprising
**a)** increasing or generating the activity of a protein selected from the group as shown in table II, application no. 2 and/or application no. 3, column 3 encoded by the nucleic acid sequences selected from the group as shown in table I, column 5, in an organelle of a non-human organism, or
**b)** increasing or generating the activity of a protein selected from the group as shown in table II, application no. 2 and/or application no. 3, column 3 encoded by the nucleic acid sequences selected from the group as shown in table I, application no. 2 and/or application no. 3, column 5, which are joined to a nucleic acid sequence encoding a transit peptide in a non-human organism, or in one or more parts thereof; or
**c)** increasing or generating the activity of a protein selected from the group as shown in table II, application no. 2 and/or application no. 3, column 3 encoded by the nucleic acid sequences selected from the group as shown in table I, application no. 2 and/or application no. 3, column 5, which are joined to a nucleic acid sequence encoding chloroplast localization sequence, in a non-human organism, or in one or more parts thereof, and
**d)** growing the organism under conditions which permit the accumuation of nitrogen and/or the production of N-containing compound in said organism.

Advantagously the activity of the protein selected from the group as shown in table II, application no. 2 and/or application no. 3, column 3 encoded by the nucleic acid sequences selected from the group as shown in table I, application no. 2 and/or application no. 3, column 5 is increased or generated in the abovementioned process in the plastid of a plant.

In principle the nucleic acid sequence encoding a transit peptide can be isolated from every organism such as microorganisms such as algae or plants containing plastids preferably chloroplasts. A "transit peptide" is an amino acid sequence, whose encoding nucleic acid sequence is translated together with the corresponding structural gene. That means the transit peptide is an integral part of the translated protein and forms an amino terminal extension of the protein. Both are translated as so called "preprotein". In general the transit peptide is cleaved off from the preprotein during or just after import of the protein into the correct cell organelle such as a plastid to yield the mature protein. The transit peptide ensures correct localization of the mature protein by facilitating the transport of proteins through intracellular membranes. Preferred nucleic acid sequences encoding a transit peptide are derived from a nucleic acid sequence encoding a protein finally resided in the plastid and stemming from an organism selected from the group consisting of the genera

Acetabularia, Arabidopsis, Brassica, Capsicum, Chlamydomonas, Cururbita, Dunaliella, Euglena, Flaveria, Glycine, Helianthus, Hordeum, Lemna, Lolium, Lycopersion, Malus, Medicago, Mesembryanthemum, Nicotiana, Oenotherea, Oryza, Petunia, Phaseolus, Physcomitrella, Pinus, Pisum, Raphanus, Silene, Sinapis, Solanum, Spinacea, Stevia, Synechococcus, Triticum and Zea.

Advantageously such transit peptides, which are beneficially used in the inventive process, are derived from the nucleic acid sequence encoding a protein selected from the group consisting of

ribulose bisphosphate carboxylase/oxygenase, 5-enolpyruvyl-shikimate-3-phosphate synthase, acetolactate synthase, chloroplast ribosomal protein CS17, Cs protein, ferredoxin, plastocyanin, ribulose bisphosphate carboxylase activase, tryptophan synthase, acyl carrier protein, plastid chaperonin-60, cytochrome c552, 22-kDA heat shock protein, 33-kDa Oxygen-evolving enhancer protein 1, ATP synthase γ subunit, ATP synthase δ subunit, chlorophyll-a/b-binding proteinII-1, Oxygen-evolving enhancer protein 2, Oxygen-evolving enhancer protein 3, photosystem I: P21, photosystem I: P28, photosystem I: P30, photosystem I: P35, photosystem I: P37, glycerol-3-phosphate acyltransferases, chlorophyll a/b binding protein, CAB2 protein, hydroxymethyl-bilane synthase, pyruvate-orthophosphate dikinase, CAB3 protein, plastid ferritin, ferritin, early light-inducible protein, glutamate-1-semialdehyde aminotransferase, protochlorophyllide reductase, starch-granule-bound amylase synthase, light-harvesting chlorophyll a/b-binding protein of photosystem II, major pollen allergen Lol p 5a, plastid ClpB ATP-dependent protease, superoxide dismutase, ferredoxin NADP oxidoreductase, 28-kDa ribonucleoprotein, 31-kDa ribonucleoprotein, 33-kDa ribonucleoprotein, acetolactate synthase, ATP synthase CFO subunit 1, ATP synthase CFO subunit 2, ATP synthase CFO subunit 3, ATP synthase CFO subunit 4, cytochrome f, ADP-glucose pyrophosphorylase, glutamine synthase, glutamine synthase 2, carbonic anhydrase, GapA protein, heat-shock-protein hsp21, phosphate translocator, plastid ClpA ATP-dependent protease, plastid ribosomal protein CL24, plastid ribosomal protein CL9, plastid ribosomal protein PsCL18, plastid ribosomal protein PsCL25, DAHP synthase, starch phosphorylase, root acyl carrier protein II, betaine-aldehyde dehydrogenase, GapB protein, glutamine synthetase 2, phosphoribulokinase, nitrite reductase, ribosomal protein L12, ribosomal protein L13, ribosomal protein L21, ribosomal protein L35, ribosomal protein L40, triose phosphate-3-phosphoglyerate-phosphate translocator, ferredoxin-dependent glutamate synthase, glyceraldehyde-3-phosphate dehydrogenase, NADP-dependent malic enzyme and NADP-malate dehydrogenase.

More preferred the nucleic acid sequence encoding a transit peptide is derived from a nucleic acid sequence encoding a protein finally resided in the plastid and stemming from an organism selected from the group consisting of the species:

Acetabularia mediterranea, Arabidopsis thaliana, Brassica campestris, Brassica napus, Capsicum annuum, Chlamydomonas reinhardtii, Cururbita moschata, Dunaliella salina, Dunaliella tertiolecta, Euglena gracilis, Flaveria trinervia, Glycine max, Helianthus annuus, Hordeum vulgare, Lemna gibba, Lolium perenne, Lycopersion esculentum, Malus domestica, Medicago falcata, Medicago sativa, Mesembryanthemum crystallinum, Nicotiana plumbaginifolia, Nicotiana sylvestris, Nicotiana tabacum, Oenotherea hookeri, Oryza sativa, Petunia hybrida, Phaseolus vulgaris, Physcomitrella patens, Pinus tunbergii, Pisum sativum, Raphanus sativus, Silene pratensis, Sinapis alba, Solanum tuberosum, Spinacea oleracea, Stevia rebaudiana, Synechococcus, Synechocystis, Triticum aestivum and Zea mays.

Even more preferred nucleic acid sequences are encoding transit peptides as disclosed by von Heijne et al. [Plant Molecular Biology Reporter, Vol. 9 (2), 1991: 104 -126], which are hereby incorparated by reference. Table V shows some examples of the transit peptide sequences disclosed by von Heijne et al. According to the disclosure of the invention especially in the examples the skilled worker is able to link other nucleic acid sequences disclosed by von Heijne et al. to the nucleic acid sequences shown in table I, application no. 2 and/or application no. 3, columns 5 and 7. Most preferred nucleic acid sequences encoding transit peptides are derived from the genus Spinacia such as chlorplast 30S ribosomal protein PSrp-1, root acyl carrier protein II, acyl carrier protein, ATP synthase: γ subunit, ATP synthase: δ subunit, cytochrom f, ferredoxin I, ferredoxin NADP oxidoreductase (= FNR), nitrite reductase, phosphoribulokinase, plastocyanin or carbonic anhydrase. The skilled worker will recognize that various other nucleic acid sequences encoding transit peptides can easely isolated from plastid-localized proteins, which are expressed from nuclear genes as precursors and are then targeted to plastids. Such transit peptides encoding sequences can be used for the construction of other expression constructs. The transit peptides advantageously used in the inventive process and which are part of the inventive nucleic acid sequences and proteins are typically 20 to 120 amino acids, preferably 25 to 110, 30 to 100 or 35 to 90 amino acids, more preferably 40 to 85 amino acids and most preferably 45 to 80 amino acids in length and functions post-tranlationally to direct the protein to the plastid preferably to the chloroplast. The nucleic acid sequences encoding such transit peptides are localized upstream of nucleic acid sequence encoding the mature protein. For the correct molecular joining of the transit peptide encoding nucleic acid and the nucleic acid encoding the protein to be targeted it is sometimes necessary to introduce additional base pairs at the joining position, which forms restriction enzyme recognition sequences useful for the molecular joining of the different nucleic acid molecules. This procedure might lead to very few additional amino acids at the N-terminal of the mature imported protein, which usually and preferably do not interfer with the protein function. In any case, the additional base pairs at the joining position which forms restriction enzyme recognition sequences have to be choosen with care, in order to avoid the formation of stop codons or codons which encode amino acids with a strong influence on protein folding, like e.g. proline. It is preferred that such additional codons encode small n.d. structural flexible amino acids such as glycine or alanine.

As mentioned above the nucleic acid sequences coding for the proteins as shown in table II, application 2 and/or application no. 3, column 3 and its homologs as disclosed in table I, columns 5 and 7, respectively are joined to a nucleic acid sequence encoding a transit peptide, This nucleic acid sequence encoding a transit peptide ensures transport of the protein to the plastid. The nucleic acid sequence of the gene to be expressed and the nucleic acid sequence encoding the transit peptide are operably linked. Therefore the transit peptide is fused in frame to the nucleic acid sequence coding for proteins as shown in table II, application no. 2 and/or application no. 3, column 3 and its homologs as disclosed in table I, application no. 2 and/or application no. 3, columns 5 and 7.

The term "organelle" according to the invention shall mean for example "mitochondria" or preferably "plastid" (throughout the specification the "plural" shall comprise the "singular" and vice versa). The term "plastid" according to the invention are intended to include various forms of plastids including proplastids, chloroplasts, chromoplasts, gerontoplasts, leucoplasts, amyloplasts, elaioplasts and etioplasts preferably chloroplasts. They all have as a common ancestor the aforementioned proplasts.

Other transit peptides are disclosed by Schmidt et al. [J. Biol. Chem., Vol. 268, No. 36, 1993: 27447 - 27457], Della-Cioppa et al. [Plant. Physiol. 84, 1987: 965 - 968], de Castro Silva Filho et al. [Plant Mol. Biol., 30, 1996: 769 - 780], Zhao et al. [J. Biol. Chem. Vol. 270, No. 11, 1995: 6081 - 6087], Römer et al. [Biochem. Biophys. Res. Commun., Vol. 196, No. 3, 1993 : 1414 - 1421], Keegstra et al. [Annu. Rev. Plant Physiol. Plant Mol. Biol., 40, 1989: 471 - 501], Lubben et al. [Photosynthesis Res., 17, 1988: 173 - 194] and Lawrence et al. [J. Biol. Chem., Vol. 272, No. 33, 1997: 20357 - 20363]. A general review about targeting is disclosed by Kermode Allison R. in Critical Reviews in Plant Science 15 (4): 285 - 423 (1996) under the title "Mechanisms of Intracellular Protein Transport and Targeting in Plant Cells."

Favored transit peptide sequences, which are used in the inventive process and which forms part of the inventive nucleic acid sequences are generally enriched in hydroxylated amino acid residues (serine and threonine), with these two residues generally constituting 20 - 35 % of the total. They often have an amino-terminal region empty of Gly, Pro, and charged residues. Furthermore they have a number of small hydrophobic amino acids such as valine and alanine and generally acidic amino acids are lacking. In addition they generally have a middle region rich in Ser, Thr, Lys and Arg. Overall they have very often a net positive charge.

Alternatively, nucleic acid sequences coding for the transit peptides may be chemically synthesized either in part or wholly according to structure of transit peptide sequences disclosed in the prior art. Said natural or chemically synthesized sequences can be directly linked to the sequences encoding the mature protein or via a linker nucleic acid sequence, which may be typically less than 500 base pairs, preferably less than 450, 400, 350, 300, 250 or 200 base pairs, more preferably less than 150, 100, 90, 80, 70, 60, 50, 40 or 30 base pairs and most preferably less than 25, 20, 15, 12, 9, 6 or 3 base pairs in length and are in frame to the coding sequence. Furthermore favorable nucleic acid sequences encoding transit peptides may comprise sequences derived from more than one biological and/or chemical source and may include a nucleic acid sequence derived from the amino-terminal region of the mature protein, which in its native state is linked to the transit peptide. In a preferred empodiment of the invention said amino-terminal region of the mature protein is typically less than 150 amino acids, preferably less than 140, 130, 120, 110, 100 or 90 amino acids, more preferably less than 80, 70, 60, 50, 40, 35, 30, 25 or 20 amino acids and most preferably less than 19, 18, 17, 16, 15, 14, 13, 12, 11 or 10 amino acids in length. But even shorter or longer stretches are also possible. In addition target sequences, which facilitate the transport of proteins to other cell compartments such as the vacuole, endoplasmic reticulum, golgi complex, glyoxysomes, peroxisomes or mitochondria may be also part of the inventive nucleic acid sequence. The proteins translated from said inventive nucleic acid sequences are a kind of fusion proteins that means the nucleic acid sequences encoding the transit peptide for example the ones shown in table V, preferably the last one of the table are joint to the nucleic acid sequences shown in table I, application no. 2 and/or application no. 3, columns 5 and 7. The person skilled in the art is able to join said sequences in a functional manner. Advantageously the transit peptide part is cleaved off from the protein part shown in table II, application no. 2 and/or application no. 3, columns 5 and 7 during the transport preferably into the plastids. All products of the cleavage of the preferred transit peptide shown in the last line of table V have preferably the N-terminal amino acid sequences QIA CSS or QIA EFQLTT in front of the start methionine of the protein metioned in table II, columns 5 and 7. Other short amino acid sequences of an range of 1 to 20 amino acids preferable 2 to 15 amino acids, more preferable 3 to 10 amino acids most preferably 4 to 8 amino acids are also possible in front of the start methionine of the protein metioned in table II, columns 5 and 7. In case of the amino acid sequence QIA CSS the three amino acids in front of the start methionine are stemming from the LIC (= ligatation independent cloning) cassette. Said short amino acid sequence is preferred in the case of the expression of E. coli genes. In case of the amino acid sequence QIA EFQLTT the six amino acids in front of the start methionine are stemming from the LIC cassette. Said short amino acid sequence is preferred in the case of the expression of S. cerevisiae genes. The skilled worker knowns that other short sequences are also useful in the expression of the genes metioned in table I, application no. 2 and/or application no. 3, columns 5 and 7. Furthermore the skilled worker is aware of the fact that there is not a need for such short sequences in the expression of the genes.

**Table V: Examples of transit peptides disclosed by von Heijne et al.**

| **Trans Pep** | **Organism** | **Transit Peptide** | **SEQ ID NO:** | **Reference** |
|---|---|---|---|---|
| 1 | Acetabularia mediterranea | MASIMMNKSVVLSKECAKPLATPK VTLNKRGFATTIATKNREMMVWQP FNNKMFETFSFLPP | 960 | Mol. Gen. Genet. 218:445-452(1989) |
| 2 | Arabidopsis thaliana | MAASLQSTATFLQSAKIATAPSRG SSHLRSTQAVGKSFGLETSSARLT CSFQSDFKDFTGKCSDAVKIAGFA LATSALVVSGASAEGAPK | 971 | EMBO J. 8:3187-3194(1989) |
| 3 | Arabidopsis thaliana | MAQVSRICNGVQNPSLICNLSKSS QRKSPLSVSLKTQQHPRAYPISSS WGLKKSGMTLIGSELRPLKVMSSV STAEKASEIVLQPIREISGLIKLP | 972 | Mol. Gen. Genet. 210: 437 - 442 (1987) |
| 4 | Arabidopsis thaliana | MAAATTTTTTSSSISFSTKPSPSS SKSPLPISRFSLPFSLNPNKSSSS SRRRGIKSSSP SS ISAVLNTTTNV TTTPSPTKPTKPETF ISRFAPDQP RKGA | 973 | Plant Physiol. 85:1110-1117 (1987) |
| 5 | Arabidopsis thaliana | MITSSLTCSLQALKLSSPFAHGST PLSSLSKPNSFPNHRMPALVPV | 974 | J. Biol. Chem. 2652763-2767 (1990) |
| 6 | Arabidopsis thaliana | MASLLGTSSSAIWASPSLSSPSSK PSSSPICFRPGKLFGSKLNAGIQI RPKKNRSRYHVSVMNVATEINSTE QVVGKFDSKKSARPVYPFAAI | 975 | EMBO J. 9:1337-1346 (1990) |
| 7 | Arabidopsis thaliana | MASTALSSAIVGTSFIRRSPAPISL RSLPSANTQSLFGLKSGTARGG RVVAM | 976 | Plant Physiol. 93: 572-577 (1990) |
| 8 | Arabidopsis thaliana | MAASTMALSSPAFAGKAVNLSPAA SEVLGSGRVTNRKTV | 977 | Nucl. Acids Res. 14: 4051 -4064 (1986) |
| 9 | Arabidopsis thaliana | MAAITSATVTIPSFTGLKLAVSSK PKTLSTISRSSSATRAPPKLALKS SLKDFGVIAVATAASIVLAGNAMA MEVLLGSDDGSLAFVPSEFT | 978 | Gene 65: 59 - 69 (1988) |
| 10 | Arabidopsis thaliana | MAAAVSTVGAINRAPLSLNGSGSG AVSAPASTFLGKKVVTVSRFAQSN KKSNGSFKVLAVKEDKQTDGDRWR GLAYDTSDDQIDI | 961 | Nucl. Acids Res. 17: 2871 (1989) |
| 11 | Arabidopsis thaliana | MkSSMLSSTAWTSPAQATMVAPF TGLKSSASFPVTRKANNDITSITS NGGRVSC | 962 | Plant Mol. Biol. 11: 745 - 759 (1988) |
| 12 | Arabidopsis thaliana | MAASGTSATFRASVSSAPSSSSQL THLKSPFKAVKY TPLPS SRSKSSS FSVSCTIAKDPPVLMAAGSDPALW QRPDSFGRFGKFGGKYVPE | 963 | Proc. Natl. Acad. Sci. USA, 86: 4604 -4608 (1989) |
| 13 | Brassica campestris | MSTTFCSSVCMQATSLAATTRISF QKPALVSTTNLSFNLRRSIPTRFS ISCAAKPETVEKVSKIVKKQLSLK DDQKVVAE | 964 | Nucl. Acids Res. 15: 7197 (1987) |
| 14 | Brassica napus | MATTFSASVSMQATSLATTTRISF QKPVLVSNHGRTNLSFNLSRTRLSISC | 965 | Eur. J. Biochem. 174: 287 -295 (1988) |
| 15 | Chlamydomon as reinhardtii | MQALSSRVNIAAKPQRAQRLVVRA EEVKAAPKKEVGPKRGSLVK | 966 | Plant Mol. Biol. 12: 463 - 474 (1989) |
| 16 | Cucurbita moschata | MAELIQDKESAQSAATAAAASSGY ERRNEPAHSRKFLEVRSEEELLSCIKK | 967 | FEBS Lett. 238: 424 -430 (1988) |
| 17 | Spinacea oleracea | MSTINGCLTSISPSRTQLKNTSTL RPTFIANSRVNPSSSVPPSLIRNQ PVFAAPAPIITPTL | 968 | J. Biol. Chem. 265: 105414 -5417 (1990) |
| 18 | Spinacea oleracea | MTTAVTAAVSFPSTKTTSLSARCS SVISPDKISYKKVPLYYRNVSATG KMGPIRAQIASDVEAPPPAPAKVEKMS | 969 | Curr. Genet. 13: 517 - 522 (1988) |
| 19 | Spinacea oleracea | MTTAVTAAVSFPSTKTTSLSARSS SVISPDKISYKKVPLYYRNVSATG KMGPIRA | 970 | |

Alternatively to the targeting of the sequences shown in table II, application no. 2 and/or application no. 3, columns 5 and 7 preferably of sequences in general encoded in the nucleus with the aid of the targeting sequences mentioned for example in table V alone or in combination with other targeting sequences preferably into the plastids, the nucleic acids of the invention can directly introduced into the plastidal genome. Preferably the nucleic acid sequences shown in table I, application no. 2 and/or application no. 3, columns 5 and 7 are directly introduced into the plastidal genome in such a way that they are under control of a promoter active in plastids.
Therefore in a preferred embodiment the nucleic acid sequences shown in table I, application no. 2 and/or application no. 3, columns 5 and 7 are directly introduced and expressed in plastids.

The term "introduced" in the context of this specification shall mean the insertion of a nucleic acid sequence into the organism by means of a "transfection", "transduction" or preferably by "transformation".

A plastid, such as a chloroplast, has been "transformed" by an exogenous (preferably foreign) nucleic acid sequence if nucleic acid sequence has been introduced into the plastid that means that this sequence has crossed the membrane or the membranes of the plastid. The foreign DNA may be integrated (covalently linked) into plastid DNA making up the genome of the plastid, or it may remain unintegrated (e.g., by including a chloroplast origin of replication). "Stably" integrated DNA sequences are those, which are inherited through plastid replication, thereby transferring new plastids, with the features of the integrated DNA sequence to the progeny.

For expression a person skilled in the art is familiar with different methods to introduce the nucleic acid sequences into different organelles such as the preferred plastids. Such methods are for example disclosed by Pal Maiga (Annu. Rev. Plant Biol., 2004, 55: 289 - 313), Thomas Evans (WO 2004/040973), Kevin E. McBride et al. (US 5,455,818), Henry Daniell et al. (US 5,932,479 and US 5,693,507) and Jeffrey M. Straub et al. (US 6,781,033). A preferred method is the transformation of microspore-derived hypocotyl or cotyledonary tissue (which are green and thus contain numerous plastids) leaf tissue and afterwards the regeneration of shoots from said transformed plant material on selective medium. As methods for the transformation bombarding of the plant material or the use of independently replicating shuttle vectors are well known by the skilled worker. But also a PEG-mediated transformation of the plastids or Agrobacterium transformation with binary vectors are possible. Useful markers for the transformation of plastids are positive selection markers for example the chloramphenicol-, streptomycin-, kanamycin-, neomycin-, amikamycin-, spectinomycin-, triazine- and/or lincomycin-resistance genes. As additional markers named in the literature often as secondary markers, genes coding for the resistence against herbicides such as phosphinothricin (= glufosinate, BASTATM, LibertyTM, encoded by the bar gene), glyphosate (= N-(phosphonomethyl)glycine, Roundup ReadyTM, encoded by the 5-enolpyruvylshikimaete-3-phosphate synthase gene = epsps), sulfonylurea (= StapleTM, encoded by the acetolactate synthase gene), imidazolinone [= IMI, imazethapyr, imazamox, ClearfieldTM, encoded by the acetohydroxyacid synthase (AHAS) gene, also known as acetolactate synthase (ALS) gene] or bromoxynil (= BuctrilTM, encoded by the oxy gene) or genes coding for antibiotics such as hygromycin or G418 are useful for further selection. Such secondary markers are useful in the case when most genome copies are transformed. In addition negative selection markers such as the bacterial cytosine deaminase (encoded by the codA gene) are also useful for the transformation of plastids.

To increase the possibility of identification of transformants it is also diserable to use reporter genes other then the aforementioned resistance genes or in addition to said genes. Reporter genes are for example β-galactosidase-, β-glucuronidase- (GUS), alkaline phosphatase- and/or green-fluorescent protein-genes (GFP).

For the inventive process it is of great advantage that by transforming the plastids the intraspecies specific transgene flow is blocked, because a lot of species such as corn, cotton and rice have a strict maternal inheritance of plastids. By placing the genes specified in table I, application no. 2 and/or application no. 3, columns 5 and 7 or active fragments thereof in the plastids of plants, these genes will not be present in the pollen of said plants.
A further preferred embodiment of the invention relates to the use of so called "chloroplast localization sequences", in which a first RNA sequence or molecule is capable of transporting or "chaperoning" a second RNA sequence, such as a RNA sequence transcribed from the sequences selected from the group as depicted in table I, application no. 2 and/or application no. 3, columns 5 and 7 or a sequence encoding a protein selected from the group as as depicted in table II, application no. 2 and/or application no. 3, columns 5 and 7, from an external environment inside a cell or outside a plastid into a chloroplast. In one embodiment the chloroplast localization signal is substantially similar or complementary to a complete or intact viroid sequence. The chloroplast localization signal may be encoded by a DNA sequence, which is transcribed into the chloroplast localization RNA. The term "viroid" refers to a naturally occurring single stranded RNA molecule (Flores, C R Acad Sci III. 2001 Oct; 324(10):943-52). Viroids usually contain about 200-500 nucleotides and generally exist as circular molecules. Examples of viroids that contain chloroplast localization signals include but are not limeted to ASBVd, PLMVd, CChMVd and ELVd. The viroid sequence or a functional part of it can be fused to the sequences selected from the group as depicted in table I, application no. 2 and/or application no. 3, columns 5 and 7 or a sequence encoding a protein selected from the group as depicted in table II, application no. 2 and/or application no. 3, columns 5 and 7 in such a manner that the viroid sequence transports a sequence transcribed from a sequence as depicted in table I, application no. 2 and/or application no. 3, columns 5 and 7 or a sequence encoding a protein as depicted in table II, application no. 2 and/or application no. 3, columns 5 and 7 into the chloroplasts. A preferred embodiment uses a modified ASBVd (Navarro et al., Virology. 2000 Mar 1;268(1):218-25).

In a further specific embodiment the protein to be expressed in the plastids such as the proteins selected from the group as depicted in table II, application no. 2 and or application no. 3, columns 5 and 7 are encoded by different nucleic acids. Such a method is disclosed in WO 2004/040973, which shall be incorporated by reference. WO 2004/040973 teaches a method, which relates to the translocation of an RNA corresponding to a gene or gene fragment into the chloroplast by means of a chloroplast localization sequence. The genes, which should be expressed in the plant or plants cells, are split into nucleic acid fragments, which are introduced into different compartments in the plant e.g. the nucleus, the plastids and/or mitochondria. Additionally plant cells are described in which the chloroplast contains a ribozyme fused at one end to an RNA encoding a fragment of a protein used in the inventive process such that the ribozyme can trans-splice the translocated fusion RNA to the RNA encoding the gene fragment to form and as the case may be reunite the nucleic acid fragments to an intact mRNA encoding a functional protein for example selected from the group as disclosed in table II, application no. 2 and/or application no. 3, columns 5 and 7.

In a preferred embodiment of the invention the nucleic acid sequences selected from the group as shown in table I, application no. 2 and/or application no. 3, columns 5 and 7 used in the inventive process are transformed into plastids, which are metabolical active. Those plastids should preferably maintain at a high copy number in the plant or plant tissue of interest, most preferably the chloroplasts found in green plant tissues, such as leaves or cotyledons or in seeds.

For a good expression in the plastids the nucleic acid sequences as shown in table I, application no. 2 and/or application no. 3, columns 5 and 7 are introduced into an expression cassette using a preferably a promoter and terminater, which are active in plastids preferably a chloroplast promoter and chloroplast terminater respectively. Examples of such promoters include the psbA promoter from the gene from spinach or pea, the rbcL promoter, and the atpB promoter from corn.

Surprisingly it was found, that the transgenic expression of the protein selected from the group as shown in table II, application no. 2 and/or application no. 3, column 3 in plastids of a plant such as Arabidopsis thaliana for example through the linkage to at least one targeting sequence for example as mentioned in table V conferred an increase in nitrogen or nitrogen containing compounds content of the transformed plants.

In one embodiment, in the process of the present invention the activity of a protein selected from the group as shown in table II, application no. 2 and/or application no. 3, column 3 is increased or generated, or a homolog thereof, preferably linked at least to one transit peptide as mentioned for example in table V.
In another embodiment, in the process of the present invention the activity of a a protein selected from the group as shown in table II, application no. 2 and/or application no. 3, column 3 is increased or generated in a subcellular compartment of the organism or organism cell such as in an organelle like a plastid or mitochondria.

The sequence of YNL241C (Accession number NP_014158) from Saccharomyces cerevisiae has been published in Goffeau et al., Science 274 (5287), 546-547, 1996 and Philippsen et al., Nature 387 (6632 Suppl), 93-98 (1997), and its activity is being defined as "glucose-6-phosphate dehydrogenase (Zwf1 p)". Accordingly, in one embodiment, the process of the present invention comprises the use of said "glucose-6-phosphate dehydrogenase" or its homolog, preferably in plastids, e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for increasing the amount of a N-containing compound in an organism or a part thereof, as mentioned.
Accordingly, in one embodiment, the process of the present invention comprises the use of said "glucose-6-phosphate dehydrogenase" or its homolog, preferably in plastids, e.g. as shown herein, for the production of nitrogen or nitrogen containing compounds and/or for conferring an enhanced nitrogen assimilation, accumulation and/or utilization and/or a increased total nitrogen content" meaning of a N-containing compound, in particular for the improved uptake and/or assimilation of nitrogen. Accordingly, in one embodiment, the process of the present invention comprises the use of said "glucose-6-phosphate dehydrogenase" or its homolog, preferably in plastids, e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for the increased uptake and/or utilization and/or assimilation of nitrogen under nitrogen limited conditions.
The sequence of b1852 (Accession number NP_416366) from Escherichia coli has been published in Blattner et al., Science 277 (5331), 1453-1474 (1997), and its activity is being defined as "glucose-6-phosphate dehydrogenase". Accordingly, in one embodiment, the process of the present invention comprises the use of a "glucose-6-phosphate dehydrogenase" or its homolog, preferably in plastids" e.g. as shown herein, for the production of nitrogen or nitrogen containing compounds and/or for conferring an enhanced nitrogen assimilation, accumulation and/or utilization and/or a increased total nitrogen content" meaning of a N-containing compound, in particular for increasing the amount of a N-containing compound in an organism or a part thereof, as mentioned.
Accordingly, in one embodiment, the process of the present invention comprises the use of a "glucose-6-phosphate dehydrogenase" or its homolog, preferably in plastids, e.g. as shown herein, for the production of nitrogen or nitrogen containing compounds and/or for conferring an enhanced nitrogen assimilation, accumulation and/or utilization and/or a increased total nitrogen content, meaning of a N-containing compound, in for the improved uptake and/or assimilation of nitrogen..
Accordingly, in one embodiment, the process of the present invention comprises the use of a "glucose-6-phosphate dehydrogenase" or its homolog, preferably in plastids" e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for the increased uptake and/or utilization and/or assimilation of nitrogen under nitrogen limited conditions.
The sequence of YJL167W (Accession number NP_012368.1) from Saccharomyces cerevisiae has been published in Goffeau et al., Science 274 (5287), 546-547, 1996 and Anderson et al., J. Biol. Chem 264, 19176-19184 (1989), and its activity is being defined as "farnesyl pyrophosphate synthetase (FPP synthase)". Accordingly, in one embodiment, the process of the present invention comprises the use of said "farnesyl pyrophosphate synthetase (FPP synthase)" or its homolog, preferably in plastids, e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for increasing the amount of a N-containing compound in an organism or a part thereof, as mentioned.
Accordingly, in one embodiment, the process of the present invention comprises the use of said "farnesyl pyrophosphate synthetase (FPP synthase)" or its homolog, preferably in plastids, e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for the improved uptake and/or assimilation of nitrogen.
Accordingly, in one embodiment, the process of the present invention comprises the use of said "farnesyl pyrophosphate synthetase (FPP synthase)" or its homolog, preferably in plastids , e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for the increased uptake and/or utilization and/or assimilation of nitrogen under nitrogen limited conditions.
The sequence of YML045C (Accession number NP_013658.1) from Saccharomyces cerevisiae has been published in Goffeau et al., Science 274 (5287), 546-547, 1996 and Guiard et al., EMBO J. 4, 3265-3272 (1985), and its activity is being defined as "L-lactate cytochrome c oxidoreductase/cytochrome b2". Accordingly, in one embodiment, the process of the present invention comprises the use of said "L-lactate cytochrome c oxidoreductase/cytochrome b2" or its homolog, preferably in plastids, e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for increasing the amount of a N-containing compound in an organism or a part thereof, as mentioned.
Accordingly, in one embodiment, the process of the present invention comprises the use of said "L-lactate cytochrome c oxidoreductase/cytochrome b2" or its homolog, preferably in plastids, e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for the improved uptake and/or assimilation of nitrogen.
Accordingly, in one embodiment, the process of the present invention comprises the use of said "L-lactate cytochrome c oxidoreductase/cytochrome b2" or its homolog, preferably in plastids , e.g. as shown herein, for the production of nitrogen or nitrogen containing compounds and/or for conferring an enhanced nitrogen assimilation, accumulation and/or utilization and/or a increased total nitrogen content, meaning of a N-containing compound, in particular for the increased uptake and/or utilization and/or assimilation of nitrogen under nitrogen limited conditions.

For example, the molecule number or the specific activity of the polypeptide or the nucleic acid molecule may be increased. Larger amounts of the N-containing compound can be produced if the polypeptide or the nucleic acid of the invention is expressed *de novo* in an organism lacking the activity of said protein, preferably the nucleic acid molecules selected from the group as mentioned in table I, application no. 2 and/or application no. 3, columns 5 and 7 alone or preferably in combination with a transit peptide for example as mentioned in table V or in another embodiment by introducing said nucleic acid molecules into an organelle such as an plastid or mitochondria in the transgenic organism. However, it is also possible to modifiy the expression of the gene which is naturally present in the organisms, for example by integrating a nucleic acid sequence, encoding a plastidic targeting sequence in front (5 prime) of the coding sequence, leading to a functional preprotein, which is directed for example to the plastids.

In one embodiment, in case the activity of the Saccharomyces cerevisiae protein YNL241C or its homologs, e.g. as indicated in Table II, columns 5 or 7, line 3, is increased preferably in a cellular compartment, preferably in the plastids,; preferably, an increase of nitrogen or nitrogen containing compounds , preferably of total nitrogen content between 9% and 12% or more is conferred, preferably an increase of amino acid content in a plant between 14% and 27% or more is conferred.

In one embodiment, in case the activity of the Saccharomyces cerevisiae protein YNL241C or its homologs is increased preferably in a cellular compartment, preferably in the plastids" preferably, an increase of nitrogen or nitrogen containing compounds and of fatty acids, phytosterol, fructose and/or glucose in leaves and/or carbohydrate in seeds of a plant is conferred.

In one embodiment, in case the activity of the E. coli protein b1852 or its homologs, e.g. as indicated in Table II, columns 5 or 7, line 2, is increased preferably in a cellular compartment, preferably in the plastids,; preferably, an increase of nitrogen or nitrogen containing compounds, preferably of total nitrogen content between 6% and 13% or more is conferred, preferably an increase of amino acid content in a plant between 28% and 62% or more is conferred.

In one embodiment, in case the activity of the Saccharomyces cerevisiae protein YJL167W or its homologs, e.g. as indicated in Table II, columns 5 or 7, application no. 3, line 4, is increased, preferably in a cellular compartment, preferably in the plastids, preferably, an increase of nitrogen or nitrogen containing compounds between 5% and 30 % or more, preferably between 8% and 26% or more is conferred preferably in the seeds.

In one embodiment, in case the activity of the Saccharomyces cerevisiae protein YML054C or its homologs, e.g. as indicated in Table II, columns 5 or 7, application no. 3, line 5, is increased, preferably in a cellular compartment, preferably in the plastids, preferably, an increase of nitrogen or nitrogen containing compounds between 5% and 20% or more, preferably between 6% and 15% or more is conferred preferably in the seeds.

In one embodiment, the process of the present invention comprises one or more of the following steps
a) stabilizing a protein conferring the increased expression of a protein encoded by the nucleic acid molecule of the invention or of the polypeptid of the invention, e.g. of a polypeptide having the activity of a protein selected from the group as indicated in table II, application no. 2 and/or application no. 3, columns 5 and 7 or its homologs activity having herein-mentioned N-containing compound increasing activity preferably in a cellular compartment, preferably in the plastids,; and/or
b) stabilizing a mRNA conferring the increased expression of a protein encoded by the nucleic acid molecule of the invention, which is in the sense of the invention a fusion of a nucleic acid sequence encoding a transit peptide and of a nucleic acid sequence selected from the group as shown in table I, application no. 2 and/or application no. 3, columns 5 and 7, e.g. a nucleic acid sequence encoding a polypeptide having the activity of a protein selected from the group as indicated in table II, application no. 2 and/or application no. 3, columns 5 and 7 or its homologs or of a mRNA encoding the polypeptide of the present invention having herein-mentioned N-containing compound increasing activity ; and/or
c) increasing the specific activity of a protein conferring the increased expression of a protein encoded by the nucleic acid molecule of the invention or of the polypeptide of the present invention having herein-mentioned N-containing compound increasing activity, e.g. of a polypeptide having the activity of a protein selected from the group as indicated in table II, application no. 2 and/or application no. 3, columns 5 and 7 or its homologs activity preferably in a cellular compartment, preferably in the plastids" or decreasing the inhibitiory regulation of the polypeptide of the invention; and/or
d) generating or increasing the expression of an endogenous or artificial transcription factor mediating the expression of a protein conferring the increased expression of a protein encoded by the nucleic acid molecule of the invention or of the polypeptide of the invention having herein-mentioned N-containing compound increasing activity, e.g. of a polypeptide having the activity of a protein selected from the group as indicated in table II, application no. 2 and/or application no. 3, columns 5 and 7 or its homologs preferably in a cellular compartment, preferably in the plastids,; and/or
e) stimulating activity of a protein conferring the increased expression of a protein encoded by the nucleic acid molecule of the present invention or a polypeptide of the present invention having herein-mentioned N-containing compound increasing activity, e.g. of a polypeptide having the activity of a protein selected from the group as indicated in table II, application no. 2 and/or application no. 3, columns 5 and 7 or its homologs activity preferably in a cellular compartment, preferably in the plastids" by adding one or more exogenous inducing factors to the organismus or parts thereof; and/or
f) expressing a transgenic gene encoding a protein conferring the increased expression of a polypeptide encoded by the nucleic acid molecule of the present invention or a polypeptide of the present invention preferably in a cellular compartment, preferably in the plastids" having herein-mentioned N-containing compound increasing activity, e.g. of a polypeptide having the activity of a protein selected from the group as indicated in table II, application no. 2 and/or application no. 3, columns 5 and 7 or its homologs activity, and/or
g) increasing the copy number of a gene conferring the increased expression of a nucleic acid molecule encoding a polypeptide encoded by the nucleic acid molecule of the invention or the polypeptide of the invention having herein-mentioned N-containing compound increasing activity, e.g. of a polypeptide having the activity of a protein selected from the group as indicated in table II, application no. 2 and/or application no. 3, columns 5 and 7 or its homologs activity; and/or
h) increasing the expression of the endogenous gene encoding the polypeptide of the invention, e.g. a polypeptide having the activity of a protein selected from the group as indicated in table II, application no. 2 and/or application no. 3, columns 5 and 7 or its homologs activity preferably in a cellular compartment, preferably in the plastids" by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced; and/or
i) modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced production of nitrogen or nitrogen containing compounds; and/or
j) selecting of organisms with expecially high activity of the proteins of the invention preferably in a cellular compartment, preferably in the plastids,from natural or from mutagenized resources and breeding them into the target organisms, eg the elite crops; and/or
k) directing a protein encoded by the nucleic acid molecule of the invention or of the polypeptide of the present invention having herein-mentioned N-containing compound increasing activity, e.g. of a polypeptide having the activity of a protein selected from the group as indicated in table II, application no. 2 and/or application no. 3, columns 5 and 7 or its homologs activity, to the plastids by the addition of a plastidial targeting sequence; and/or
l) generating the expression of a protein encoded by the nucleic acid molecule of the invention or of the polypeptide of the present invention having herein-mentioned N-containing compound increasing activity, e.g. of a polypeptide having the activity of a protein selected from the group as indicated in table II, application no. 2 and/or application no. 3, columns 5 and 7 or its homologs activity in plastids by the stable or transient transformation advantageously stable transformation of organelles preferably plastids with an inventive nucleic acid sequence preferably in form of an expression cassette containing said sequence leading to the plastidial expression of of the nucleic acids or polypeptides of the invention; and/or
m) generating the expression of a protein encoded by the nucleic acid molecule of the invention or of the polypeptide of the present invention having herein-mentioned N-containing compound increasing activity, e.g. of a polypeptide having the activity of a protein selected from the group as indicated in table II, application no. 2 and/or application no. 3, columns 5 and 7 or its homologs activity in plastids by integration of a nucleic acid of the invention into the plastidal genome under control of preferable a plastidial promoter.

One can also envisage to introduce nucleic acids sequences, encoding plastidal targeting signals, like for example present in table V, by homologous recombination or other methods of site specific integration, into the genome in that way, that an endogenous gene is functionally fused to the targeting sequence and the protein is redirected to to the plastids. Eventually the integration can also occur randomly and the desired fusion event is selected for.

In a preferred embodiment, the present invention relates to a process for the accumulation and/or production of nitrogen or nitrogen containing compounds respectively comprising or generating in an organism or a part thereof, preferably in a cell compartment such as a plastid or mitochondria, the expression of at least one nucleic acid molecule comprising a nucleic acid molecule selected from the group consisting of:
a) nucleic acid molecule encoding, preferably at least the mature form, of the polypeptide selected from the group shown in table II, application no. 2 and/or application no. 3, columns 5 and 7 or a fragment thereof, which confers an increase in the amount of nitrogen or nitrogen containing compounds in an organism or a part thereof;
b) nucleic acid molecule comprising, preferably at least the mature form, of the nucleic acid molecule selected from the group shown in table I, application no. 2 and/or application no. 3, columns 5 and 7;
c) nucleic acid molecule whose sequence can be deduced from a polypeptide sequence encoded by a nucleic acid molecule of (a) or (b) as result of the degeneracy of the genetic code and conferring an increase in the amount of nitrogen or nitrogen containing compounds in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of nitrogen or nitrogen containing compounds in an organism or a part thereof;
e) nucleic acid molecule which hybidizes with a nucleic acid molecule of (a) to (c) under under stringent hybridisation conditions and conferring an increase in the amount of nitrogen or nitrogen containing compounds in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of nitrogen or nitrogen containing compounds in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of nitrogen or nitrogen containing compounds in an organism or a part thereof;
h) nucleic acid molecule comprising a nucleic acid molecule which is obtained by amplifying nucleic acid molecules from a cDNA library or a genomic library using the primers shown in table III, application no. 2 and/or application no. 3, column 7 and conferring an increase in the amount of nitrogen or nitrogen containing compounds in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of nitrogen or nitrogen containing compounds in an organism or a part thereof;
j) nucleic acid molecule which encodes a polypeptide comprising the consensus sequence selected from the group shown in table IV, application no. 2 and/or application no. 3, column 7 and conferring an increase in the amount of nitrogen or nitrogen containing compounds in an organism or a part thereof;
k) nucleic acid molecule comprising one or more of the nucleic acid molecule encoding the amino acid sequence of a polypeptide encoding a domain of the polypeptide selected from the group shown in table II, application no. 2 and/or application no. 3, columns 5 and 7 and conferring an increase in the amount of nitrogen or nitrogen containing compounds in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of nitrogen or nitrogen containing compounds in an organism or a part thereof;
   or which comprises a sequence which is complementary thereto.

Nucleic acid molecules with the sequence shown in table I, application no. 2 and/or application no. 3, columns 5 and 7, nucleic acid molecules which are derived from the amino acid sequences shown in table II, application no. 2 and/or application no. 3, columns 5 and 7 or from polypeptides comprising the consensus sequence shown in table IV, application no. 2 and/or application no. 3, column 7, or their derivatives or homologues encoding polypeptides with the enzymatic or biological activity of a protein as shown in table II, application no. 2 and/or application no. 3, column 3 or conferring nitrogen or nitrogen containing compounds increase after increasing its expression or activity are advantageously increased in the process according to the invention by expression either in the cytsol or in an organelle such as a plastid or mitochondria or both, preferably in plastids.

The nucleic acid molecules used in the process according to the invention take the form of isolated nucleic acid sequences, which encode polypeptides with the activity of the proteins selected from the group as shown in table II, application no. 2 and/or application no. 3, column 3 and conferring nitrogen or nitrogen containing compounds increase increase by expression either in the cytsol or in an organelle such as a plastid or mitochondria or both, preferably in plastids.

**[0108.0.0.2] to [0210.0.0.2] see [0101.0.0.1] to [0203.0.0.1]**

The nucleic acid sequence according to the invention mentioned above is advantageously functionally joined to a nucleic acid sequence encoding a transit peptide, in such a manner that a preprotein is translated, which is able to direct the polypeptide to the organelle such as to the plastid. In another preferred embodiment the nucleic acids according to the invention mentioned above is advantageously functionally joined to a promotor region functional in plastids like for example the RNA operon promoter fused to the 5'UTR of the *rbcL* gene and in another preferred embodiment joined to a plastome sequences homologous to the integration sites. Example for useful integration sites are the *trnV-rps12*/*7* (Skidar et al., Plant Cell Rep. 1998, 18: 20-24 and other reports), thr *rbvL-aacD* site (Svab et al. 1993, Proc. Natl. Acad. Sci. USA 90: 913-917), *the trnI-trnA* site (De Cosa et al., 2001, Nat. Biotech. 19, 71-74) the *rps7-ndhB* site (Hou et al., 2003, Transgenic Res. 12, 111-114) and the *ndhF-trnL* site Zhang et al., 2001 c, Plant Physiol. 127, 131-141)

The nucleic acid sequence coding for the transit peptide is advantageously derived from a nucleic acid sequence encoding a protein finally resided in the plastid and is stemming from an organism selected from the group consisting of the Genera
Acetabularia, Arabidopsis, Brassica, Chlamydomonas, Cururbita, Dunaliella, Euglena, Flaveria, Glycine, Helianthus, Hordeum, Lemna, Lolium, Lycopersion, Malus, Mesembryanthemum, Nicotiana, Oenotherea, Oryza, Petúnia, Phaseolus, Physcomitrella, Pinus, Pisum Raphanus, Silene, Sinapis, Solanum, Spinacea, Triticum and Zea.

Preferably the transit peptide is derived from a protein selected from the group consisting of
ribulose bisphosphate carboxylase/oxygenase, 5-enolpyruvyl-shikimate-3-phosphate synthase, acetolactate synthase, chloroplast ribosomal protein CS17, Cs protein, ferredoxin, plastocyanin, ribulose bisphosphate carboxylase activase, tryptophan synthase, acyl carrier protein, plastid chaperonin-60, cytochrome C₅₅₂, 22-kDA heat shock protein, 33-kDa Oxygen-evolving enhancer protein 1, ATP synthase γ subunit, ATP synthase δ subunit, chlorophyll-a/b-binding proteinll-1, Oxygen-evolving enhancer protein 2, Oxygen-evolving enhancer protein 3, photosystem I: P21, photosystem I: P28, photosystem I: P30, photosystem I: P35, photosystem I: P37, glycerol-3-phosphate acyltransferases, chlorophyll a/b binding protein, CAB2 protein, hydroxymethyl-bilane synthase, pyruvate-orthophosphate dikinase, CAB3 protein, plastid ferritin, ferritin, early light-inducible protein, glutamate-1-semialdehyde aminotransferase, protochlorophyllide reductase, starch-granule-bound amylase synthase, light-harvesting chlorophyll a/b-binding protein of photosystem II, major pollen allergen Lol p 5a, plastid ClpB ATP-dependent protease, superoxide dismutase, ferredoxin NADP oxidoreductase, 28-kDa ribonucleoprotein, 31-kDa ribonucleoprotein, 33-kDa ribonucleoprotein, acetolactate synthase, ATP synthase CF₀ subunit 1, ATP synthase CF₀ subunit 2, ATP synthase CF₀ subunit 3, ATP synthase CF₀ subunit 4, cytochrome f, ADP-glucose pyrophosphorylase, glutamine synthase, glutamine synthase 2, carbonic anhydrase, GapA protein, heat-shock-protein hsp21, phosphate translocator, plastid ClpA ATP-dependent protease, plastid ribosomal protein CL24, plastid ribosomal protein CL9, plastid ribosomal protein PsCL18, plastid ribosomal protein PsCL25, DAHP synthase, starch phosphorylase, root acyl carrier protein II, betaine-aldehyde dehydrogenase, GapB protein, glutamine synthetase 2, phosphoribulokinase, nitrite reductase, ribosomal protein L12, ribosomal protein L13, ribosomal protein L21, ribosomal protein L35, ribosomal protein L40, triose phosphate-3-phosphoglyerate-phosphate translocator, ferredoxin-dependent glutamate synthase, glyceraldehyde-3-phosphate dehydrogenase, NADP-dependent malic enzyme and NADP-malate dehydrogenase. The plastome sequences are preferential derived from the plastome of the target organisms themselves and are advantegously derived from one of the following intergration sites: *trnV-rps12*/*7* (Skidar et al., Plant Cell Rep. 1998, 18: 20-24 and other reports), *rbvL-aacD* (Svab et al.1993, Proc. Natl. Acad. Sci. USA 90: 913-917), *trnI-trnA* (De Cosa et al., 2001, Nat. Biotech. 19, 71-74) *rps7-ndhB* (Hou et al., 2003, Transgenic Res. 12, 111-114) or *ndhF-trnL* site Zhang et al., 2001c, Plant Physiol. 127, 131-141).

The nucleic acid sequences used in the process are advantageously introduced in a nucleic acid construct, preferably an expression cassette, which makes possible the expression of the nucleic acid molecules in an organism, advantageously a plant or a microorganism such as an algae, advantegously in the plastids of those organisms.

In principle, the nucleic acid construct can comprise the herein described regulator sequences and further sequences relevant for the expression of the comprised genes. Thus, the nucleic acid construct of the invention can be used as expression cassette and thus can be used directly for introduction into the plant, or else they may be introduced into a vector. Accordingly in one embodiment the nucleic acid construct is an expression cassette comprising a microorganism promoter or a microorganism terminator or both. In another embodiment the expression cassette encompasses a plant promoter or a plant terminator or both. In another embodiment the expression cassette encompasses sequences for transcription by plastid RNA polymerases.

Accordingly, in one embodiment, the process according to the invention comprises the following steps:
(a) introducing of a nucleic acid construct comprising the nucleic acid molecule of the invention or used in the process of the invention or encoding the polypeptide of the present invention or used in the process of the invention; or
(b) introducing of a nucleic acid molecule, including regulatory sequences or factors, which expression increases the expression of the nucleic acid molecule of the invention or used in the process of the invention or encoding the polypeptide of the present invention or used in the process of the invention;
   in a cell, or an organism or a part thereof, preferably in a plant, plant cell or a microorganism preferably in the organelles such as the plastids thereof, and
(c) expressing of the gene product encoded by the nucleic acid construct or the nucleic acid molecule mentioned under (a) or (b) in the cell or the organism or part thereof.

**[0217.0.0.2] to [0370.0.0.2] see [0204.0.0.1] to [0357.0.0.1]**

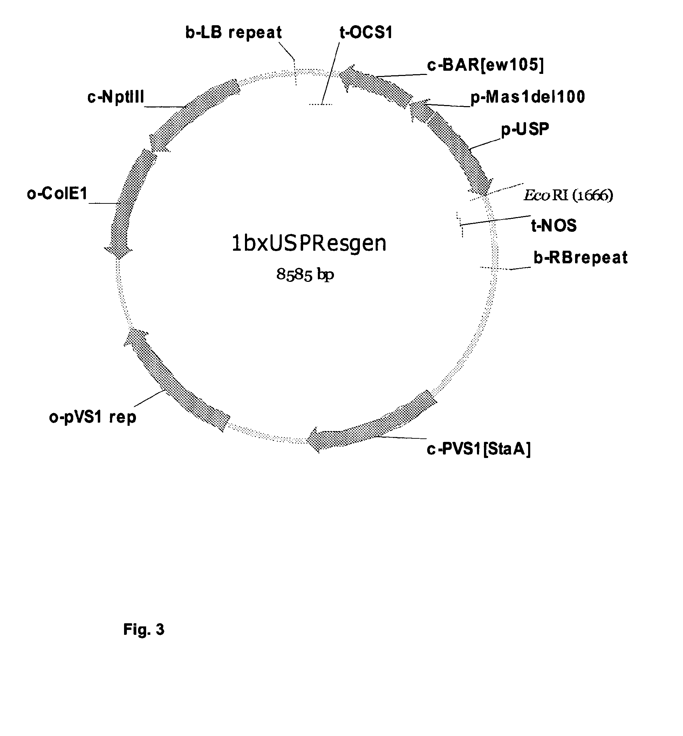

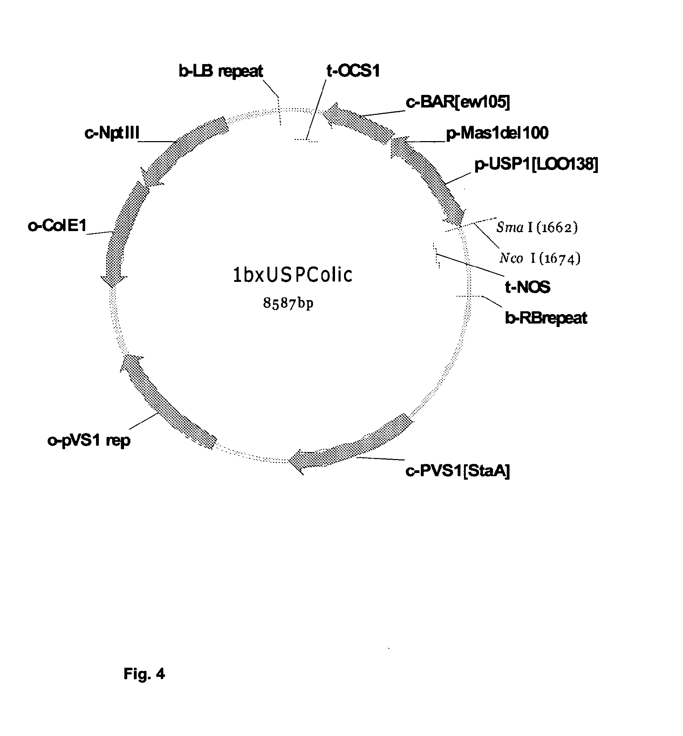

In one embodiment of the invention, plant expression vectors encompass those which are described in the figures: Fig. 3 and/or Fig.4.

**[0372.0.0.2] to [0441.0.0.2] see [0359.0.0.1] to [0428.0.0.1]**

Example 1: Cloning of the inventive sequences according to the SEQ ID NO: 1, 327, 987 or 1156 for the expression in plants

Unless otherwise specified, standard methods as described in Sambrook et al., Molecular Cloning: A laboratory manual, Cold Spring Harbor 1989, Cold Spring Harbor Laboratory Press are used.

The inventive sequences were amplified by PCR as described in the protocol of the Pfu Turbo or Herculase DNA polymerase (Stratagene).

The composition for the protocol of the *Pfu* Turbo or Herculase DNA polymerase was as follows: 1x PCR buffer (Stratagene), 0.2 mM of each dNTP, 100 ng genomic DNA of *Saccharomyces cerevisiae* (strain S288C; Research Genetics, Inc., now Invitrogen) or *Escherichia coli* (strain MG1655; *E.coli* Genetic Stock Center), 50 pmol forward primer, 50 pmol reverse primer, 2.5 u *Pfu* Turbo or Herculase DNA polymerase. The amplification cycles were as follows:

*Saccharomyces cerevisiae:* 1 cycle of 3 minutes at 94-95°C, followed by 25-36 cycles of in each case 1 minute at 95°C or 30 seconds at 94°C, 45 seconds at 50°C, 30 seconds at 50°C or 30 seconds at 55°C and 210-480 seconds at 72°C, followed by 1 cycle of 8 minutes at 72°C, then 4°C.

*Escherichia coli :* 1 cycle of 2-3 minutes at 94°C, followed by 25-30 cycles of in each case 30 seconds at 94°C, 30 seconds at 55-60°C and 5-10 minutes at 72°C, followed by 1 cycle of 10 minutes at 72°C, then 4°C.

The following adapter sequences were added to Saccharomyces cerevisiae ORF specific primers (see table IV) for cloning purposes:
i) foward primer:5'-GGAATTCCAGCTGACCACC-3' SEQ ID NO: 985
ii) reverse primer: 5'-GATCCCCGGGAATTGCCATG-3' SEQ ID NO: 986
The following adapter sequences were added to *Escherichia coli* ORF specific primers for cloning purposes:
iii) forward primer: 5'-TTGCTCTTCC- 3´SEQ ID NO: 979
iiii) reverse primer: 5'-TTGCTCTTCG-3' SEQ ID NO: 980
Therefore for amplification and cloning of *Saccharomyces cerevisiae* SEQ ID NO: 327, a primer consisting of the adaptor sequence i) and the ORF specific sequence SEQ ID NO: 675 and a second primer consisting of the adaptor sequence ii) and the ORF specific sequence SEQ ID NO: 676 were used. For amplification and cloning of *Echerichia coli* SEQ ID NO: 1, a primer consisting of the adaptor sequence iii) and the ORF specific sequence SEQ ID NO: 317 and a second primer consisting of the adaptor sequence iiii) and the ORF specific sequence SEQ ID NO: 318 were used.
For amplification and cloning of *Saccharomyces cerevisiae* SEQ ID NO: 987, a primer consisting of the adaptor sequence i) and the ORF specific sequence SEQ ID NO: 1147, as indicated in table III, column 7, line 4 and a second primer consisting of the adaptor sequence ii) and the ORF specific sequence SEQ ID NO: 1148 as indicated in table III, column 7, line 4 were used.
For amplification and cloning of *Saccharomyces cerevisiae* SEQ ID NO: 1156, a primer consisting of the adaptor sequence i) and the ORF specific sequence SEQ ID NO: 1184, as indicated in table III, column 7, line 5 and a second primer consisting of the adaptor sequence ii) and the ORF specific sequence SEQ ID NO: 1185 as indicated in table III, column 7, line 5 were used.

Construction of binary vectors for targeting of expressed proteins to the plastids.
For constitutive expression the binary vectors used for cloning the targeting sequence were 1bxSuperResgen SEQ ID NO: 958, and 1bxSuperColi SEQ ID NO: 957. For strong expression in seeds the binary vectors used for cloning the targeting sequence, were 1bxUSPResgen SEQ ID NO: 1194 (Fig. 3), and 1bxUSPColi SEQ ID NO: 1195 (Fig. 4), containing the USP promotor (Baeumlein et al., Mol Gen Genet, 1991, 225 (3):459-67). Other useful binary vectors are known to the skilled worker; an overview of binary vectors and their use can be found in Hellens, R., Mullineaux, P. and Klee H., [(2000) "A guide to Agrobacterium binary vectors", Trends in Plant Science, Vol. 5 No.10, 446-451. Such vectors have to be equally equipped with appropriate promoters and targeting sequences.

Amplification of the targeting sequence of the gene FNR from *Spinacia oleracea*
In order to amplify the targeting sequence of the FNR gene from *S. oleracea,* genomic DNA was extracted from leaves of 4 weeks old *S. oleracea* plants (DNeasy Plant Mini Kit, Qiagen, Hilden). The gDNA was used as the template for a PCR.
To enable cloning of the transit sequence into the vector 1 bxSuperResgen an EcoRI restriction enzyme recognition sequence was added to both the forward and reverse primers, whereas for cloning in the vectors 1bxSuperColi a Pmel restriction enzyme recognition sequence was added to the forward primer and a Ncol site was added to the reverse primer.
FNR5EcoResgen ATA gAA TTC gCA TAA ACT TAT CTT CAT AgT TgC C SEQ ID NO: 983
FNR3EcoResgen ATA gAA TTC AgA ggC gAT CTg ggC CCT SEQ ID NO: 981
FNR5PmeColic ATA gTT TAA ACg CAT AAA CTT ATC TTC ATA gTT gCC SEQ ID NO: 984
FNR3NcoColic ATA CCA Tgg AAg AgC AAg Agg CgA TCT ggg CCC T SEQ ID NO: 982
The sequence amplified from spinach, SEQ ID NO: 959 comprised a 5'UTR (bp1-166), and the coding region (bp 167-275 and 353-419). The coding sequence is interrupted by an intronic sequence from bp 276 to-bp 352. 
Gcataaacttatcttcatagttgccactccaatttgctccttgaatctcctccacccaatacataatccactcctccatcaccc
acttcactactaaatcaaacttaactctgtttttctctctcctcctttcatttcttattcttccaatcatcgtactccgccatgaccacc
gctgtcaccgccgctgtttctttcccctctaccaaaccacctctctctccgcccgaagctcctccgtcatttcccctgacaaa
atcagctacaaaaaggtgattcccaatttcactgtgtttttattaataatttgttattttgatgatgagatgattaatttgggtgctg
caggttcctttgtactacaggaatgtatctgcaactgggaaaatgggacccatcagggcccagatcgcctct
 The PCR fragment derived with the primers FNR5EcoResgen and FNR3EcoResgen was digested with EcoRI and ligated in the vector 1bxSuperResgen SEQ ID NO: 958 or 1bxUSPResgen SEQ ID NO: 1194 that had also been digested with EcoRI. The correct orientation of the FNR targeting sequence was tested by sequencing. The vector generated in this ligation step was 1 bxSuperTPFNRResgen or 1 bxUSPTPFNRResgen respectively.
The PCR fragment derived with the primers FNR5PmeColic and FNR3NcoColic was digested with Pmel and Ncol and ligated in the vector 1bxSuperColic SEQ ID NO: 957, or 1 bxUSPColic SEQ NO: 1195 that had been digested with Smal and Ncol. The vector generated in this ligation step was 1bxSuperTPFNRColic or 1bxUSPTPFNRColic respectively.

For cloning the ORF of SEQ ID NO: 327, 987 or 1156 respectively from *S. cerevisiae* the vector DNA was treated with the restriction enzyme Ncol. For cloning of ORFs from *E*. *coli ,* eg SEQ ID NO: 1, the vector DNA was treated with the restriction enzymes Pacl and Ncol following the standard protocol (MBI Fermentas). The reaction was stopped by inactivation at 70°C for 20 minutes and purified over QIAquick columns following the standard protocol (Qiagen).
Then the PCR-product representing the amplified ORF and the vector DNA were treated with T4 DNA polymerase according to the standard protocol (MBI Fermentas) to produce single stranded overhangs with the parameters 1 unit T4 DNA polymerase at 37°C for 2-10 minutes for the vector and 1 u T4 DNA polymerase at 15°C for 10-60 minutes.
The reactions were stopped by addition of high-salt buffer and purified over QIAquick columns following the standard protocol (Qiagen).

Approximately 30 ng of prepared vector and a defined amount of prepared amplificate were mixed and hybridized at 65°C for 15 minutes followed by 37°C 0,1 °C/1 seconds, followed by 37°C 10 minutes, followed by 0,1 °C/1 seconds, then 4 °C.

The ligated constructs were transformed in the same reaction vessel by addition of competent *E. coli* cells (strain DH5alpha) and incubation for 20 minutes at 1°C followed by a heat shock for 90 seconds at 42°C and cooling to 4°C. Then, complete medium (SOC) was added and the mixture was incubated for 45 minutes at 37°C. The entire mixture was subsequently plated onto an agar plate with 0.05 mg/ml kanamycine and incubated overnight at 37°C.

The outcome of the cloning step was verified by amplification with the aid of primers which bind upstream and downstream of the integration site, thus allowing the amplification of the insertion. The amplifications were carried as described in the protocol of *Taq* DNA polymerase (Gibco-BRL).

The amplification cycles were as follows: 1 cycle of 5 minutes at 94°C, followed by 35 cycles of in each case 15 seconds at 94°C, 15 seconds at 50-66°C and 5 minutes at 72°C, followed by 1 cycle of 10 minutes at 72°C, then 4°C.

Several colonies were checked, but only one colony for which a PCR product of the expected size was detected was used in the following steps.

A portion of this positive colony was transferred into a reaction vessel filled with complete medium (LB) supplemented with kanamycin (50 µg/ml) and incubated overnight at 37°C.

The plasmid preparation was carried out as specified in the Qiaprep standard protocol (Qiagen).

Example 2: Generation of transgenic plants which express SEQ ID NO: 1 or SEQ ID NO: 327, or SEQ ID NO: 987 or SEQ ID NO: 1156 respectively

1-5 ng of the plasmid DNA isolated was transformed by electroporation into competent cells of *Agrobacterium tumefaciens, of* strain GV 3101 pMP90 (Koncz and Schell, Mol. Gen. Gent. 204, 383-396, 1986). Thereafter, complete medium (YEP) was added and the mixture was transferred into a fresh reaction vessel for 3 hours at 28°C. Thereafter, all of the reaction mixture was plated onto YEP agar plates supplemented with the respective antibiotics, e.g. rifampicine (0.1 mg/ml), gentamycine (0.025 mg/ml and kanamycine (0.05 mg/ml) and incubated for 48 hours at 28°C.

The agrobacteria that contains the plasmid construct were then used for the transformation of plants.

A colony was picked from the agar plate with the aid of a pipette tip and taken up in 3 ml of liquid TB medium, which also contained suitable antibiotics as described above. The preculture was grown for 48 hours at 28°C and 120 rpm.

400 ml of LB medium containing the same antibiotics as above were used for the main culture. The preculture was transferred into the main culture. It was grown for 18 hours at 28°C and 120 rpm. After centrifugation at 4 000 rpm, the pellet was resuspended in infiltration medium (MS medium, 10% sucrose).

In order to grow the plants for the transformation, dishes (Piki Saat 80, green, provided with a screen bottom, 30 x 20 x 4.5 cm, from Wiesauplast, Kunststofftechnik, Germany) were half-filled with a GS 90 substrate (standard soil, Werkverband E.V., Germany). The dishes were watered overnight with 0.05% Proplant solution (Chimac-Apriphar, Belgium). *Arabidopsis thaliana* C24 seeds (Nottingham Arabidopsis Stock Centre, UK ; NASC Stock N906) were scattered over the dish, approximately 1 000 seeds per dish. The dishes were covered with a hood and placed in the stratification facility (8 h, 110 µmol/m²/s⁻¹, 22°C; 16 h, dark, 6°C). After 5 days, the dishes were placed into the short-day controlled environment chamber (8 h 130 µmol/m²/s⁻¹, 22°C; 16 h, dark 20°C), where they remained for approximately 10 days until the first true leaves had formed.

The seedlings were transferred into pots containing the same substrate (Teku pots, 7 cm, LC series, manufactured by Pöppelmann GmbH & Co, Germany). Five plants were pricked out into each pot. The pots were then returned into the short-day controlled environment chamber for the plant to continue growing.

After 10 days, the plants were transferred into the greenhouse cabinet (supplementary illumination, 16 h, 340 µE, 22°C; 8 h, dark, 20°C), where they were allowed to grow for further 17 days.

For the transformation, 6-week-old Arabidopsis plants, which had just started flowering were immersed for 10 seconds into the above-described agrobacterial suspension which had previously been treated with 10 I Silwett L77 (Crompton S.A., Osi Specialties, Switzerland). The method in question is described in Clough and Bent, 1998 (Clough, JC and Bent, AF. 1998 Floral dip: a simplified method for Agrobacterium-mediated transformation of Arabidopsis thaliana, Plant J. 16:735-743.

The plants were subsequently placed for 18 hours into a humid chamber. Thereafter, the pots were returned to the greenhouse for the plants to continue growing. The plants remained in the greenhouse for another 10 weeks until the seeds were ready for harvesting.

Depending on the resistance marker used for the selection of the transformed plants the harvested seeds were planted in the greenhouse and subjected to a spray selection or else first sterilized and then grown on agar plates supplemented with the respective selection agent. Since the vector contained the bar gene as the resistance marker, plantlets were sprayed four times at an interval of 2 to 3 days with 0.02 % BASTA® and transformed plants were allowed to set seeds. The seeds of the transgenic A. thaliana plants were stored in the freezer (at -20°C).

The plants were subsequently placed for 18 hours into a humid chamber. Thereafter, the pots were returned to the greenhouse for the plants to continue growing. The plants remained in the greenhouse for another 10 weeks until the seeds were ready for harvesting.

Eample 3: Nitrogen Content Analysis

The determination of nitrogen in the samples is performed using the Dumas method which relies on the complete combustion of the test material. The sample is heated in a high temperature furnace and rapidly combusted in the presence of pure oxygen. The combustion products (mainly CO2, H2O, NOx, and N2) are collected and allowed to equilibrate. An aliquot of the gas mixture is passed over hot copper to remove any oxygen and convert NO2 to N2. The sample is then passed through a trap that removes CO2 and H2O. The remaining nitrogen is measured by a thermal conductivity detector.

For the analysis of leaf material or for seed kernels, homogenized freeze-dryed material is used. In the case of Arabidopsis seeds, the seeds are analyzed directly without pretreatment.

4 - 7 mg of the sample were weighed into a tin foil cup together with 15 mg of tungsten(VI)-oxide (WO3). Analysis was performed using a commercial elementar analyzer (e.g. ELEMENTAR vario EL III, ELEMENTAR, Hanau, Germany).

Table VII shows the increased total nitrogen content of seeds from transgenic plants expressing the *Escherichia coli* ORF yeast ORF b1852, corresponding to SeqID NO: 1 or the Saccharomyces cerevisiae ORF YNL241c, corresponding to SEQ ID NO: 327, or the Saccharomyces cerevisiae ORF YJL167W, corresponding SEQ ID NO: 987 or or the Saccharomyces cerevisiae ORF YML054C, corresponding SEQ ID NO: 1156 respectively in the plastidial compartment under control of the USP promotor as described above. Column 1 shows the analysed ORF expressed in the plastidial compartment, column 2 shows the measured element, column 3 shows the wild type variability as "relative standard deviation", column 4 shows the mean change in the element content for different transgenic lines transformed with SEQ ID NO: 1 or SEQ ID NO: 327, or SEQ ID NO: 987 or SEQ ID NO: 1156 respectively relative to the wildtype control wich is standardized as "1", column 5 shows the standard deviation for the different transgenic lines and column 6 shows the maximal observed change. As expected, the relative increase in nitrogen corresponds to a relative decrease in carbon content.

| **ORF** | **Parameter** | **WT variability(RSD; %)** | **Mean, relative to wildtype control** | **SD** | **Max. Change** |
|---|---|---|---|---|---|
| b1852 | %N | 0.05 | 1.06 | 0.08 | 1.13 |
| b1852 | %C | 0.01 | 1.01 | 0.01 | 0.99 |
| YNL241C | %N | 0.05 | 1.09 | 0.04 | 1.12 |
| YNL241C | %C | 0.01 | 0.96 | 0.02 | 0.94 |
| YJL167W | %N | na | 1.08 | 0.1 | 1.26 |
| YJL167W | %C | na | 0.96 | 0.37 | 0.89 |
| YML054C | %N | na | 1.06 | 0.06 | 1.15 |
| YML054C | %C | na | 0.99 | 0.02 | 0.95 |

Example 4: Enhanced nitrogen use efficiency
In order to test enhanced nitrogen use efficiency of the transgenic lines, plants were grown under nitrogen limited conditions:
Transgenic lines expressing the *Escherichia coli* ORF yeast ORF b1852, corresponding to SeqID NO: 1 or Saccharomyces cerevisiae ORF YNL241c, corresponding to SEQ ID NO: 327 or the Saccharomyces cerevisiae ORF YJL167W, corresponding SEQ ID NO: 987 or or the Saccharomyces cerevisiae ORF YML054C, corresponding SEQ ID NO: 1156 respectively in the plastidal compartment, showed enhanced growth under nitrogen limited conditions and less symptoms of nitrogen deficiency (bleaching, retarded growth, senescence) in comparison to wilde type control plants.

**Table I A: Nucleic acid sequence ID numbers**

| | **1.** | **2.** | **3.** | **4.** | **5.** | **6.** | **7.** |
|---|---|---|---|---|---|---|---|
| **Application no.** | **Hit no.** | **Project no.:** | **Gene/ORF Locus Name** | **Organism** | **Lead NA SEQ ID** | **Metabolite** | **Homologs NA SEQ IDs** |
| | | OEX_N-Hits | YPR138C | Saccharomyces cerevisiae | 689 | n/a | 691,693,695,697,699,701,703,705, 707, 709, 711, 713, 715, 717, 719, 721, 723, 725, 727, 729, 731, 733, 735, 737, 739,741,743,745,747,749,751,753, 755, 757, 759, 761, 763, 765, 767, 769, 771,773,775,777,779,781,783,785, 787,789,791,793,795,797,799,801, 803,805,807,809,811,813,815,817, 819,821,823,825,827,829,831,833, 835,837,839,841,843,845,847,849, 851,853,855,857,859,861,863,865, 867,869,871,873,875,877,879,881, 883,885,887,889,891,893,895,897, 899,901,903,905,907,909,911,913, 915,917,919,921,923,925,927,929, 931,933,935,937,939,941,943,945, 947 |
| 1 | 1 | | | | | | |
| | | OEX_N-Hits | b1852 | Escherichia coli K12 | 1 | n/a | 3, 5, 7, 9, 11, 13, 15, 17, 19, 21, 23, 25, 27,29,31,33,35,37,39,41,43,45, 47,49,51,53,55,57,59,61,63,65, 67,69,71,73,75,77,79,81,83,85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201,203,205,207,209,211,213,215, 217,219,221,223,225,227,229,231, 233,235,237,239,241,243,245,247, 249,251,253,255,257,259,261,263, 265,267,269,271,273,275,277,279, 281, 283, 285, 287, 289, 291, 293, 295, 297,299,301,303,305,307,309,311, 313,315 |
| 2 | 2 | | | | | | |
| | | OEX_N-Hits | YNL241C | Saccharomyces cerevisiae | 327 | n/a | 329,331,333,335,337,339,341,343, 345,347,349,351,353,355,357,359, 361,363,365,367,369,371,373,375, 377,379,381,383,385,387,389,391, 393,395,397,399,401,403,405,407, 409, 411, 413, 415, 417, 419, 421, 423, 425,427,429,431,433,435,437,439, 441, 443, 445, 447, 449, 451, 453, 455, 457,459,461,463,465,467,469,471, 473,475,477,479,481,483,485,487, 489,491,493,495,497,499,501,503, 505,507,509,511,513,515,517,519, 521,523,525,527,529,531,533,535, 537,539,541,543,545,547,549,551, 553,555,557,559,561,563,565,567, 569,571,573,575,577,579,581,583, 585,587,589,591,593,595,597,599, 601,603,605,607,609,611,613,615, 617,619,621,623,625,627,629,631, 633,635,637,639,641,643,645,647, 649,651,653,655,657,659,661,663, 665,667,669,671,673 |
| 2 | 3 | | | | | | |
| 3 | 4 | OEX_N-Hits | Yjl167w | Saccharomyces cerevisiae | 987 | n/a | 989, 991, 993, 995, 997, 999, 1001, 1003, 1005, 1007, 1009, 1011, 1013, 1015, 1017, 1019, 1021, 1023, 1025, 1027, 1029, 1031, 1033, 1035, 1037, 1039, 1041, 1043, 1045, 1047, 1049, 1051, 1053, 1055, 1057_{,} 1059, 1061, 1063, 1065, 1067, 1069, 1071, 1073, 1075, 1077, 1079, 1081, 1083, 1085, 1087, 1089, 1091, 1093, 1095, 1097, 1099, 1101, 1103, 1105, 1107, 1109, 1111, 1113, 1115, 1117, 1119, 1121, 1123, 1125, 1127, 1129, 1131, 1133, 1135, 1137 |
| 3 | 5 | OEX_N-Hits | Yml054c | Saccharomyces cerevisiae | 1156 | n/a | 1158, 1160, 1162, 1164, 1166, 1168, 1170, 1172, 1174, 1176, 1178, 1180, 1182 |

**Table I B: Nucleic acid sequence ID numbers**

| | **1.** | **2.** | **3.** | **4.** | **5.** | **6.** | **7.** |
|---|---|---|---|---|---|---|---|
| **Application no.** | **Hit no.** | **Project no.:** | **Gene/ORF Locus Name** | **Organism** | **Lead NA SEQ ID** | **Metabolite** | **Homologs NA SEQ IDs** |
| 1 | 1 | OEX_N-Hits | YPR138C | Saccharomyces cerevisiae | 689 | n/a | 955 |
| 2 | 2 | OEX_N-Hits | b1852 | Escherichia coli K12 | 1 | n/a | 323,325 |
| 2 | 3 | OEX_N-Hits | YNL241C | Saccharomyces cerevisiae | 327 | n/a | 681,683,685,687 |
| 3 | 4 | OEX_N-Hits | Yjl167w | Saccharomyces cerevisiae | 987 | n/a | 1139, 1141, 1143, 1145 |
| 3 | 5 | OEX_N-Hits | Yml054c | Saccharomyces cerevisiae | 1156 | n/a | - |

**Table II A: Amino acid sequence ID numbers**

| | **1.** | **2.** | **3.** | **4.** | **5.** | **6.** | **7.** |
|---|---|---|---|---|---|---|---|
| **Application no** | **Hit no.** | **Project no.:** | **Gene/ORF Locus Name** | **Organism** | **Lead AA SEQ ID** | **Metabolite** | **Homologs AA SEQ IDs** |
| | | OEX_N-Hits | YPR138C | Saccharomyces cerevisiae | 690 | n/a | 692, 694, 696, 698, 700, 702, 704, 706, 708, 710, 712, 714, 716, 718, 720, 722, 724, 726, 728, 730, 732, 734, 736, 738, 740, 742, 744, 746, 748, 750, 752, 754, 756, 758, 760, 762, 764, 766, 768, 770, 772, 774, 776, 778, 780, 782, 784, 786, 788, 790, 792, 794, 796, 798, 800, 802, 804,806,808,810,812,814,816, 818,820,822,824,826,828,830, 832,834,836,838,840,842,844, 846, 848, 850, 852, 854, 856, 858, 860, 862, 864, 866, 868, 870, 872, 874, 876, 878, 880, 882, 884, 886, 888,890,892,894,896,898,900, 902,904,906,908,910,912,914, 916,918,920,922,924,926,928, 930, 932, 934, 936, 938, 940, 942, 944,946,948 |
| 1 | 1 | | | | | | |
| | | OEX_N-Hits | b1852 | Escherichia coli K12 | 2 | n/a | 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202,204,206,208,210,212,214, 216,218,220,222,224,226,228, 230,232,234,236,238,240,242, 244,246,248,250,252,254,256, 258,260,262,264,266,268,270, 272, 274, 276, 278, 280, 282, 284, 286,288,290,292,294,296,298, 300, 302, 304, 306, 308, 310, 312, 314,316 |
| 2 | 2 | | | | | | |
| | | OEX_N-Hits | YNL241C | Saccharomyces cerevisiae | 328 | n/a | 330, 332, 334, 336, 338, 340, 342, 344, 346, 348, 350, 352, 354, 356, 358,360,362,364,366,368,370, 372,374,376,378,380,382,384, 386,388,390,392,394,396,398, 400,402,404,406,408,410,412, 414, 416, 418, 420, 422, 424, 426, 428,430,432,434,436,438,440, 442, 444, 446, 448, 450, 452, 454, 456, 458, 460, 462, 464, 466, 468, 470, 472, 474, 476, 478, 480, 482, 484, 486, 488, 490, 492, 494, 496, 498,500,502,504,506,508,510, 512, 514, 516, 518, 520, 522, 524, 526, 528, 530, 532, 534, 536, 538,540, 542, 544, 546, 548, 550, 552, 554,556,558,560,562,564,566, 568,570,572,574,576,578,580, 582,584,586,588,590,592,594, 596, 598, 600, 602, 604, 606, 608, 610,612,614,616,618,620,622, 624, 626, 628, 630, 632, 634, 636, 638, 640, 642, 644, 646, 648, 650, 652, 654, 656, 658, 660, 662, 664, 666,668,670,672,674 |
| 2 | 3 | | | | | | |
| 3 | 4 | OEX_N-Hits | Yjl167w | Saccharomyces cerevisiae | 988 | n/a | 990, 992, 994, 996, 998, 1000, 1002, 1004, 1006, 1008, 1010, 1012, 1014, 1016, 1018, 1020, 1022, 1024, 1026, 1028, 1030, 1032, 1034, 1036, 1038, 1040, 1042, 1044, 1046, 1048, 1050, 1052, 1054, 1056, 1058, 1060, 1062, 1064, 1066, 1068, 1070, 1072, 1074, 1076, 1078, 1080, 1082, 1084, 1086, 1088, 1090, 1092, 1094, 1096, 1098, 1100, 1102, 1104, 1106, 1108, 1110, 1112, 1114, 1116, 1118, 1120, 1122, 1124, 1126, 1128, 1130, 1132, 1134, 1136, 1138 |
| 3 | 5 | OEX_N-Hits | Yml054c | Saccharomyces cerevisiae | 1157 | n/a | 1159, 1161, 1163, 1165, 1167, 1169, 1171, 1173, 1175, 1177, 1179, 1181, 1183 |

**Table II B: Amino acid sequence ID numbers**

| | **1.** | **2.** | **3.** | **4.** | **5.** | **6.** | **7.** |
|---|---|---|---|---|---|---|---|
| **Application no** | **Hit no.** | **Project no.:** | **Gene/ORF Locus Name** | **Organism** | **Lead AA SEQ ID** | **Metabolite** | **Homologs AA SEQ IDs** |
| 1 | 1 | OEX_N-Hits | YPR138C | Saccharomyces cerevisiae | 690 | n/a | 956 |
| 2 | 2 | OEX_N-Hits | b1852 | Escherichia coli K12 | 2 | n/a | 324,326 |
| 2 | 3 | OEX_N-Hits | YNL241C | Saccharomyces cerevisiae | 328 | n/a | 682,684,686,688 |
| 3 | 4 | OEX_N-Hits | Yjl167w | Saccharomyces cerevisiae | 988 | n/a | 1140, 1142, 1144, 1146 |
| 3 | 5 | OEX_N-Hits | Yml054c | Saccharomyces cerevisiae | 1157 | n/a | - |

**Table III: Primer nucleic acid sequence ID numbers**

| | **1.** | **2.** | **3.** | **4.** | **5.** | **6.** | **7.** |
|---|---|---|---|---|---|---|---|
| **Application no** | **Hit no.** | **Project no.:** | **Gene/ORF Locus Name** | **Organism** | **Lead NA SEQ ID** | **Metabolite** | **Primer NA SEQ IDs** |
| 1 | 1 | OEX_N-Hits | YPR138C | Saccharomyces cerevisiae | 689 | n/a | 949,950 |
| 2 | 2 | OEX_N-Hits | b1852 | Escherichia coli K12 | 1 | n/a | 317,318 |
| 2 | 3 | OEX_N-Hits | YNL241C | Saccharomyces cerevisiae | 327 | n/a | 675,676 |
| 3 | 4 | OEX_N-Hits | Yjl167w | Saccharomyces cerevisiae | 987 | n/a | 1147, 1148 |
| 3 | 5 | OEX_N-Hits | Yml054c | Saccharomyces cerevisiae | 1156 | n/a | 1184, 1185 |

**Table IV: Consensus nucleic acid sequence ID numbers**

| | **1.** | **2.** | **3.** | **4.** | **5.** | **6.** | **7.** |
|---|---|---|---|---|---|---|---|
| **Application no** | **Hit no.** | **Project no.:** | **Gene/ORF Locus Name** | **Organism** | **Lead AA SEQ ID** | **Metabolite** | **Consensus AA SEQ ID** |
| 1 | 1 | OEX_N-Hits | YPR138C | Saccharomyces cerevisiae | 690 | n/a | 951, 952, 953, 954 |
| 2 | 2 | OEX_N-Hits | b1852 | Escherichia coli K12 | 2 | n/a | 319,320,321,322 |
| 2 | 3 | OEX_N-Hits | YNL241C | Saccharomyces cerevisiae | 328 | n/a | 677,678,679,680 |
| 3 | 4 | OEX_N-Hits | Yjl167w | Saccharomyces cerevisiae | 988 | n/a | 1149, 1150, 1151, 1152, 1153, 1154, 1155 |
| 3 | 5 | OEX_N-Hits | Yml054c | Saccharomyces cerevisiae | 1157 | n/a | 1186, 1187, 1188, 1189, 1190, 1191, 1192,1193 |

## Claims

1. A process for
a) increasing nitrogen assimilation, accumulation and/or utilization and/or for increased total nitrogen content in a photosynthetic active organism; or
b) increasing amino acid content in the fruit or seed or in the whole organism; or
c) increasing protein content in the fruit or seed or in the whole organism; or
d) increasing biomass yield;
comprising increasing or generating the activity of a protein encoded by the nucleic acid sequences as shown in table I, application no. 3, Hit no. 4, column 5 or column 7 in a photosynthetic organism and growing the photosynthetic active organism under conditions which permit the enhanced trait.

2. A process for
a) increasing amino acid content in the fruit or seed or in the whole organism; or
b) increasing protein content in the fruit or seed or in the whole organism; or
c) increasing biomass yield;
comprising increasing or generating the activity of a protein encoded by the nucleic acid sequences as shown in table I, application no. 1, Hit no. 1, column 5 or column 7 or application no. 2, Hit no. 2, column 5 or column 7, or application no. 2, Hit no. 3, column 5 or column 7, or application no. 3, Hit no. 5, column 5 or column 7 in a photosynthetic organism and growing the photosynthetic active organism under conditions which permit the enhanced trait.

3. A process for increasing fine chemicals, preferably amino acids, in an organism comprising increasing or generating the activity of a protein encoded by the nucleic acid sequences as shown in table I, application no. 1, Hit no. 1, column 5 or column 7 or application no. 2, Hit no. 2, column 5 or column 7, or application no. 2, Hit no. 3, column 5 or column 7, or application no. 3, Hit no. 4, column 5 or column 7, or application no. 3, Hit no. 5, column 5 or column 7
and growing the organism under conditions which permit the increased content of fine chemicals, preferably amino acids.

4. The process of claim 1 comprising
a) increasing or generating the activity of a protein encoded by the nucleic acid sequences as shown in table I, application no. 3, Hit no. 4, column 5 or column 7, preferably derived from an microorganism, in an organelle of a non-human organism; or
b) increasing or generating the activity of a protein encoded by the nucleic acid sequences as shown in table I, application no. 3, Hit no. 4, column 5 or column 7, preferably derived from an microorganism, which are joined to a nucleic acid sequence encoding a transit peptide in a non-human organism, or in one or more parts thereof; or
c) or increasing or generating the activity of a protein encoded by the nucleic acid sequences as shown in table I, application no. 3, Hit no. 4 column 5 or column 7, preferably derived from an microorganism, which are joined to a nucleic acid sequence encoding chloroplast localization sequence, in a non-human organism, or in one or more parts thereof, and
d) growing the photosynthetic organism under conditions which permit the enhanced trait of one of claim 1a-1d.

5. The process of claim 1, which comprises, increasing or generating in an organism or a part or a compartment thereof the expression of at least one nucleic acid molecule, preferably derived from an microorganism, comprising a nucleic acid molecule selected from the group consisting of:
a) nucleic acid molecule encoding a polypeptide as shown in table II, application no. 3, Hit no. 4 columns 5 and 7 or a fragment thereof, which confers the enhanced trait of one of claim 1 a-1 d,
b) nucleic acid molecule comprising of a nucleic acid molecule as shown in table I, application no. 3, Hit no. 4 columns 5 and 7 which confers the enhanced trait of one of claim 1 a-1 d;
c) nucleic acid molecule whose sequence can be deduced from a polypeptide sequence encoded by a nucleic acid molecule of (a) or (b) as a result of the degeneracy of the genetic code and which confers the enhanced trait of one of claim 1a-1d;
d) nucleic acid molecule which encodes a polypeptide which has at least 50% identity with the amino acid sequence of the polypeptide encoded by the nucleic acid molecule of (a) to (c) and which confers the enhanced trait of one of claim 1 a-1 d;
e) nucleic acid molecule which hybridizes with a nucleic acid molecule of (a) to (c) under stringent hybridization and which confers the enhanced trait of one of claim 1a-1d;
f) nucleic acid molecule which encompasses a nucleic acid molecule which is obtained by amplifying nucleic acid molecules from a cDNA library or a genomic library using the primers or primer pairs as indicated in table III, application no. 3, Hit no. 4 column 7 and which confers the enhanced trait of one of claim 1 a-1 d;
g) nucleic acid molecule encoding a polypeptide which is isolated with the aid of monoclonal antibodies against a polypeptide encoded by one of the nucleic acid molecules of (a) to (f) and which confers the enhanced trait of one of claim 1 a-1 d;
h) nucleic acid molecule encoding a polypeptide comprising a consensus as shown in table IV, application no. 3, Hit no. 4 columns 7 and which confers the enhanced trait of one of claim 1 a-1 d;
i) nucleic acid molecule which is obtainable by screening a suitable nucleic acid library under stringent hybridization conditions with a probe comprising one of the sequences of the nucleic acid molecule of (a) to (h) or with a fragment thereof having at least 15 nt, preferably 20 nt, 30 nt, 50 nt, 100 nt, 200 nt or 500 nt of the nucleic acid molecule **characterized in** (a) to (h) and which confers the enhanced trait of one of claim 1 a-1 d;
or comprising a sequence which is complementary thereto.

6. An isolated nucleic acid molecule comprising a nucleic acid molecule selected from the group consisting of:
a) nucleic acid molecule encoding a polypeptide as shown in table II, application no. 3, Hit no. 4 columns 5 and 7, preferably as shown in table II B, application no. 3, Hit no. 4 column 5 and 7, or a fragment thereof, and which confers the enhanced trait of one of claim 1 a-1 d in a photosynthetic organism or a part thereof
b) nucleic acid molecule comprising of a nucleic acid molecule as shown in table IB, application no. 3, Hit no. 4 columns 5 and 7 and which confers the enhanced trait of one of claim 1 a-1 d in a photosynthetic organism or a part thereof;
c) nucleic acid molecule whose sequence can be deduced from a polypeptide sequence encoded by a nucleic acid molecule of (a) or (b) as a result of the degeneracy of the genetic code and which confers the enhanced trait of one of claim 1 a-1 d in a photosynthetic organism or a part thereof;
d) nucleic acid molecule which encodes a polypeptide which has at least 50% identity with the amino acid sequence of the polypeptide encoded by the nucleic acid molecule of (a) to (c) and which confers the enhanced trait of one of claim 1 a-1 d in a photosynthetic organism or a part thereof;
e) nucleic acid molecule which hybridizes with a nucleic acid molecule of (a) to (c) under stringent hybridization conditions and which confers the enhanced trait of one of claim 1 a-1 d in a photosynthetic organism or a part thereof;
f) nucleic acid molecule which encompasses a nucleic acid molecule which is obtained by amplifying nucleic acid molecules from a cDNA library or a genomic library using the primers or primer pairs as shown in table III, application no. 3, Hit no. 4 column 7 and which confers the enhanced trait of one of claim 1 a-1 d in a photosynthetic organism or a part thereof;
g) nucleic acid molecule encoding a polypeptide which is isolated with the aid of monoclonal antibodies against a polypeptide encoded by one of the nucleic acid molecules of (a) to (f) and conferring and which confers the enhanced trait of one of claim 1 a-1 d in a photosynthetic organism or a part thereof;
h) nucleic acid molecule encoding a polypeptide comprising a consensus as shown in table IV, application no. 3, Hit no. 4 column 7 and which confers the enhanced trait of one of claim 1 a-1 d in a photosynthetic organism or a part thereof; and
i) nucleic acid molecule which is obtainable by screening a suitable nucleic acid library under stringent hybridization conditions with a probe comprising one of the sequences of the nucleic acid molecule of (a) to (h) or with a fragment thereof having at least 15 nt, preferably 20 nt, 30 nt, 50 nt, 100 nt, 200 nt or 500 nt of the nucleic acid molecule **characterized in** (a) to (h) and conferring an and which confers the enhanced trait of one of claim 1 a-1 d in a photosynthetic organism or a part thereof,
whereby the nucleic acid molecule preferably distinguishes over the sequence as indicated in Table I A or I B, application no. 3, Hit no. 4, columns 5 and 7, more preferably distinguishes over the sequence as indicated in Table IA, application no. 3, Hit no. 4, columns 5 and 7 by one or more nucleotides.

7. A nucleic acid construct which confers the expression of the nucleic acid molecule of claim 6, comprising one or more regulatory elements.

8. A vector comprising the nucleic acid molecule as claimed in claim 6 or the nucleic acid construct of claim 7, wherein preferably the nucleic acid molecule is in operable linkage with regulatory sequences for the expression in a prokaryotic or eukaryotic, or in a prokaryotic and eukaryotic, host.

9. A host cell, preferably a plant host cell, which has been transformed stably or transiently with the vector as claimed in claim 8 or the nucleic acid molecule as claimed in claim 6 or the nucleic acid construct of claim 7.

10. The host cell of claim 9, which is a transgenic host cell and/or which is resistant to a herbicide inhibiting the enhanced trait of one of claim 1a-1d.

11. A plant tissue, propagation material, harvested material or a plant comprising the host cell as claimed in claim 9 or 10 which is plant cell or an Agrobacterium.

12. A method for the identification of a gene product conferring the enhanced trait of one of claim 1 a-1 d in a photosynthetic organism or a part thereof comprising the following steps:
a) contacting the nucleic acid molecules of a sample, which can contain a candidate gene encoding a gene product conferring the enhanced trait of one of claim 1 a-1 d in a photosynthetic organism or a part thereof after expression with the nucleic acid molecule of claim 6;
b) identifying the nucleic acid molecules, which hybridize under relaxed stringent conditions with the nucleic acid molecule of claim 6;
c) introducing the candidate nucleic acid molecules in host cells appropriate for enhanced the enhanced trait of one of claim 1 a-1 d;
d) expressing the identified nucleic acid molecules in the host cells;
e) assaying the enhanced trait of one of claim 1 a-1 d; and
f) identifying nucleic acid molecule and its gene product which expression confers an increased trait of one of claim 1 a-1 d in the host cell in the host cell after expression compared to the wild type.

13. A method for the identification of a gene product conferring an enhanced trait of one of claim 1 a-1 d in a photosynthetic organism or a part thereof comprising the following steps:
a) identifying in a data bank nucleic acid molecules of an organism; which can contain a candidate gene encoding a gene product conferring an enhanced trait of one of claim 1 a-1 d in a photosynthetic organism or a part thereof after expression, and which are at least 20% homolog to the nucleic acid molecule of claim 6;
b) introducing the candidate nucleic acid molecules in host cells appropriate enhanced trait of one of claim 1 a-1 d;
c) expressing the identified nucleic acid molecules in the host cells;
d) assaying the trait of one of claim 1a-1d; and
e) identifying nucleic acid molecule and its gene product which expression confers an increased trait of one of claim 1 a-1 d in the host cell in the host cell after expression compared to the wild type.

14. A composition comprising the nucleic acid molecule of claim 6, a polypeptide produced by a process for producing a polypeptide, wherein the polypeptide is expressed in a host cell as claimed in claim 9 or 10, the nucleic acid construct of claim 7, the vector of claim 8, the gene product of claim 12 or 13, the antibody of claim 5g) or 6g, and optionally an agricultural acceptable carrier,
preferably a agrochemical, pharmaceutical, food or feed composition comprising the nucleic acid molecule of claim 6, the polypeptide produced by a process for producing a polypeptide, wherein the polypeptide is expressed in a host cell as claimed in claim 9 or 10, the nucleic acid construct of claim 7, the vector of claim 8, the antibody of claim 5g) or 6g, the plant or plant tissue of claim 11, the harvested material of claim 11, the host cell of claim 9 or 10 or the gene product identified according to the method of claim 7 or claim 8.

15. Use of the nucleic acid molecule as claimed in claim 6 for the identification of a nucleic acid molecule conferring an enhanced trait of one of claim 1 a-1 d in the host cell in the host cell after expression compared to the wild type; or
use of a polypeptide produced by a process for producing a polypeptide, wherein the polypeptide is expressed in a host cell as claimed in claim 9 or 10 or encoded by the nucleic acid molecule as claimed in claim 6 whereby the polypeptide preferably distinguishes over a sequence as indicated in Table IIA by one or more amino acids or the nucleic acid construct claim 8 or the gene product identified according to the method of claim 12 or 13
for identifying compounds capable of conferring a modulation of the enhanced trait of one of claim 1a-1d in an organism or part thereof.

16. A process according to claims 1 to 5 **characterized by** enhanced growth of plants under nitrogen limiting conditions.

17. Use of the nucleic acid molecule of claim 6, a polypeptide produced by a process for producing a polypeptide, wherein the polypeptide is expressed in a host cell as claimed in claim 9 or 10 or encoded by the nucleic acid molecule as claimed in claim 6 whereby the polypeptide preferably distinguishes over a sequence as indicated in Table IIA by one or more amino acids, the nucleic acid construct of claim 7, the vector of claim 8, the plant or plant tissue of claim 11, the harvested material of claim 11, the host cell of claim 9 or 10 or the gene product identified according to the method of claim 12 or 13 for obtaining enhanced total yield in biomass, preferably seed or yield in protein mass, preferably for obtaining enhanced growth or yield under nitrogen limiting conditions.
